# EUROPEAN PATENT SPECIFICATION

(11) **EP 4 142 454 B1**
(45) Date of publication and mention of the grant of the patent: **01.05.2024**
(21) Application number: 22190831.2
(22) Date of filing: 17.08.2022
(51) Int. Cl.: H10K 85/60

(54) **HETEROCYCLIC COMPOUND AND LIGHT-EMITTING DEVICE INCLUDING THE HETEROCYCLIC COMPOUND**
HETEROCYCLISCHE VERBINDUNG UND LICHTEMITTIERENDE VORRICHTUNG MIT DER HETEROCYCLISCHEN VERBINDUNG
COMPOSÉ HÉTÉROCYCLIQUE ET DISPOSITIF ÉLECTROLUMINESCENT COMPRENANT LE COMPOSÉ HÉTÉROCYCLIQUE

(30) Priority: 27.08.2021 KR 20210114256
(43) Date of publication of application: 01.03.2023
(73) Proprietor: Samsung Display Co., Ltd., Yongin-si, Gyeonggi-do 17113 (KR)
(72) Inventor: AHN, Heechoon, 17113 Yongin-si (KR); KIM, Hyeongmin, 17113 Yongin-si (KR); PARK, Youngjin, 17113 Yongin-si (KR); YUN, Juhui, 17113 Yongin-si (KR); LEE, Hyoyoung, 17113 Yongin-si (KR); IM, Yirang, 17113 Yongin-si (KR)
(74) Representative: Newcombe, Christopher David

(56) References cited:
- US-A1- 2020 168 819
- NEUGEBAUER FRANZ ALFRED ET AL: "Aminyle, 6. tert.-Butyl-substituierte 9-Carbazolyl-Radikale, Carbazol-Radikalkationen und Carbazol-9-oxyl-Radikale", CHEMISCHE BERICHTE, vol. 105, no. 8, 1 August 1972 (1972-08-01), pages 2694-2713, XP093005844, DE ISSN: 0009-2940, DOI: 10.1002/cber.19721050830 Retrieved from the Internet: URL:https://onlinelibrary.wiley.com/doi/pd f/10.1002/cber.19721050830>

## Description

### BACKGROUND

### 1. Field

One or more embodiments relate to a heterocyclic compound, a light-emitting device including the heterocyclic compound, and an electronic apparatus including the light-emitting device.

### 2. Description of the Related Art

From among light-emitting devices, organic light-emitting devices are self-emissive devices that, as compared with devices of the related art, have wide viewing angles, high contrast ratios, short response times, and excellent characteristics in terms of luminance, driving voltage, and response speed, and produce full-color images.

In an example of organic light-emitting devices, a first electrode is located on a substrate, and a hole transport region, an emission layer, an electron transport region, and a second electrode are sequentially formed on the first electrode. Holes provided from the first electrode move toward the emission layer through the hole transport region, and electrons provided from the second electrode move toward the emission layer through the electron transport region. Carriers, such as holes and electrons, recombine in the emission layer to produce excitons. These excitons transition from an excited state to a ground state, thereby generating light.

US 2020/0168819 discloses host compounds for OLEDs. Compound 59 is an example compound comprising a carbazole moiety substituted by two tert-butyl groups in the 3- and 6-positions and also having a triazole moiety.

### SUMMARY

One or more embodiments include a heterocyclic compound, a light-emitting device including the heterocyclic compound, and an electronic apparatus including the light-emitting device.

Additional aspects will be set forth in part in the description, which follows and, in part, will be apparent from the description, or may be learned by practice of the presented embodiments of the disclosure.

According to an unclaimed embodiment the heterocyclic compound represented by Formula 1 is provided.

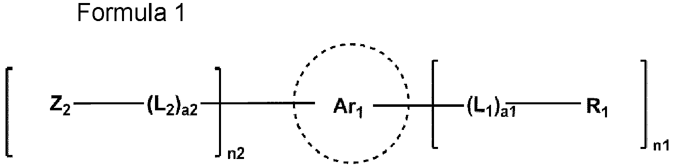

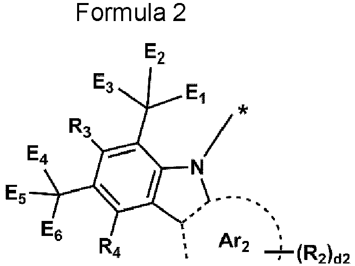

In Formula 1 and Formula 2,
Z₂ is a group represented by Formula 2,
Ar₁ and Ar₂ are each independently a C₅-C₆₀ carbocyclic group or a C₁-C₆₀ heterocyclic group,
L₁ and L₂ are each independently a single bond, a C₅-C₆₀ carbocyclic group unsubstituted or substituted with at least one R₁₀ₐ, or a C₁-C₆₀ heterocyclic group unsubstituted or substituted with at least one R₁₀ₐ,
a1 and a2 are each independently an integer from 1 to 3,
R₁ to R₄ are each independently
hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, - Si(Q₁)(Q₂)(Q₃), -B(Q₁)(Q₂), -C(=O)(Q₁), -S(=O)₂(Q₁), or -P(=O)(Q₁)(Q₂), or
a group represented by Formula 2,
E₁ to E₆ are each independently a C₁-C₆₀ alkyl group unsubstituted or substituted with at least one R₁₀ₐ, a C₂-C₆₀ alkenyl group unsubstituted or substituted with at least one R₁₀ₐ, or a C₂-C₆₀ alkynyl group unsubstituted or substituted with at least one R₁₀ₐ,
d2 is an integer from 0 to 10,
n1 is an integer from 1 to 10,
n2 is an integer from 1 to 5,
* indicates a binding site to a neighboring atom,
R₁₀ₐ is
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₁₁)(Q₁₂)(Q₁₃), -N(Q₁₁)(Q₁₂), -B(Q₁₁)(Q₁₂), -C(=O)(Q₁₁), - S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, or a C₂-C₆₀ heteroaryl alkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), - P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ are each independently hydrogen, deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, or a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a C₃-C₆₀ carbocyclic group, a C₁-C₆₀ heterocyclic group, or any combination thereof.

According to one or more embodiments, provided is a light-emitting device including
a first electrode,
a second electrode facing the first electrode,
an interlayer located between the first electrode and the second electrode and comprising an emission layer, and
at least one heterocyclic compound represented by Formula 1.

According to one or more embodiments, provided is an electronic apparatus including the light-emitting device.

According to an embodiment of the invention the heterocyclic compound according to Formula 1 is provided wherein the heterocyclic compound represented by Formula 1 satisfies at least one of Conditions 1 and 2:
Condition 1
   Ar₁ is a π electron-deficient nitrogen-containing C₁-C₆₀ cyclic group; and
Condition 2
   wherein at least one of R₁(s) in the number of n1 is -Si(Q₁)(Q₂)(Q₃).

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a schematic view of a light-emitting device according to an embodiment; and
FIGS. 2 and 3 are each a schematic cross-sectional view of a light-emitting apparatus according to an embodiment of the present disclosure.

### DETAILED DESCRIPTION

Reference will now be made in detail to embodiments, examples of which are illustrated in the accompanying drawings, wherein like reference numerals refer to like elements throughout the specification. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the same associated listed items. Throughout the disclosure, the expression "at least one of a, b or c" indicates only a, only b, only c, both a and b, both a and c, both b and c, all of a, b, and c, or variations thereof.

An aspect of the present disclosure provides a heterocyclic compound represented by Formula 1: 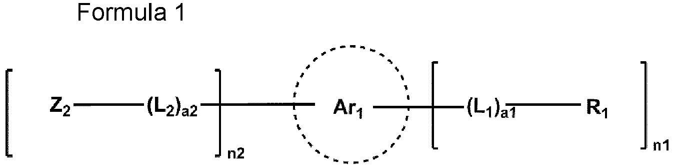 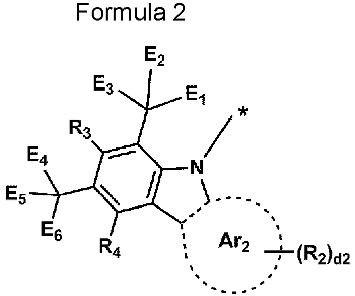 wherein, in Formula 1 and Formula 2,
Z₂ may be a group represented by Formula 2.

In an embodiment, Ar₁ and Ar₂ may each independently be a C₅-C₆₀ carbocyclic group or a C₁-C₆₀ heterocyclic group.

In an embodiment, Ar₁ may be a cyclopentadiene group, an adamantane group, a norbornane group, a benzene group, a pentalene group, a naphthalene group, an azulene group, an indacene group, an acenaphthylene group, a phenalene group, a phenanthrene group, an anthracene group, a fluoranthene group, a triphenylene group, a pyrene group, a chrysene group, a perylene group, a pentaphene group, a heptalene group, a naphthacene group, a picene group, a hexacene group, a pentacene group, a rubicene group, a coronene group, an ovalene group, an indene group, a fluorene group, a spiro-bifluorene group, a benzofluorene group, an indenophenanthrene group, an indenoanthracene group, a 1H-pyrrole group, a silole group, a borole group, a 2H-pyrrole group, a 3H-pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, or an azadibenzofuran group.

In an embodiment, Ar₂ may be a benzene group, a naphthalene group, an anthracene group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a pyridine group, a pyrimidine group, a pyrazine group, a pyridazine group, or a triazine group.

In an embodiment, L₁ and L₂ may each independently be a single bond, a C₅-C₆₀ carbocyclic group unsubstituted or substituted with at least one R₁₀ₐ, or a C₁-C₆₀ heterocyclic group unsubstituted or substituted with at least one R₁₀ₐ.

In an embodiment, L₁ and L₂ may each independently be:
a single bond; or
a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-a fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluoren-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group, each unsubstituted or substituted with at least one R₁₀ₐ, and
R₁₀ₐ may be the same as described herein.

In one or more embodiments, L₁ and L₂ may each independently be:
a single bond; or
one of groups represented by Formulae 3-1 to 3-40: 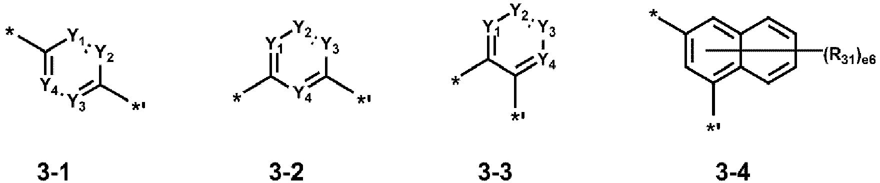 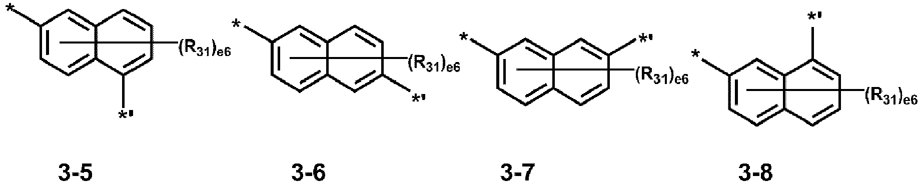 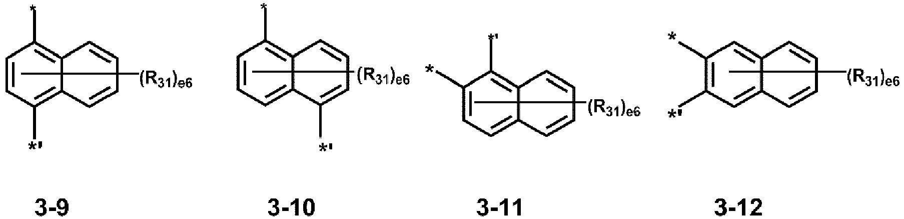 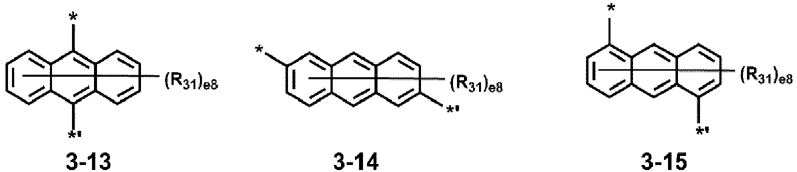 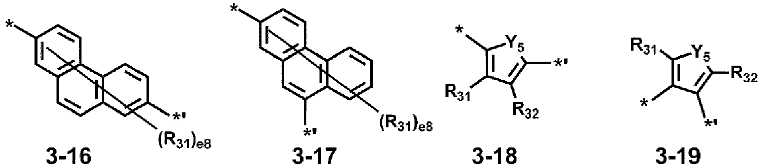 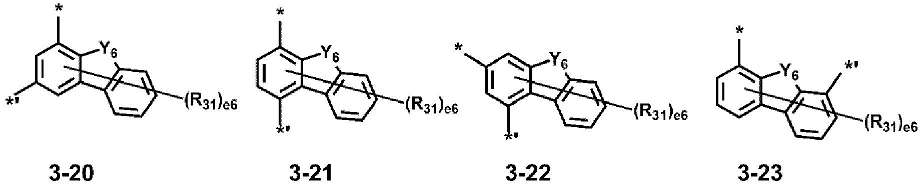 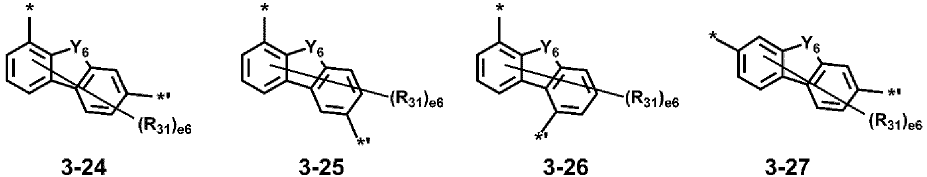 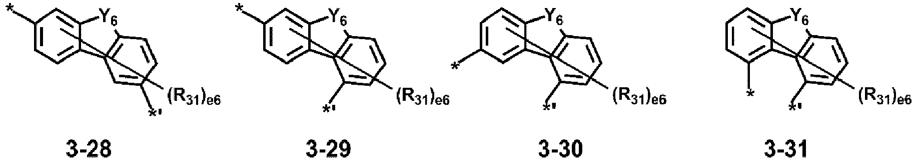 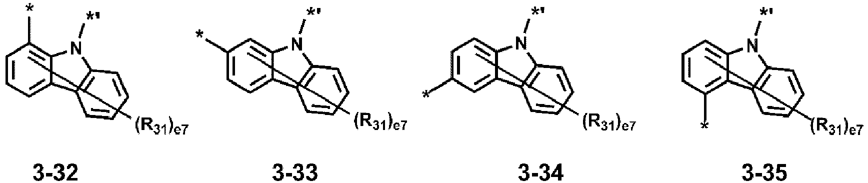 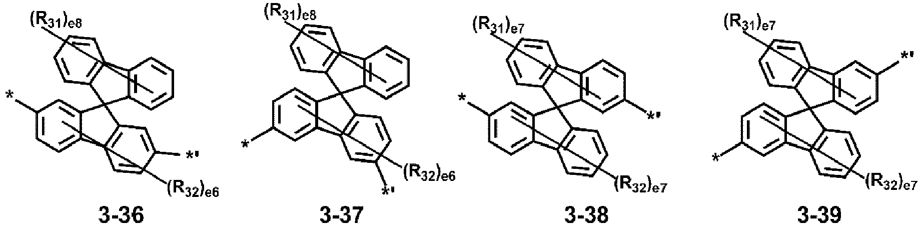 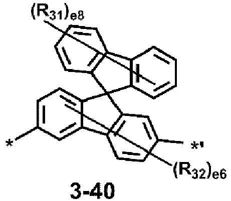 In Formulae 3-1 to 3-40,
   Y₁ may be N or C(R₃₃),
   Y₂ may be N or C(R₃₄),
   Y₃ may be N or C(R₃₅),
   Y₄ may be N or C(R₃₆),
   Y₅ may be O, S, or Se,
   Y₆ may be O, S, Se, N(R₃₇), or C(R₃₇)(R₃₈),
   R₃₁ to R₃₈ may each independently be:
   hydrogen, deuterium, -F, -Cl, -Br, -I, a hydroxyl group, a cyano group, or a nitro group;
   a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, -Si(Q₁₁)(Q₁₂)(Q₁₃), -B(Q₁₁)(Q₁₂), - C(=O)(Q₁₁), -S(=O)₂(Q₁₁), -P(=O)(Q₁₁)(Q₁₂), or any combination thereof;
   a C₃-C₆₀ carbocyclic group, a C₁-C₆₀ heterocyclic group, a C₆-C₆₀ aryloxy group; or a C₆-C₆₀ arylthio group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, -Si(Q₂₁)(Q₂₂)(Q₂₃), -B(Q₂₁)(Q₂₂), -C(=O)(Q₂₁), -S(=O)₂(Q₂₁), -P(=O)(Q₂₁)(Q₂₂), or any combination thereof; or
   -Si(Q₃₁)(Q₃₂)(Q₃₃), -B(Q₃₁)(Q₃₂), -C(=O)(Q₃₁), -S(=O)₂(Q₃₁), or -P(=O)(Q₃₁)(Q₃₂),
   Q₁ to Q₃, Q₁₁ to Q₁₃, Q₂₁ to Q₂₃, and Q₃₁ to Q₃₃ may each be the same as described herein,
   e4 may be an integer from 0 to 4,
   e6 may be an integer from 0 to 6,
   e7 may be an integer from 0 to 7,
   e8 may be an integer from 0 to 8, and
   * and *' each indicate a binding site to a neighboring atom.

In an embodiment, a1 and a2 may each independently be an integer from 1 to 3.

In an embodiment, R₁ to R₄ may each independently be: hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, -Si(Q₁)(Q₂)(Q₃), -B(Q₁)(Q₂), - C(=O)(Q₁), -S(=O)₂(Q₁), or -P(=O)(Q₁)(Q₂); or a group represented by Formula 2.

In an embodiment, R₃ and R₄ may each independently be hydrogen or deuterium.

In an embodiment, E₁ to E₆ may each independently be a C₁-C₆₀ alkyl group unsubstituted or substituted with at least one R₁₀ₐ, a C₂-C₆₀ alkenyl group unsubstituted or substituted with at least one R₁₀ₐ, or a C₂-C₆₀ alkynyl group unsubstituted or substituted with at least one R₁₀ₐ.

In one or more embodiments, E₁ to E₆ may each independently be: a C₁-C₂₀ alkyl group, a C₂-C₆₀ alkenyl group, or a C₂-C₆₀ alkynyl group; or
a C₁-C₂₀ alkyl group, a C₂-C₆₀ alkenyl group, or a C₂-C₂₀ alkynyl group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazine group, a hydrazone group, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a naphthyl group, a pyridinyl group, a pyrimidinyl group, or any combination thereof.

In an embodiment, d2 may be an integer from 0 to 10.

In an embodiment, n1 may be an integer from 1 to 10.

In an embodiment, n2 may be an integer from 1 to 5.

In one or more embodiments, n2 may be an integer from 1 to 3.

In an embodiment, * indicates a binding site to a neighboring atom.

In an embodiment, the heterocyclic compound represented by Formula 1 may satisfy one of Conditions 1 and 2:
Condition 1
   Ar₁ is a π electron-deficient nitrogen-containing C₁-C₆₀ cyclic group; and
Condition 2
   at least one of R₁(s) in the number of n1 is -Si(Q₁)(Q₂)(Q₃).

In one or more embodiments, the heterocyclic compound represented by Formula 1 may satisfy: Condition 1 only; Condition 2 only; or both Conditions 1 and 2.

In an embodiment, the heterocyclic compound represented by Formula 1 may be represented by Formula 1-A: 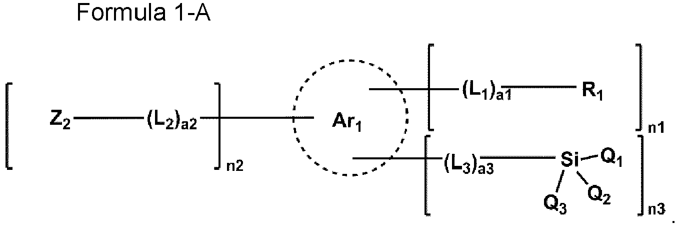 In Formula 1-A,
Z₂, Ar₁, L₁, L₂, n1, n2, a1, a2, R₁, and Q₁ to Q₃ may each be the same as described herein,
L₃ may be the same as described in connection with L₁,
a3 may be the same as described in connection with a1, and
n3 may be an integer from 0 to 10.

In an embodiment, n3 may be 0, 1, or 2.

In one or more embodiments, the heterocyclic compound represented by Formula 1 may be represented by one of Formulae 1-1 to 1-5: 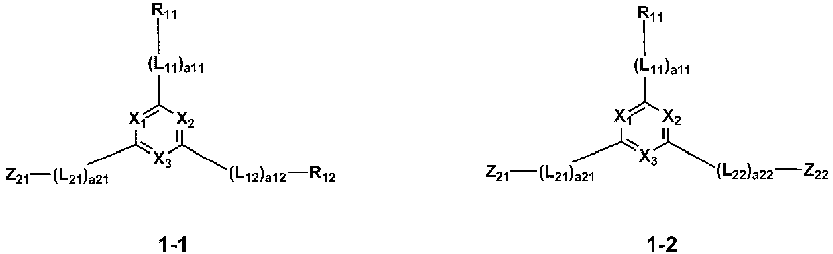 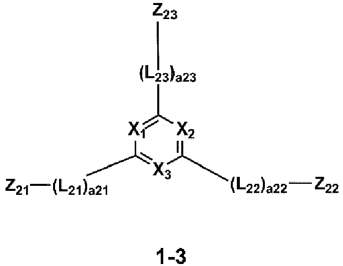 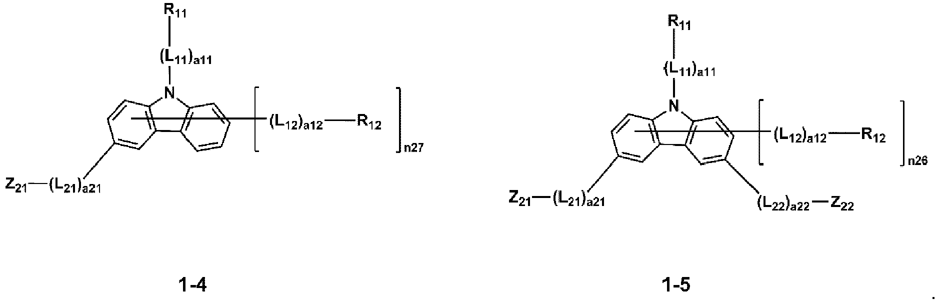 In Formulae 1-1 to 1-5,
X₁ may be C(R₁₃) or N,
X₂ may be C(R₁₄) or N,
X₃ may be C(R₁₅) or N,
R₁₁ to R₁₅ may each be the same as described in connection with R₁,
L₁₁ and L₁₂ may each be the same as described in connection with L₁,
a11 and a12 may each be the same as described in connection with a1,
n26 may be an integer from 1 to 6,
n27 may be an integer from 1 to 7,
L₂₁ to L₂₃ may each be the same as described in connection with L₂,
a21 to a23 may each be the same as described in connection with a2, and
Z₂₁ to Z₂₃ may each be the same as described in connection with Z₂.

In an embodiment, at least one of X₁ to X₃ may be N.

In an embodiment, R₁₁ may be -Si(Q₁)(Q₂)(Q₃).

In an embodiment, in Formula 1-1, R₁₁ and R₁₂ may each independently be - Si(Q₁)(Q₂)(Q₃).

In an embodiment, Q₁ to Q₃ may each be the same as described herein.

In an embodiment, the group represented by Formula 2 may be represented by Formula 2-1: 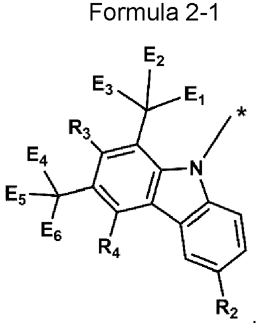 In Formula 2-1,
E₁ to E₆ and R₂ to R₄ may each be the same as described herein, and
* indicates a binding site to a neighboring atom.

In an embodiment, R₂ may be:
hydrogen, deuterium, or a cyano group;
a group represented by Formula 2; or
one of groups represented by Formulae 4-1 to 4-3: 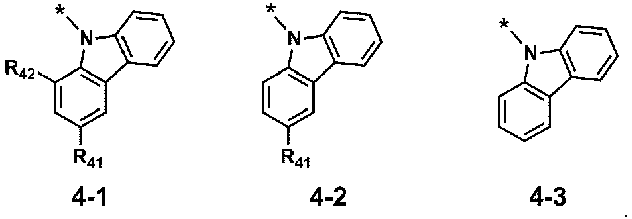 In Formulae 4-1 to 4-3,
   R₄₁ and R₄₂ may each independently be deuterium, -F, -Cl, -Br, -I, a hydroxyl group, a cyano group, a nitro group, an amidino group, a hydrazino group, a hydrazono group, -CF₃, -CF₂H, -CFH₂, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a naphthyl group, a fluorenyl group, a spiro-bifluorenyl group, a spiro-fluorene-benzofluorenyl group, a benzofluorenyl group, a dibenzofluorenyl group, a phenalenyl group, a phenanthrenyl group, an anthracenyl group, a fluoranthenyl group, a triphenylenyl group, a pyridinyl group, a pyrimidinyl group, a pyrazinyl group, a triazinyl group, a quinolinyl group, an isoquinolinyl group, a benzoquinolinyl group, a naphthyridinyl group, a quinoxalinyl group, a quinazolinyl group, a phenanthridinyl group, an acridinyl group, a phenanthrolinyl group, a phenazinyl group, a carbazolyl group, a dibenzofuranyl group, a dibenzothiophenyl group, a dibenzosilolyl group, -Si(Q₃₁)(Q₃₂)(Q₃₃), -N(Q₃₁)(Q₃₂), or-B(Q₃₁)(Q₃₂),
   Q₃₁ to Q₃₃ may each independently be a C₁-C₁₀ alkyl group, a C₁-C₁₀ alkoxy group, a phenyl group, a biphenyl group, a terphenyl group, or a naphthyl group, and
   * indicates a binding site to a neighboring atom.

The term "R₁₀ₐ" as used herein may be:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₁₁)(Q₁₂)(Q₁₃), -N(Q₁₁)(Q₁₂), -B(Q₁₁)(Q₁₂), -C(=O)(Q₁₁), - S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, or a C₂-C₆₀ heteroaryl alkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), - P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ may each independently be: hydrogen; deuterium; -F; -Cl; -Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; or a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a C₃-C₆₀ carbocyclic group, a C₁-C₆₀ heterocyclic group, or any combination thereof.

In one or more embodiments, the heterocyclic compound represented by Formula 1 may be one of Compounds 1 to 50, but embodiments of the present disclosure are not limited thereto:

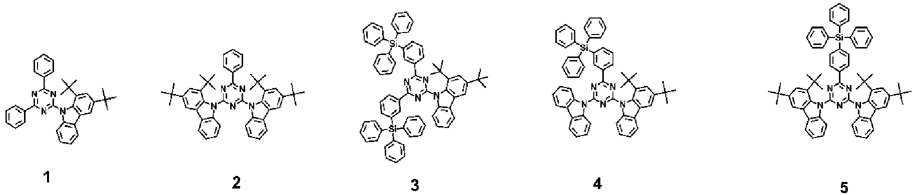

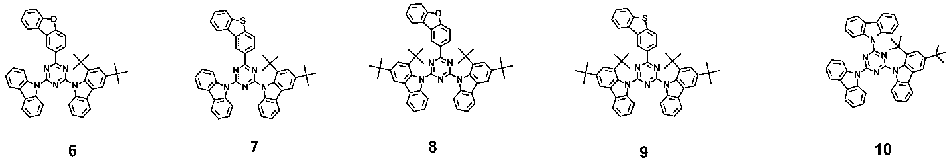

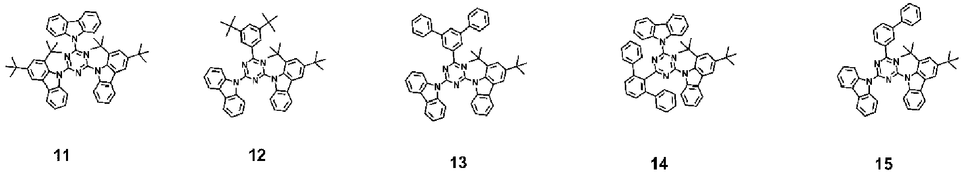

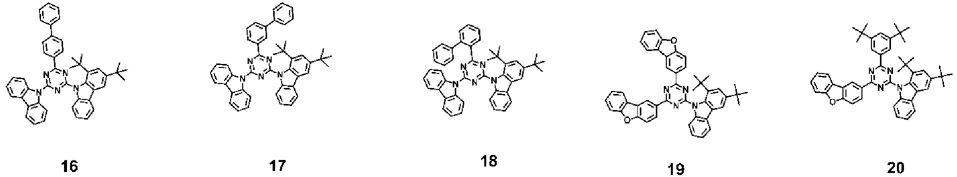

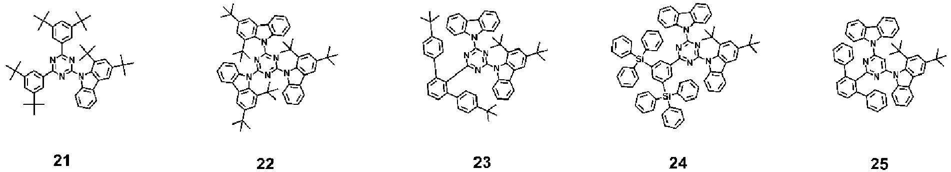

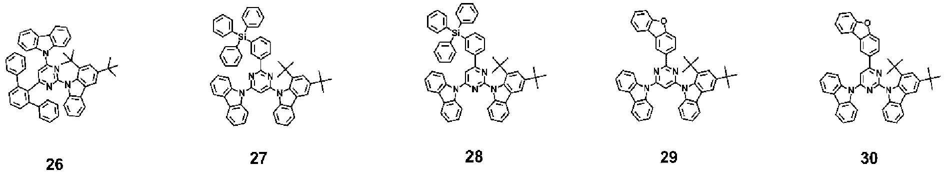

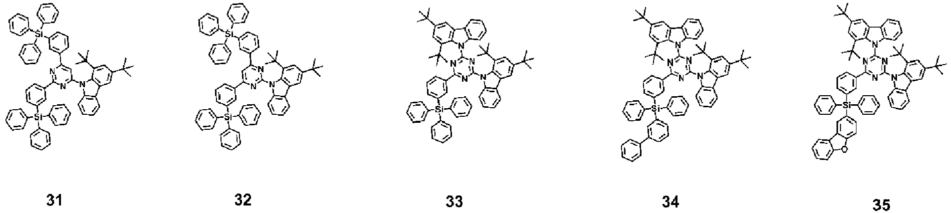

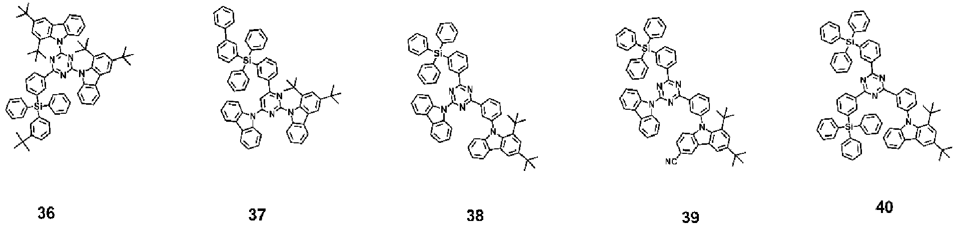

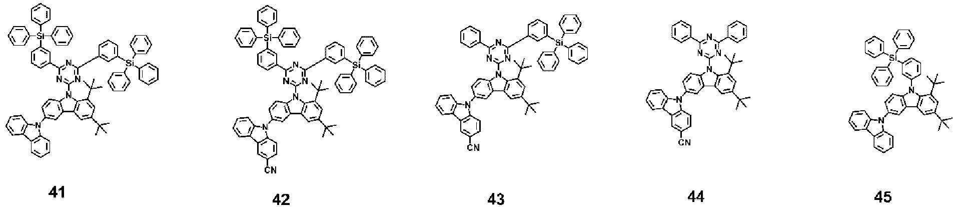

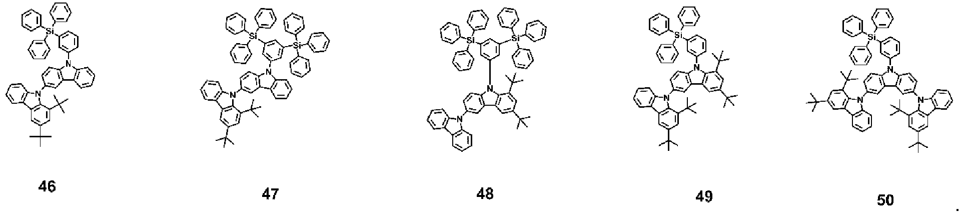

The heterocyclic compound represented by Formula 1 disclosed herein has a structure that includes a carbazole-based substituent in which a bulky substituent (for example, a t-butyl group) is located at C1 and C3 positions.

As the heterocyclic compound represented by Formula 1 includes a carbazole-based substituent in which a bulky substituent is located at C1 and C3 positions, degradation of color purity due to the formation of an exciplex between a host and a dopant may be suppressed, thereby having the advantage of improving the electrical stability of the molecule.

In addition, as the heterocyclic compound represented by Formula 1 includes at least one π electron-deficient nitrogen-containing C₁-C₆₀ cyclic group or at least one silyl group, the electron transport ability may be increased, and thus the heterocyclic compound may have advantageous in terms of luminescence efficiency and energy transfer.

Therefore, an electronic device, for example, an organic light-emitting device, employing the heterocyclic compound represented by Formula 1 may have low driving voltage, high EQE, high efficiency, and a long lifespan.

Methods of synthesizing the heterocyclic compound represented by Formula 1 should be readily apparent to those of ordinary skill in the art by referring to Examples described herein.

At least one heterocyclic compound represented by Formula 1 may be used in a light-emitting device (for example, an organic light-emitting device).

Another aspect of the present disclosure provides a light-emitting device including: a first electrode; a second electrode facing the first electrode; an interlayer between the first electrode and the second electrode and including an emission layer; and at least one the heterocyclic compound.

In an embodiment, the first electrode may be an anode,
the second electrode may be a cathode,
the interlayer may further include a hole transport region between the emission layer and the first electrode and an electron transport region between the emission layer and the second electrode,
the hole transport region may include a hole injection layer, a hole transport layer, an emission auxiliary layer, an electron blocking layer, or any combination thereof, and
the electron transport region may include a buffer layer, a hole blocking layer, an electron control layer, an electron transport layer, an electron injection layer, or any combination thereof.

In an embodiment, the interlayer may include the at least one the heterocyclic compound.

In an embodiment, the emission layer may include the at least one the heterocyclic compound.

In an embodiment, the emission layer may include a host and a dopant, wherein
an amount of the host may be greater than that of the dopant, and
the host may include the at least one the heterocyclic compound.

In an embodiment, the emission layer may further include a transition metal-containing compound.

In an embodiment, the emission layer may emit blue light or blue-green light.

In an embodiment, the emission layer may emit blue light or blue-green light, each having a maximum emission wavelength in a range of about 400 nm to about 500 nm.

As used herein, the expression the "(interlayer) includes at least one heterocyclic compound" may be construed as meaning the "(interlayer) may include one heterocyclic compound of Formula 1 or two different heterocyclic compounds of Formula 1".

In an embodiment, the interlayer may include only Compound 1 as the heterocyclic compound. In this regard, Compound 1 may be in the emission layer of the light-emitting device. In one or more embodiments, the interlayer may include, as the heterocyclic compound, Compound 1 and Compound 2. In this regard, Compound 1 and Compound 2 may be in an identical layer (for example, Compound 1 and Compound 2 may all be in the emission layer), or may be in different layers (for example, Compound 1 may be in the emission layer and Compound 2 may be in the electron transport region).

The term "interlayer" as used herein refers to a single layer and/or all of a plurality of layers between the first electrode and the second electrode of the light-emitting device.

Another aspect of the present disclosure provides an electronic apparatus including the light-emitting device. The electronic apparatus may further include a thin-film transistor.

For example, the electronic apparatus may further include a thin-film transistor including a source electrode and a drain electrode, wherein the first electrode of the light-emitting device may be electrically connected to the source electrode or the drain electrode.

In an embodiment, the electronic apparatus may further include a color filter, a color conversion layer, a touch screen layer, a polarizing layer, or any combination thereof. For example, the electronic apparatus may be a flat panel display apparatus, but embodiments of the present disclosure are not limited thereto.

More details for the electronic apparatus are the same as described herein.

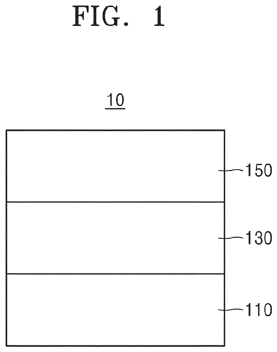

### [Description of FIG. 1]

FIG. 1 is a schematic cross-sectional view of a light-emitting device 10 according to an embodiment. The light-emitting device 10 includes a first electrode 110, an interlayer 130, and a second electrode 150.

Hereinafter, the structure of the light-emitting device 10 according to an embodiment and a method of manufacturing the light-emitting device 10 will be described with reference to FIG. 1.

### [First electrode 110]

In FIG. 1, a substrate may be additionally located under the first electrode 110 or on the second electrode 150. In an embodiment, as the substrate, a glass substrate or a plastic substrate may be used. In one or more embodiments, the substrate may be a flexible substrate, and for example, may include plastics with excellent heat resistance and durability, such as polyimide, polyethylene terephthalate (PET), polycarbonate, polyethylene napthalate, polyarylate (PAR), polyetherimide, or any combination thereof.

The first electrode 110 may be formed by, for example, depositing or sputtering a material for forming the first electrode 110 on the substrate. When the first electrode 110 is an anode, a material for forming the first electrode 110 may be a high-work function material that facilitates injection of holes.

The first electrode 110 may be a reflective electrode, a semi-transmissive electrode, or a transmissive electrode. In an embodiment, when the first electrode 110 is a transmissive electrode, a material for forming the first electrode 110 may include indium tin oxide (ITO), indium zinc oxide (IZO), tin oxide (SnO₂), zinc oxide (ZnO), or any combination thereof. In one or more embodiments, when the first electrode 110 is a semi-transmissive electrode or a reflective electrode, a material for forming the first electrode 110 may include magnesium (Mg), silver (Ag), aluminum (Al), aluminum-lithium (Al-Li), calcium (Ca), magnesium-indium (Mg-In), magnesium-silver (Mg-Ag), or any combination thereof.

The first electrode 110 may have a single-layered structure consisting of a single layer or a multi-layered structure including a plurality of layers. For example, the first electrode 110 may have a three-layered structure of ITO/Ag/ITO.

### [Interlayer 130]

The interlayer 130 is located on the first electrode 110. The interlayer 130 may include an emission layer.

The interlayer 130 may further include a hole transport region between the first electrode 110 and the emission layer, and an electron transport region between the emission layer and the second electrode 150.

The interlayer 130 may further include, in addition to various organic materials, a metal-containing compound, such as an organometallic compound, an inorganic material, such as a quantum dot, and the like.

In an embodiment, the interlayer 130 may include: i) two or more emitting units sequentially stacked between the first electrode 110 and the second electrode 150; and ii) a charge generation layer located between the two or more emitting units. When the interlayer 130 includes the two or more emitting units and the charge generation layer as described above, the light-emitting device 10 may be a tandem light-emitting device.

### [Hole transport region in interlayer 130]

The hole transport region may have: i) a single-layered structure consisting of a single layer consisting of a single material; ii) a single-layered structure consisting of a single layer consisting of a plurality of different materials; or iii) a multi-layered structure including a plurality of layers including different materials.

The hole transport region may include a hole injection layer, a hole transport layer, an emission auxiliary layer, an electron-blocking layer, or any combination thereof.

For example, the hole transport region may have a multi-layered structure including a hole injection layer/hole transport layer structure, a hole injection layer/hole transport layer/emission auxiliary layer structure, a hole injection layer/emission auxiliary layer structure, a hole transport layer/emission auxiliary layer structure, or a hole injection layer/hole transport layer/electron-blocking layer structure, wherein constituent layers of each structure are stacked sequentially from the first electrode 110.

The hole transport region may include a compound represented by Formula 201, a compound represented by Formula 202, or any combination thereof: 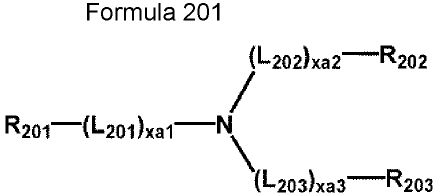 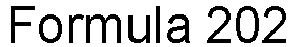 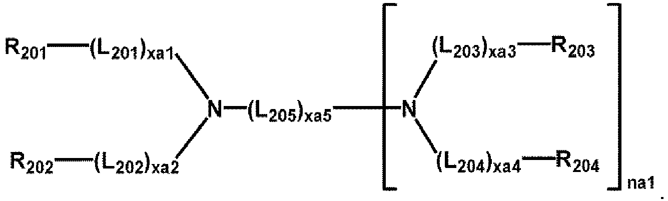 In Formulae 201 and 202,
L₂₀₁ to L₂₀₄ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
L₂₀₅ may be *-O-*', *-S-*', *-N(Q₂₀₁)-*', a C₁-C₂₀ alkylene group unsubstituted or substituted with at least one R₁₀ₐ, a C₂-C₂₀ alkenylene group unsubstituted or substituted with at least one R₁₀ₐ, a C₃-C₆₀ carbocyclic group unsubstituted or substituted with at least one R₁₀ₐ, or a C₁-C₆₀ heterocyclic group unsubstituted or substituted with at least one R10a,
xa1 to xa4 may each independently be an integer from 0 to 5,
xa5 may be an integer from 1 to 10,
R₂₀₁ to R₂₀₄ and Q₂₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
R₂₀₁ and R₂₀₂ may optionally be linked to each other via a single bond, a C₁-C₅ alkylene group unsubstituted or substituted with at least one R₁₀ₐ, or a C₂-C₅ alkenylene group unsubstituted or substituted with at least one R₁₀ₐ, to form a C₈-C₆₀ polycyclic group (for example, a carbazole group or the like) unsubstituted or substituted with at least one R₁₀ₐ (for example, Compound HT16),
R₂₀₃ and R₂₀₄ may optionally be linked to each other via a single bond, a C₁-C₅ alkylene group unsubstituted or substituted with at least one R₁₀ₐ, or a C₂-C₅ alkenylene group unsubstituted or substituted with at least one R₁₀ₐ, to form a C₈-C₆₀ polycyclic group unsubstituted or substituted with at least one R₁₀ₐ, and
na1 may be an integer from 1 to 4.

For example, each of Formulae 201 and 202 may include at least one of groups represented by Formulae CY201 to CY217:

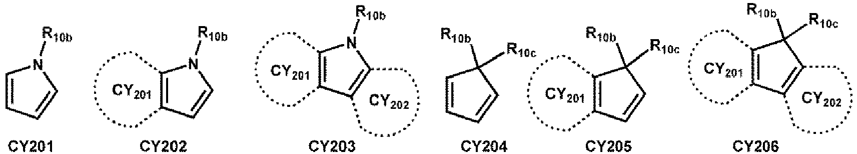

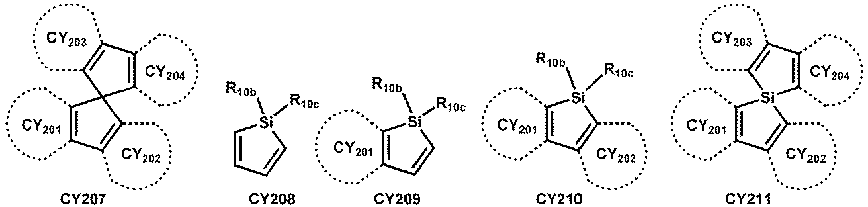

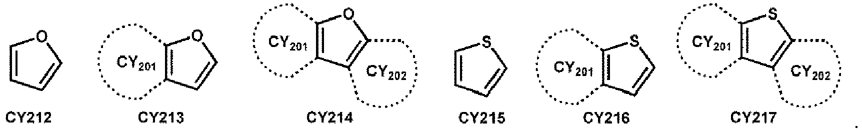

In Formulae CY201 to CY217, R_{10b} and R_{10c} may each be the same as described in connection with R₁₀ₐ, ring CY₂₀₁ to ring CY₂₀₄ may each independently be a C₃-C₂₀ carbocyclic group or a C₁-C₂₀ heterocyclic group, and at least one hydrogen in Formulae CY201 to CY217 may be unsubstituted or substituted with R₁₀ₐ as described above.

In an embodiment, ring CY₂₀₁ to ring CY₂₀₄ in Formulae CY201 to CY217 may each independently be a benzene group, a naphthalene group, a phenanthrene group, or an anthracene group.

In one or more embodiments, each of Formulae 201 and 202 may include at least one of groups represented by Formulae CY201 to CY203.

In one or more embodiments, Formula 201 may include at least one of groups represented by Formulae CY201 to CY203 and at least one of groups represented by Formulae CY204 to CY217.

In one or more embodiments, in Formula 201, xa1 may be 1, R₂₀₁ may be a group represented by one of Formulae CY201 to CY203, xa2 may be 0, and R₂₀₂ may be a group represented by one of Formulae CY204 to CY207.

In one or more embodiments, each of Formulae 201 and 202 may not include a group represented by one of Formulae CY201 to CY203.

In one or more embodiments, each of Formulae 201 and 202 may not include a group represented by one of Formulae CY201 to CY203, and may include at least one of groups represented by Formulae CY204 to CY217.

In one or more embodiments, each of Formulae 201 and 202 may not include a group represented by one of Formulae CY201 to CY217.

For example, the hole transport region may include one of Compounds HT1 to HT46, m-MTDATA, TDATA, 2-TNATA, NPB(NPD), β-NPB, TPD, Spiro-TPD, Spiro-NPB, methylated NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), or any combination thereof:

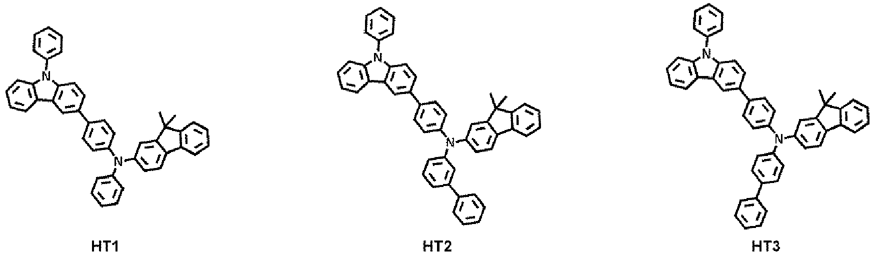

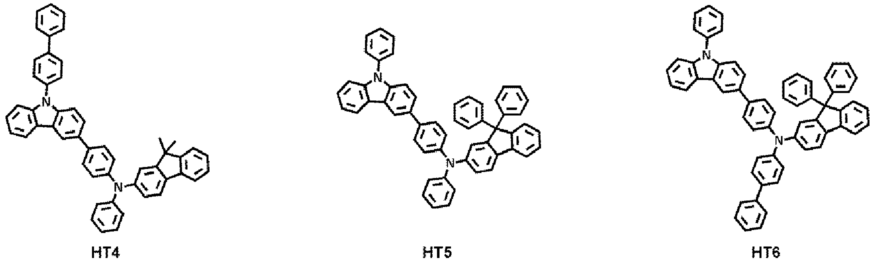

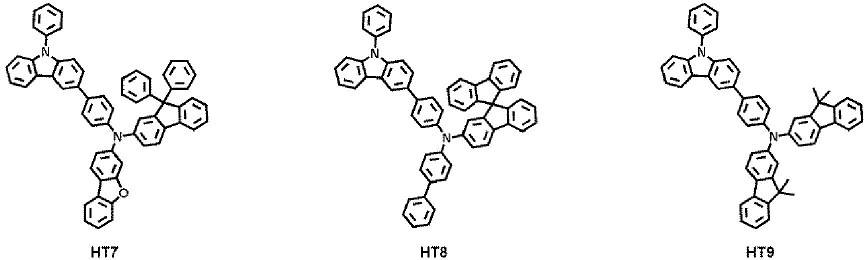

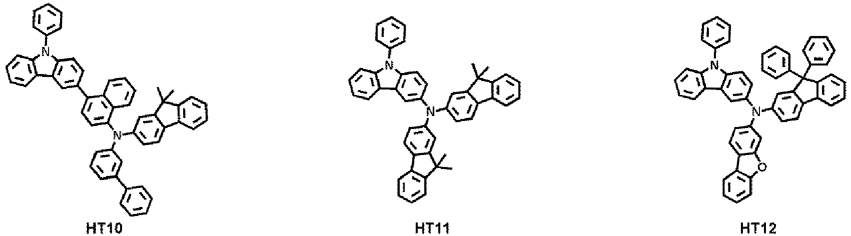

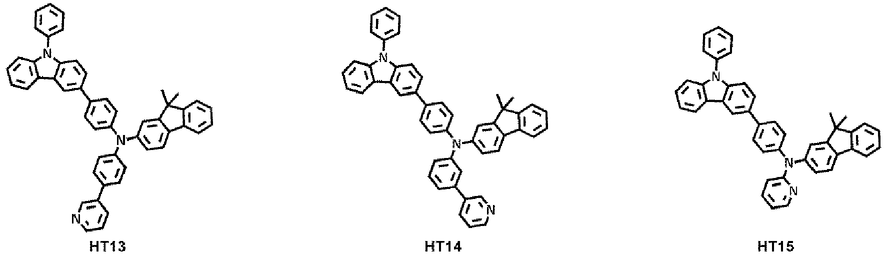

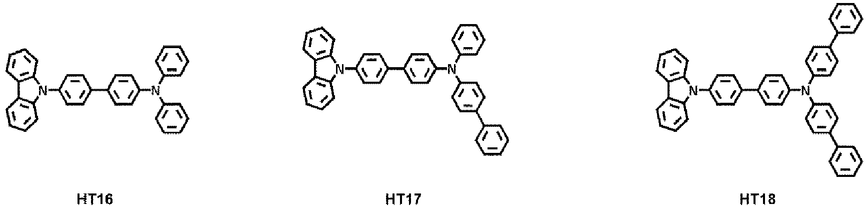

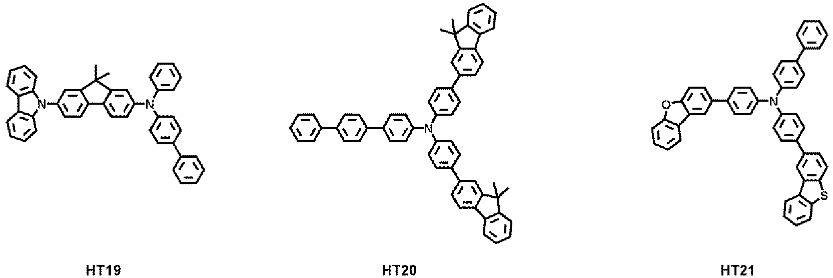

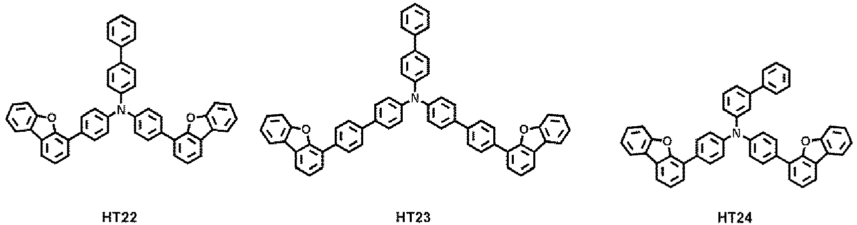

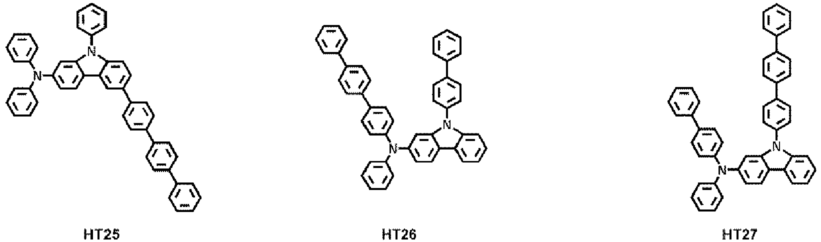

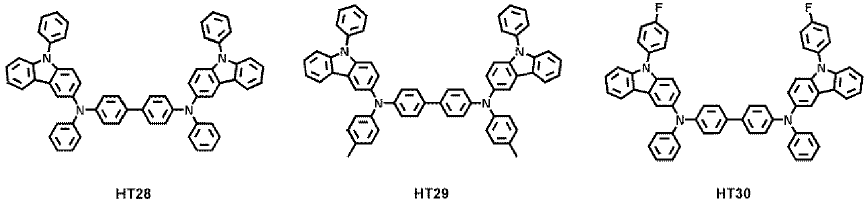

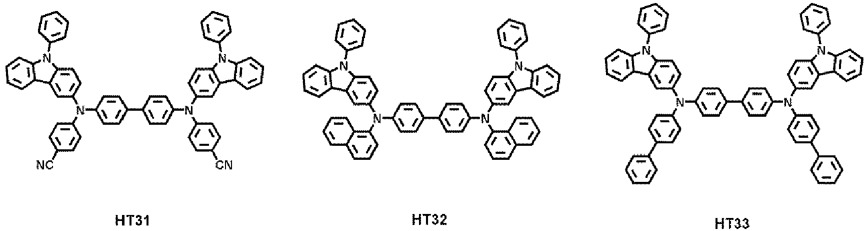

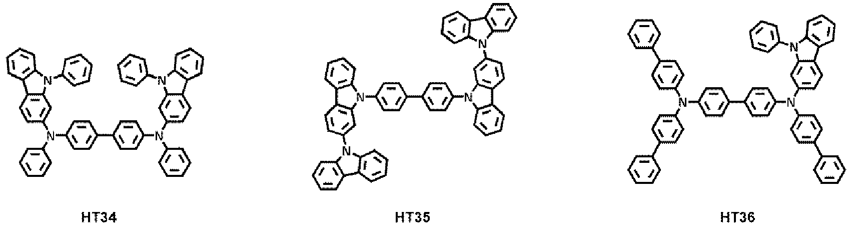

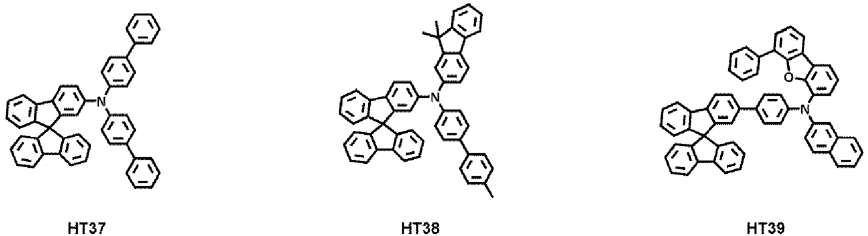

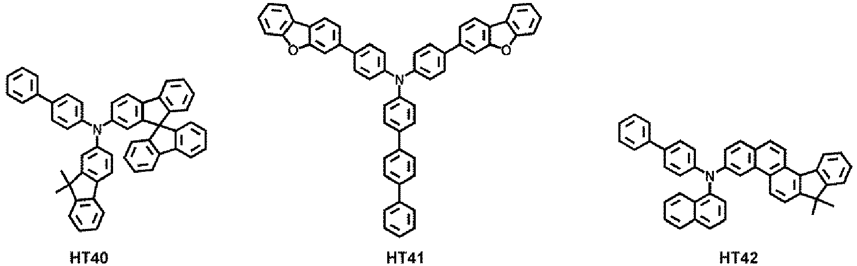

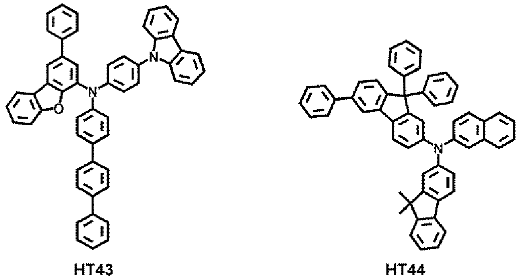

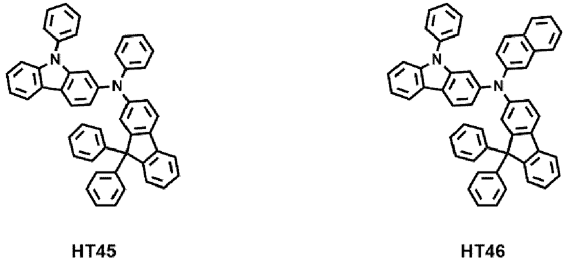

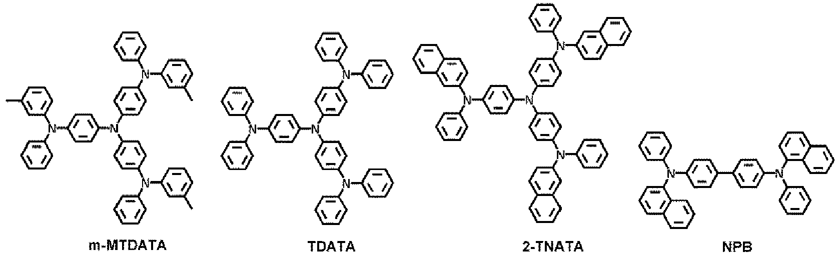

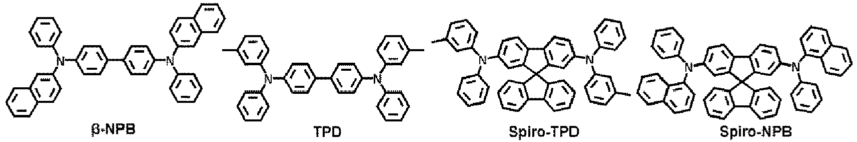

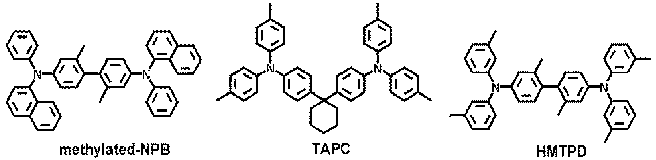

A thickness of the hole transport region may be in a range of about 50 Å to about 10,000 Å, for example, about 100 Å to about 4,000 Å. When the hole transport region includes a hole injection layer, a hole transport layer, or any combination thereof, a thickness of the hole injection layer may be in a range of about 100 Å to about 9,000 Å, for example, about 100 Å to about 1,000 Å, and a thickness of the hole transport layer may be in a range of about 50 Å to about 2,000 Å, for example, about 100 Å to about 1,500 Å. When the thicknesses of the hole transport region, the hole injection layer, and the hole transport layer are within these ranges, satisfactory hole transporting characteristics may be obtained without a substantial increase in driving voltage.

The emission auxiliary layer may increase light-emission efficiency by compensating for an optical resonance distance according to the wavelength of light emitted by the emission layer, and the electron blocking layer may block the leakage of electrons from the emission layer to the hole transport region. Materials that may be included in the hole transport region may be included in the emission auxiliary layer and the electron-blocking layer.

### [p-dopant]

The hole transport region may further include, in addition to these materials, a charge-generation material for the improvement of conductive properties. The charge-generation material may be uniformly or non-uniformly dispersed in the hole transport region (for example, in the form of a single layer consisting of a charge-generation material).

The charge-generation material may be, for example, a p-dopant.

For example, a lowest unoccupied molecular orbital (LUMO) energy level of the p-dopant may be about -3.5 eV or less.

In an embodiment, the p-dopant may include a quinone derivative, a cyano group-containing compound, a compound including element EL1 and element EL2, or any combination thereof.

Examples of the quinone derivative are TCNQ, F4-TCNQ, and the like.

Examples of the cyano group-containing compound are HAT-CN, a compound represented by Formula 221, and the like: 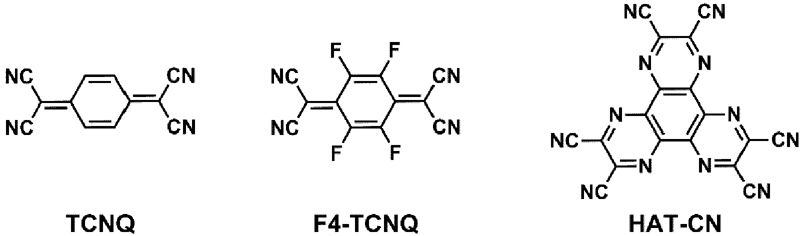 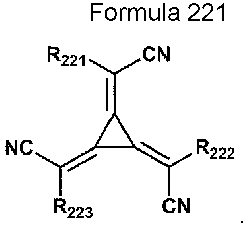 In Formula 221,
R₂₂₁ to R₂₂₃ may each independently be a C₃-C₆₀ carbocyclic group unsubstituted or substituted with at least one R₁₀ₐ or a C₁-C₆₀ heterocyclic group unsubstituted or substituted with at least one R₁₀ₐ, and
at least one of R₂₂₁ to R₂₂₃ may each independently be a C₃-C₆₀ carbocyclic group or a C₁-C₆₀ heterocyclic group, each substituted with: a cyano group; -F; -Cl; -Br; -I; a C₁-C₂₀ alkyl group substituted with a cyano group, -F, -Cl, -Br, -I, or any combination thereof; or any combination thereof.

In the compound including element EL1 and element EL2, element EL1 may be metal, metalloid, or any combination thereof, and element EL2 may be non-metal, metalloid, or any combination thereof.

Examples of the metal are an alkali metal (for example, lithium (Li), sodium (Na), potassium (K), rubidium (Rb), cesium (Cs), and the like); alkaline earth metal (for example, beryllium (Be), magnesium (Mg), calcium (Ca), strontium (Sr), barium (Ba), and the like); transition metal (for example, titanium (Ti), zirconium (Zr), hafnium (Hf), vanadium (V), niobium (Nb), tantalum (Ta), chromium (Cr), molybdenum (Mo), tungsten (W), manganese (Mn), technetium (Tc), rhenium (Re), iron (Fe), ruthenium (Ru), osmium (Os), cobalt (Co), rhodium (Rh), iridium (Ir), nickel (Ni), palladium (Pd), platinum (Pt), copper (Cu), silver (Ag), gold (Au), and the like); post-transition metal (for example, zinc (Zn), indium (In), tin (Sn), and the like); lanthanide metal (for example, lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), lutetium (Lu), and the like); and the like.

Examples of the metalloid are silicon (Si), antimony (Sb), tellurium (Te), and the like.

Examples of the non-metal are oxygen (O), halogen (for example, F, Cl , Br, I, and the like), and the like.

Examples of the compound including element EL1 and element EL2 are metal oxide, metal halide (for example, metal fluoride, metal chloride, metal bromide, metal iodide, and the like), metalloid halide (for example, metalloid fluoride, metalloid chloride, metalloid bromide, metalloid iodide, and the like), metal telluride, or any combination thereof.

Examples of the metal oxide are tungsten oxide (for example, WO, W₂O₃, WO₂, WO₃, W₂O₅, and the like), vanadium oxide (for example, VO, V₂O₃, VO₂, V₂O₅, and the like), molybdenum oxide (MoO, Mo₂O₃, MoO₂, MoO₃, Mo₂O₅, and the like), rhenium oxide (for example, ReOs and the like), and the like.

Examples of the metal halide are alkali metal halide, alkaline earth metal halide, transition metal halide, post-transition metal halide, lanthanide metal halide, and the like.

Examples of the alkali metal halide are LiF, NaF, KF, RbF, CsF, LiCI, NaCl, KCI, RbCI, CsCI, LiBr, NaBr, KBr, RbBr, CsBr, Lil, Nal, KI, Rbl, Csl, and the like.

Examples of the alkaline earth metal halide are BeF₂, MgF₂, CaF₂, SrF₂, BaF₂, BeCl₂, MgCl₂, CaCl₂, SrCl₂, BaCl₂, BeBr₂, MgBr₂, CaBr₂, SrBr₂, BaBr₂, BeI₂, MgI₂, CaI₂, SrI₂, BaI₂, and the like.

Examples of the transition metal halide are titanium halide (for example, TiF₄, TiCl₄, TiBr₄, TiI₄, and the like), zirconium halide (for example, ZrF₄, ZrCl₄, ZrBr₄, ZrI₄, and the like), hafnium halide (for example, HfF₄, HfCl₄, HfBr₄, HfI₄, and the like), vanadium halide (for example, VF₃, VCl₃, VBr₃, VI₃, and the like), niobium halide (for example, NbF₃, NbCl₃, NbBr₃, NbI₃, and the like), tantalum halide (for example, TaF₃, TaCl₃, TaBr₃, TaI₃, and the like), chromium halide (for example, CrF₃, CrCl₃, CrBr₃, CrI₃, and the like), molybdenum halide (for example, MoF₃, MoCl₃, MoBr₃, MoI₃, and the like), tungsten halide (for example, WF₃, WCl₃, WBr₃, WI₃, and the like), manganese halide (for example, MnF₂, MnCl₂, MnBr₂, MnI₂, and the like), technetium halide (for example, TcF₂, TcCl₂, TcBr₂, TcI₂, and the like), rhenium halide (for example, ReF₂, ReCl₂, ReBr₂, ReI₂, and the like), iron halide (for example, FeF₂, FeCl₂, FeBr₂, FeI₂, and the like), ruthenium halide (for example, RuF₂, RuCl₂, RuBr₂, RuI₂, and the like), osmium halide (for example, OsF₂, OsCl₂, OsBr₂, OsI₂, and the like), cobalt halide (for example, CoF₂, CoCl₂, CoBr₂, CoI₂, and the like), rhodium halide (for example, RhF₂, RhCl₂, RhBr₂, RhI₂, and the like), iridium halide (for example, IrF₂, IrCl₂, IrBr₂, IrI₂, and the like), nickel halide (for example, NiF₂, NiCl₂, NiBr₂, NiI₂, and the like), palladium halide (for example, PdF₂, PdCl₂, PdBr₂, PdI₂, and the like), platinum halide (for example, PtF₂, PtCl₂, PtBr₂, PtI₂, and the like), copper halide (for example, CuF, CuCI, CuBr, Cul, and the like), silver halide (for example, AgF, AgCl, AgBr, Agl, and the like), gold halide (for example, AuF, AuCI, AuBr, Aul, and the like), and the like

Examples of the post-transition metal halide are zinc halide (for example, ZnF₂, ZnCl₂, ZnBr₂, ZnI₂, and the like), indium halide (for example, InI₃ and the like), tin halide (for example, SnI₂ and the like), and the like

Examples of the lanthanide metal halide are YbF, YbF₂, YbF₃, SmF₃, YbCl, YbCl₂, YbCl₃ SmCl₃, YbBr, YbBr₂, YbBr₃ SmBr₃, Ybl, YbI₂, YbI₃, SmI₃, and the like.

Examples of the metalloid halide antimony halide (for example, SbCl₅ and the like) and the like.

Examples of the metal telluride are alkali metal telluride (for example, Li₂Te, Na₂Te, K₂Te, Rb₂Te, Cs₂Te, and the like), alkaline earth metal telluride (for example, BeTe, MgTe, CaTe, SrTe, BaTe, and the like), transition metal telluride (for example, TiTe₂, ZrTe₂, HfTe₂, V₂Te₃, Nb₂Te₃, Ta₂Te₃, Cr₂Te₃, Mo₂Te₃, W₂Te₃, MnTe, TcTe, ReTe, FeTe, RuTe, OsTe, CoTe, RhTe, IrTe, NiTe, PdTe, PtTe, Cu₂Te, CuTe, Ag₂Te, AgTe, Au₂Te, and the like), post-transition metal telluride (for example, ZnTe, and the like), lanthanide metal telluride (for example, LaTe, CeTe, PrTe, NdTe, PmTe, EuTe, GdTe, TbTe, DyTe, HoTe, ErTe, TmTe, YbTe, LuTe, and the like), and the like.

### [Emission layer in interlayer 130]

When the light-emitting device 10 is a full-color light-emitting device, the emission layer may be patterned into a red emission layer, a green emission layer, and/or a blue emission layer, according to a sub-pixel. In an embodiment, the emission layer may have a stacked structure of two or more layers of a red emission layer, a green emission layer, and a blue emission layer, wherein the two or more layers contact each other or are separated from each other layer to emit white light. In one or more embodiments, the emission layer may include two or more materials of a red light-emitting material, a green light-emitting material, and a blue light-emitting material, wherein the two or more materials are mixed with each other in a single layer to emit white light.

In an embodiment, the emission layer may include a host and a dopant. The dopant may include a phosphorescent dopant, a fluorescent dopant, or any combination thereof.

In the emission layer, an amount of the dopant may be in a range of about 0.01 parts by weight to about 15 parts by weight based on 100 parts by weight of the host.

In an embodiment, the emission layer may include a quantum dot.

In one or more embodiments, the emission layer may include a delayed fluorescence material. The delayed fluorescence material may act as a host or a dopant in the emission layer.

A thickness of the emission layer may be in a range of about 100 Å to about 1,000 Å, for example, about 200 Å to about 600 Å. When the thickness of the emission layer is within these ranges, excellent luminescence characteristics may be obtained without a substantial increase in driving voltage.

### [Host]

In an embodiment, the host may include a compound represented by Formula 301:

Formula 301 [Ar₃₀₁]_{xb11}-[(L₃₀₁)_{xb1}-R₃₀₁]_{xb21}

[00238] wherein, in Formula 301,
Ar₃₀₁ and L₃₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xb11 may be 1, 2, or 3,
xb1 may be an integer from 0 to 5,
R₃₀₁ may be hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ,-Si(Q₃₀₁)(Q₃₀₂)(Q₃₀₃), -N(Q301)(Q302), - B(Q₃₀₁)(Q₃₀₂), -C(=O)(Q₃₀₁), -S(=O)₂(Q₃₀₁), or -P(=O)(Q₃₀₁)(Q₃₀₂),
xb21 may be an integer from 1 to 5, and
Q₃₀₁ to Q₃₀₃ may each be the same as described in connection with Q₁.

For example, when xb11 in Formula 301 is 2 or more, two or more of Ar₃₀₁ may be linked together via a single bond.

In one or more embodiments, the host may include a compound represented by Formula 301-1, a compound represented by Formula 301-2, or any combination thereof: 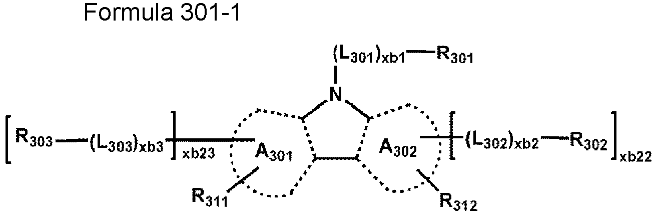 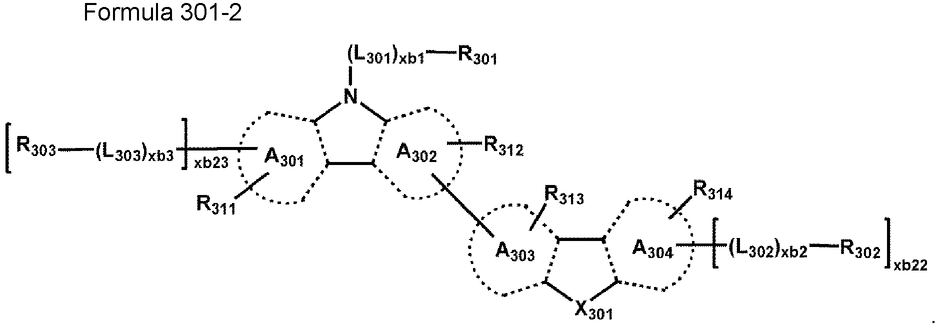 In Formulae 301-1 and 301-2,
ring A₃₀₁ to ring A₃₀₄ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
X₃₀₁ may be O, S, N-[(L₃₀₄)_{xb4}-R₃₀₄], C(R₃₀₄)(R₃₀₅), or Si(R₃₀₄)(R₃₀₅),
xb22 and xb23 may each independently be 0, 1, or 2,
L₃₀₁, xb1, and R₃₀₁ may each be the same as described herein,
L₃₀₂ to L₃₀₄ may each independently be the same as described in connection with L301,
xb2 to xb4 may each independently be the same as described in connection with xb1, and
R₃₀₂ to R₃₀₅ and R₃₁₁ to R₃₁₄ may each be the same as described in connection with R₃₀₁.

In one or more embodiments, the host may include an alkali earth metal complex, a post-transition metal complex, or any combination thereof. For example, the host may include a Be complex (for example, Compound H55), an Mg complex, a Zn complex, or any combination thereof.

In one or more embodiments, the host may include one of Compounds H1 to H124, 9,10-di(2-naphthyl)anthracene (ADN), 2-methyl-9,10-bis(naphthalen-2-yl)anthracene (MADN), 9,10-di-(2-naphthyl)-2-t-butyl-anthracene (TBADN), 4,4'-bis(N-carbazolyl)-1,1'-biphenyl (CBP), 1,3-di-9-carbazolylbenzene (mCP), 1,3,5-tri(carbazol-9-yl)benzene (TCP), or any combination thereof:

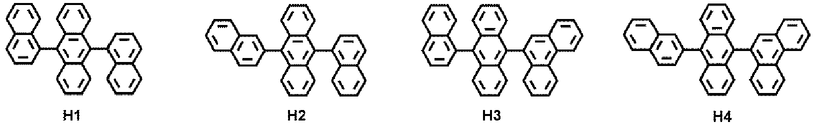

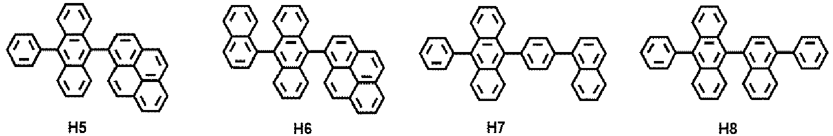

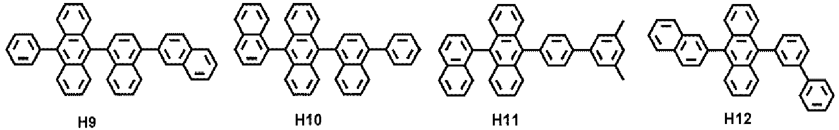

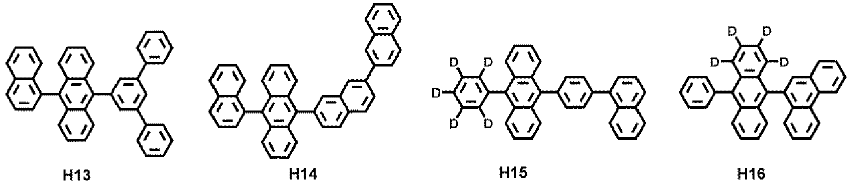

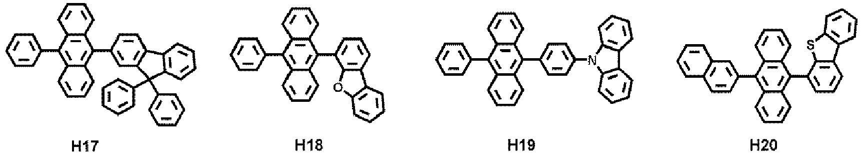

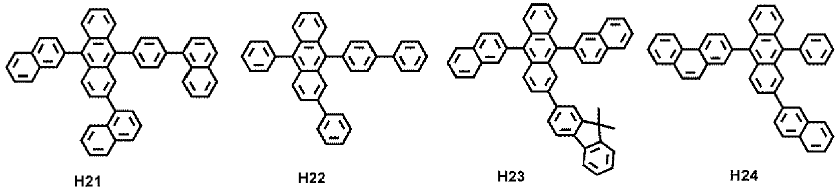

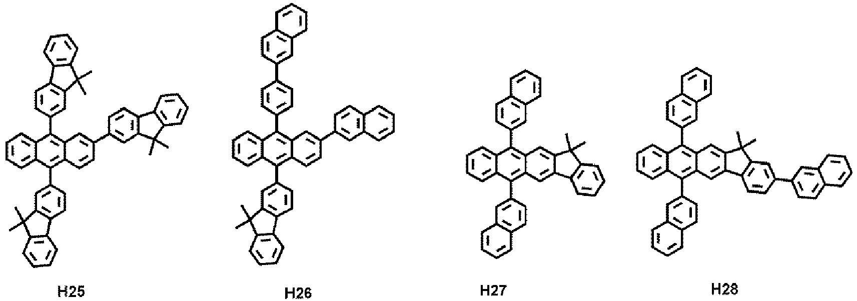

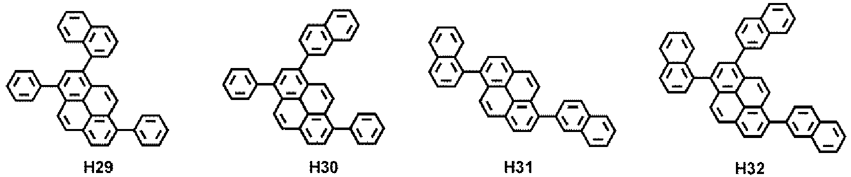

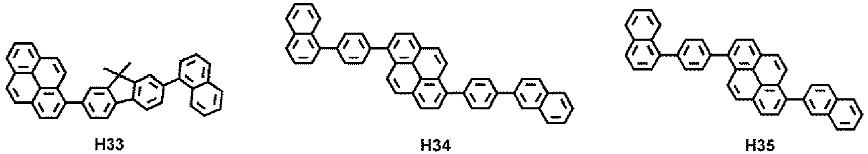

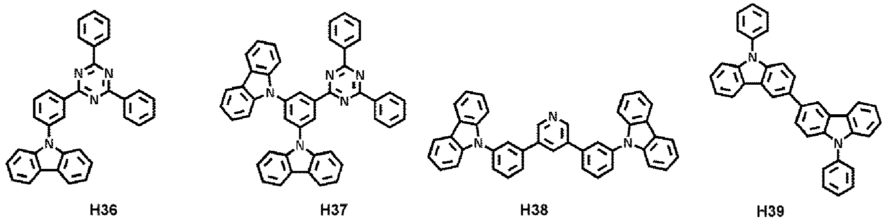

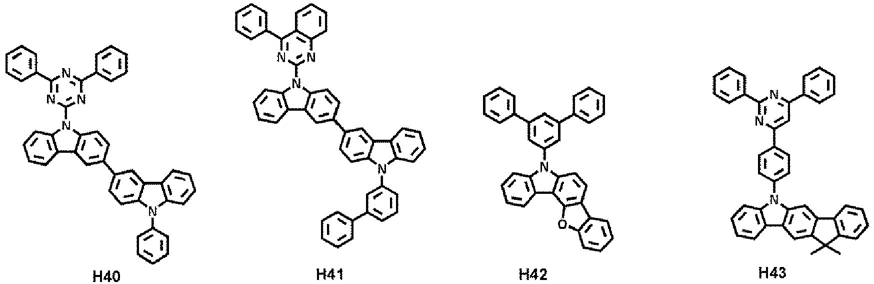

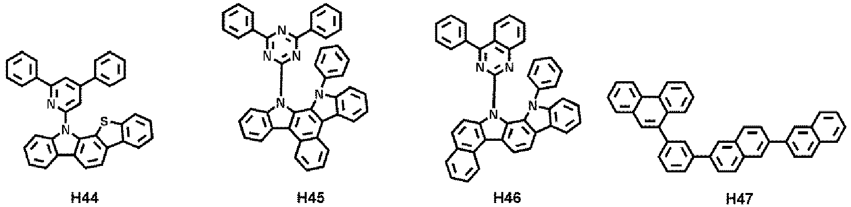

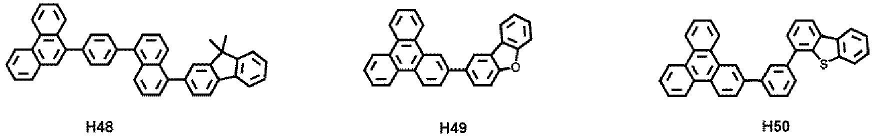

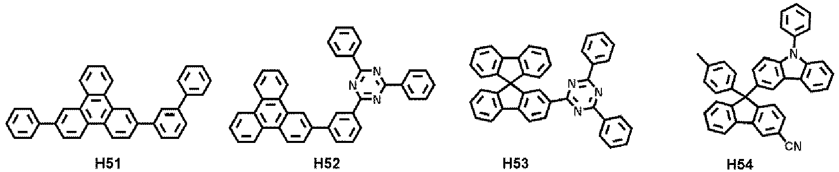

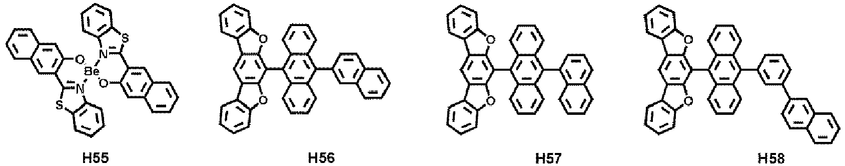

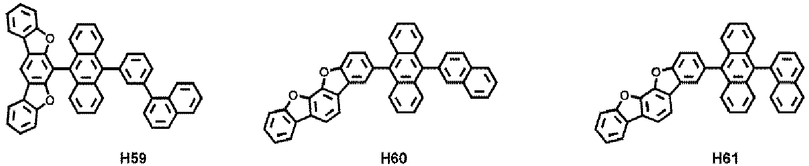

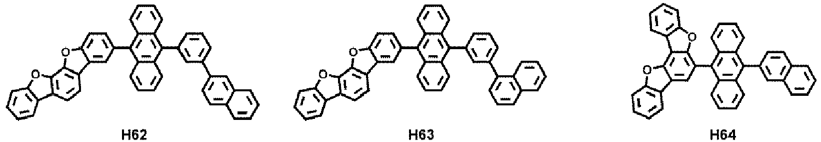

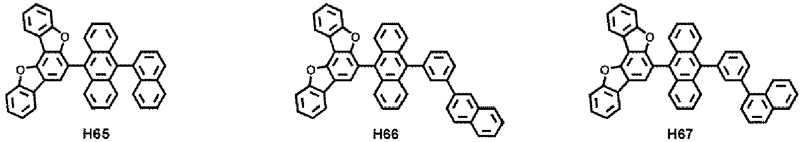

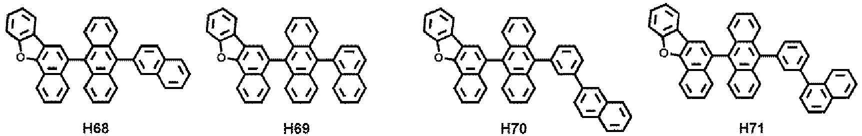

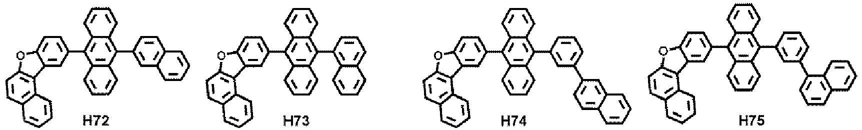

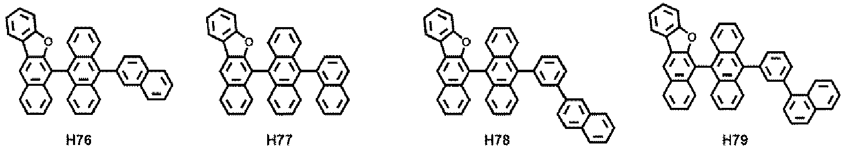

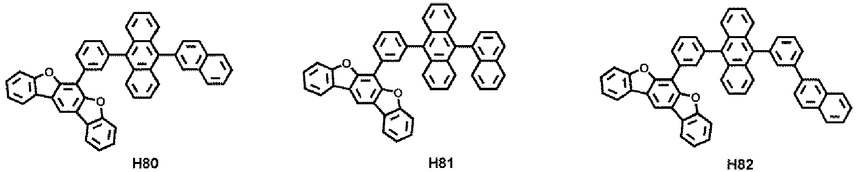

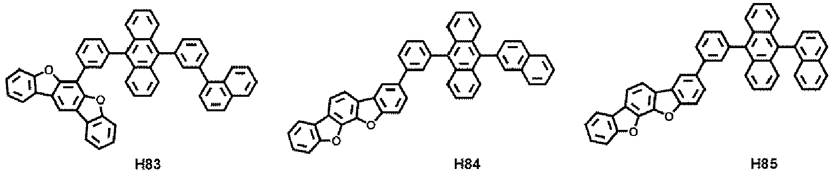

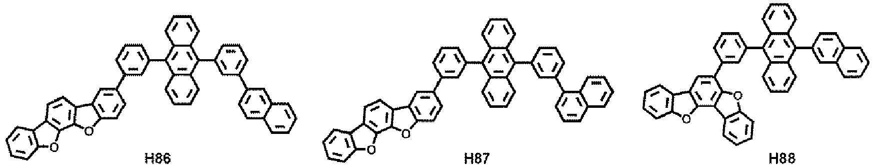

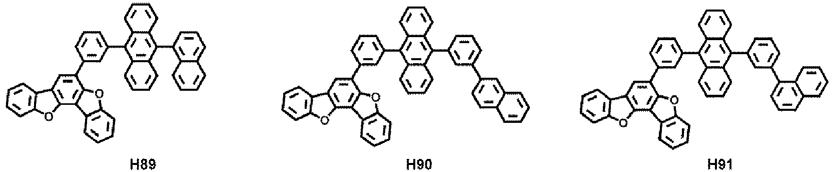

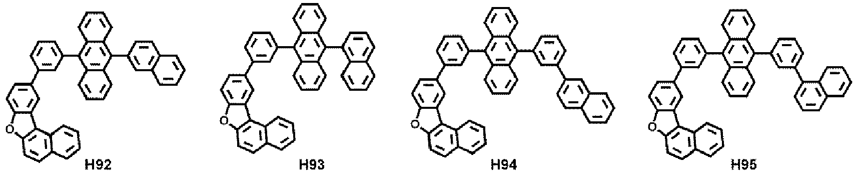

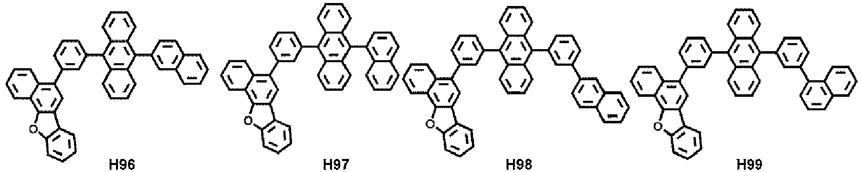

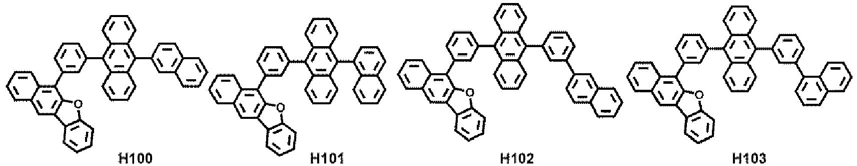

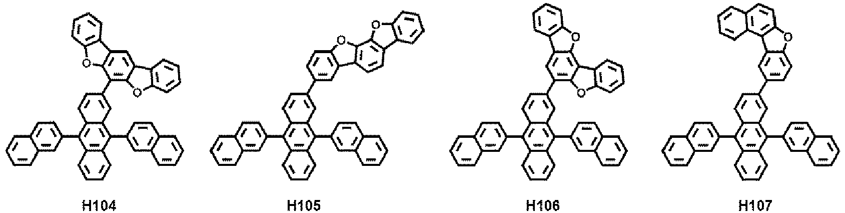

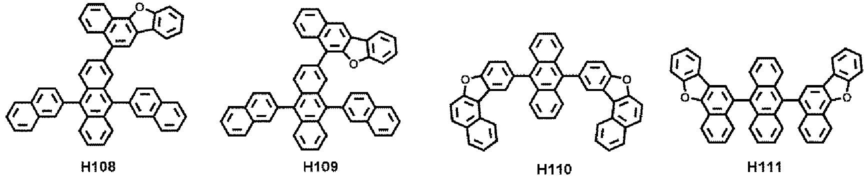

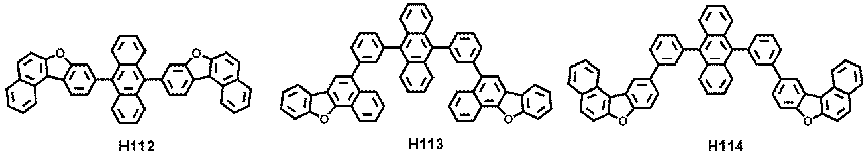

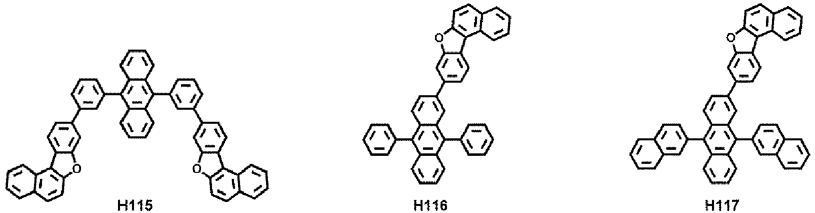

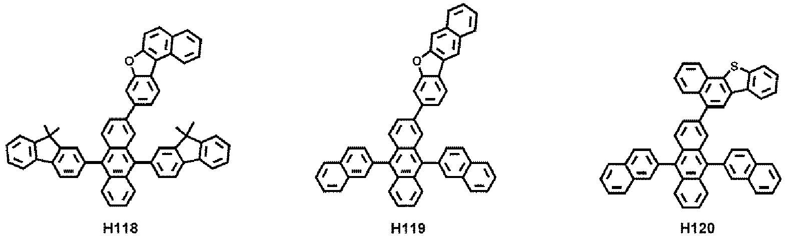

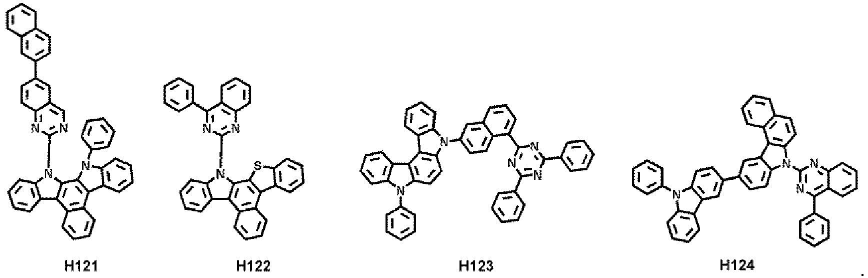

### [Phosphorescent dopant]

In an embodiment, the phosphorescent dopant may include at least one transition metal as a central metal.

The phosphorescent dopant may include a monodentate ligand, a bidentate ligand, a tridentate ligand, a tetradentate ligand, a pentadentate ligand, a hexadentate ligand, or any combination thereof.

The phosphorescent dopant may be electrically neutral.

In one or more embodiments, the phosphorescent dopant may include an organometallic compound represented by Formula 401:

Formula 401 M(L₄₀₁)_{xc1}(L₄₀₂)_{xc2}

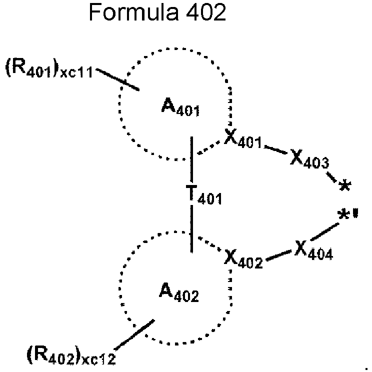

In Formulae 401 and 402,
M may be a transition metal (for example, iridium (Ir), platinum (Pt), palladium (Pd), osmium (Os), titanium (Ti), gold (Au), hafnium (Hf), europium (Eu), terbium (Tb), rhodium (Rh), rhenium (Re), or thulium (Tm)),
L₄₀₁ may be a ligand represented by Formula 402, and xc1 may be 1, 2, or 3, wherein, when xc1 is 2 or more, two or more of L₄₀₁ may be identical to or different from each other,
L₄₀₂ may be an organic ligand, and xc2 may be 0, 1, 2, 3, or 4, wherein, when xc2 is 2 or more, two or more of L₄₀₂ may be identical to or different from each other,
X₄₀₁ and X₄₀₂ may each independently be nitrogen or carbon,
ring A₄₀₁ and ring A₄₀₂ may each independently be a C₃-C₆₀ carbocyclic group or a C₁-C₆₀ heterocyclic group,
T₄₀₁ may be a single bond, *-O-*', *-S-*', *-C(=O)-*', *-N(Q₄₁₁)-*', *-C(Q₄₁₁)(Q₄₁₂)-*', *-C(Q₄₁₁)=C(Q₄₁₂)-*', *-C(Q₄₁₁)=*', or*=C(Q₄₁₁)=*',
X₄₀₃ and X₄₀₄ may each independently be a chemical bond (for example, a covalent bond or a coordinate bond), O, S, N(Q413), B(Q413), P(Q413), C(Q413)(Q414), or Si(Q413)(Q414),
Q₄₁₁ to Q₄₁₄ may each be the same as described in connection with Q₁,
R₄₀₁ and R₄₀₂ may each independently be hydrogen, deuterium, -F, -Cl, -Br, -I, a hydroxyl group, a cyano group, a nitro group, a C₁-C₂₀ alkyl group unsubstituted or substituted with at least one R₁₀ₐ, a C₁-C₂₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, - Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), -S(=O)₂(Q₄₀₁), or - P(=O)(Q₄₀₁)(Q402),
Q₄₀₁ to Q₄₀₃ may each be the same as described in connection with Q₁,
xc11 and xc12 may each independently be an integer from 0 to 10, and
* and *' in Formula 402 each indicate a binding site to M in Formula 401.

For example, in Formula 402, i) X₄₀₁ may be nitrogen, and X₄₀₂ may be carbon, or ii) each of X₄₀₁ and X₄₀₂ may be nitrogen.

In an embodiment, when xc1 in Formula 401 is 2 or more, two ring A₄₀₁(s) among two or more of L₄₀₁ may optionally be linked to each other via T₄₀₂, which is a linking group, and two ring A₄₀₂(s) among two or more of L₄₀₁ may optionally be linked to each other via T₄₀₃, which is a linking group (see Compounds PD1 to PD4 and PD7). T₄₀₂ and T₄₀₃ may each be the same as described in connection with T₄₀₁.

In Formula 401, L₄₀₂ may be an organic ligand. For example, L₄₀₂ may include a halogen group, a diketone group (for example, an acetylacetonate group), a carboxylic acid group (for example, a picolinate group), -C(=O), an isonitrile group, a -CN group, a phosphorus group (for example, a phosphine group, a phosphite group, and the like), or any combination thereof.

The phosphorescent dopant may include, for example, one of Compounds PD1 to PD39, or any combination thereof:

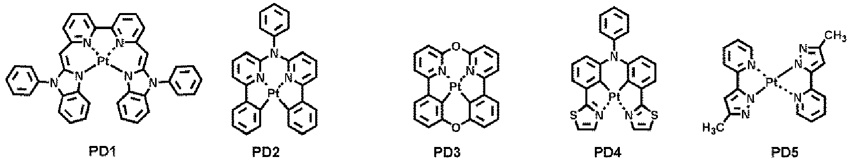

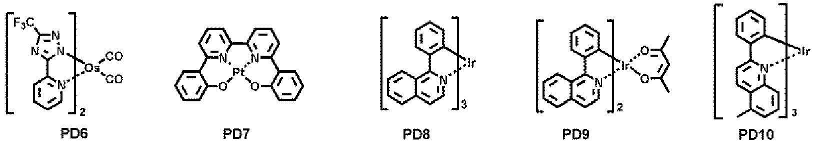

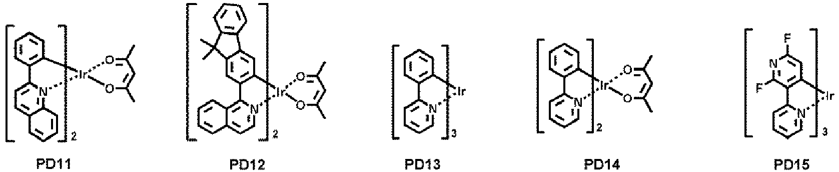

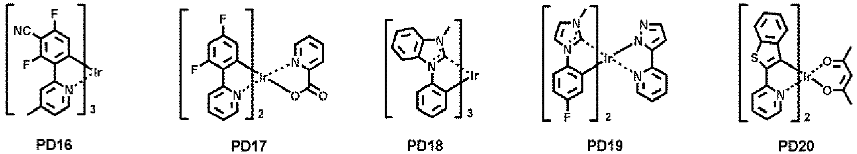

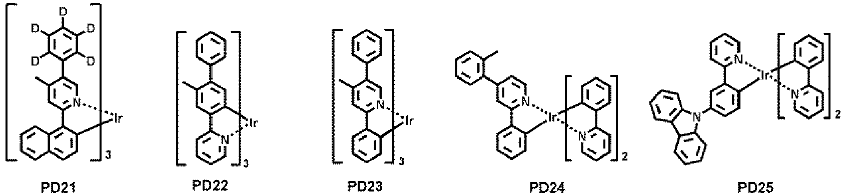

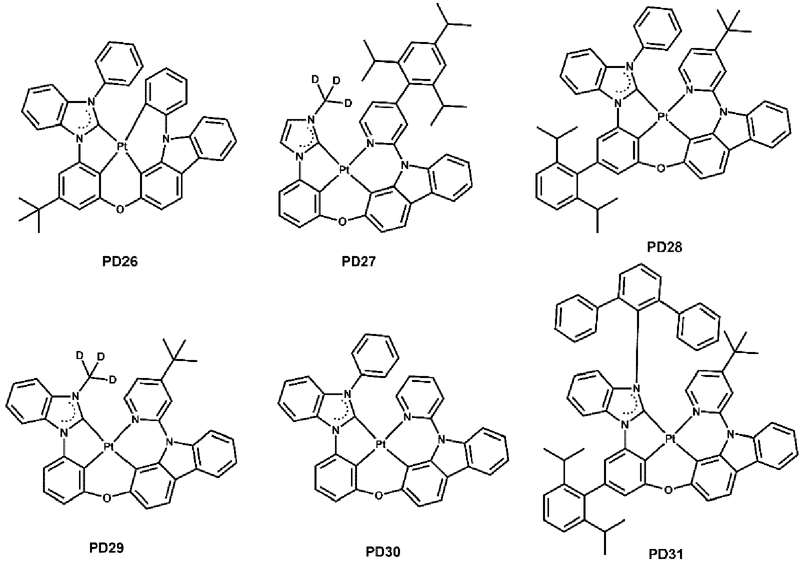

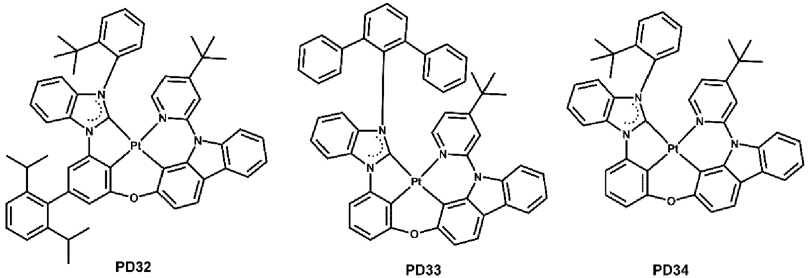

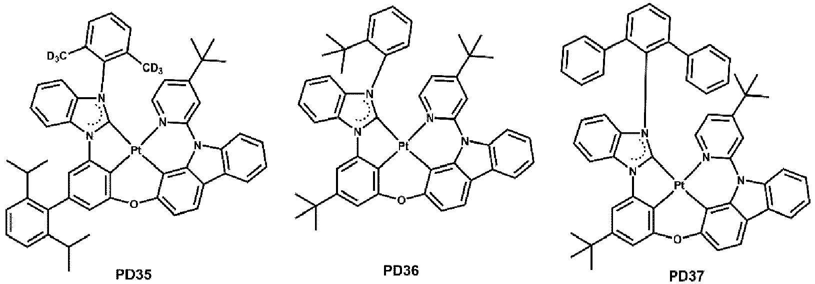

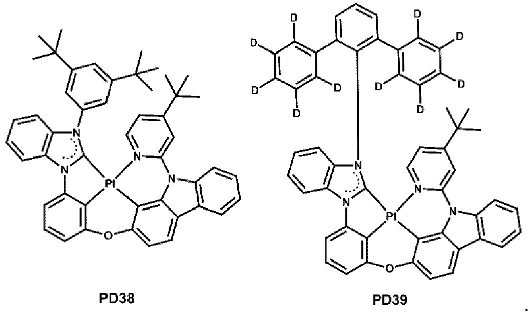

### [Fluorescent dopant]

The fluorescent dopant may include an amine group-containing compound, a styryl group-containing compound, or any combination thereof.

For example, the fluorescent dopant may include a compound represented by Formula 501: 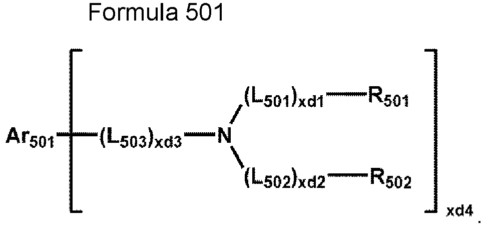 In Formula 501,
Ar₅₀₁, L₅₀₁ to L₅₀₃, R₅₀₁, and R₅₀₂ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xd1 to xd3 may each independently be 0, 1, 2, or 3, and
xd4 may be 1, 2, 3, 4, 5, or 6.

For example, Ar₅₀₁ in Formula 501 may be a condensed cyclic group (for example, an anthracene group, a chrysene group, a pyrene group, and the like) in which three or more monocyclic groups are condensed together.

For example, xd4 in Formula 501 may be 2.

In one or more embodiments, the fluorescent dopant may include: one of Compounds FD1 to FD36; DPVBi; DPAVBi; or any combination thereof:

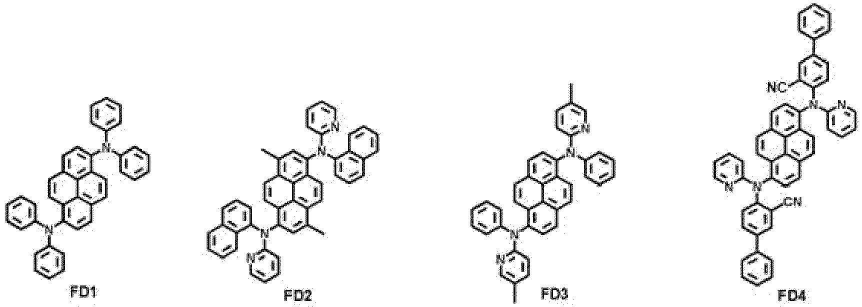

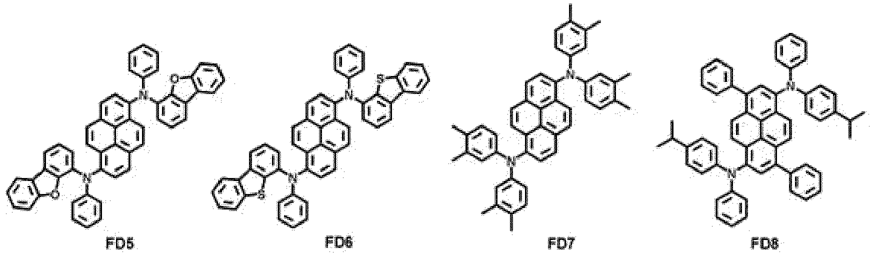

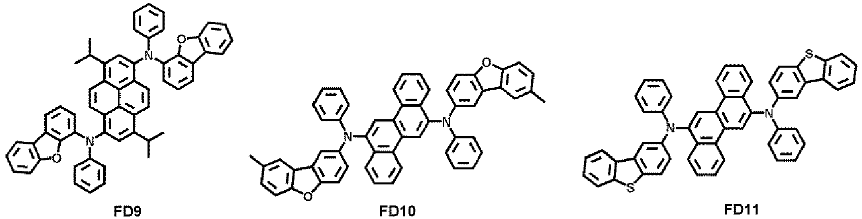

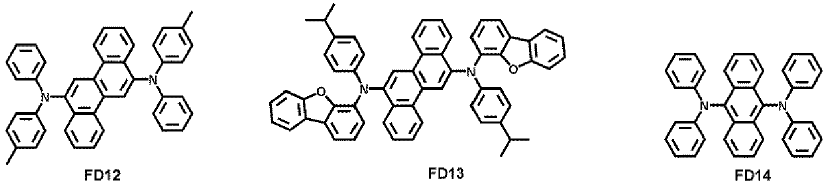

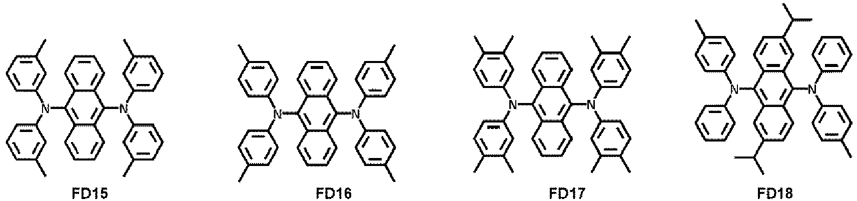

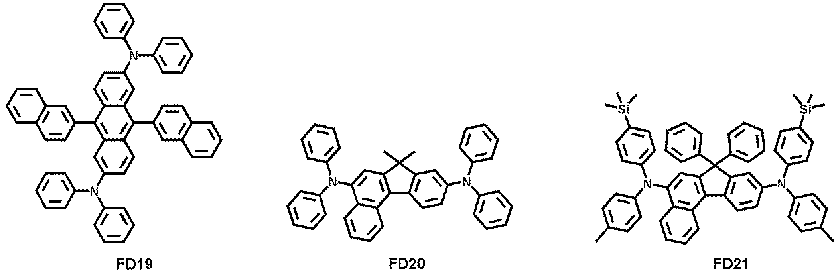

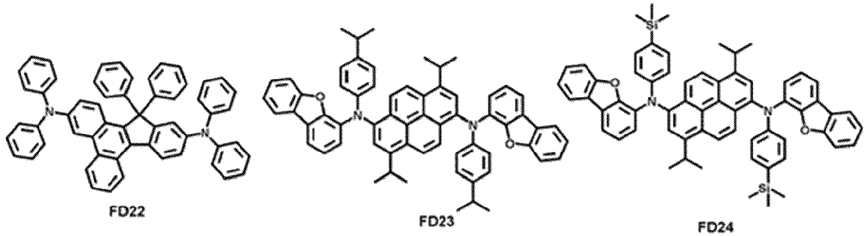

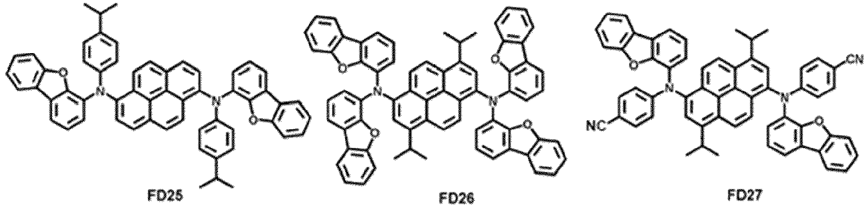

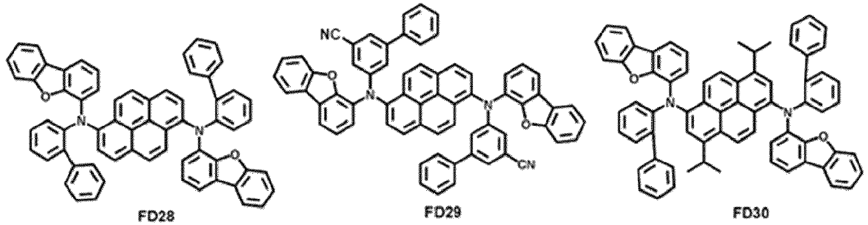

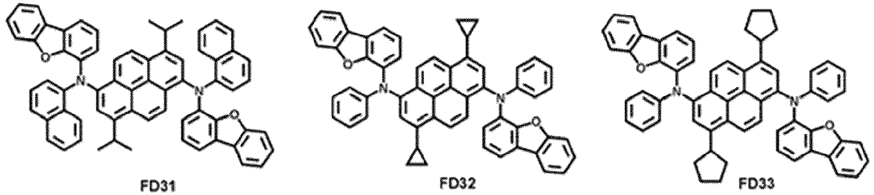

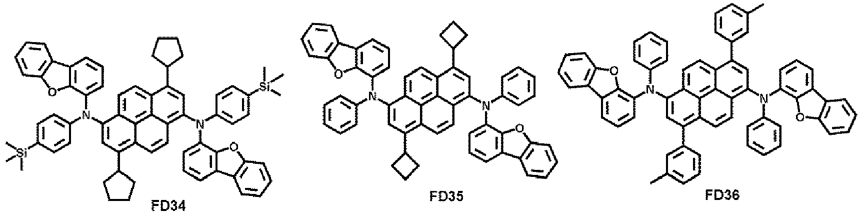

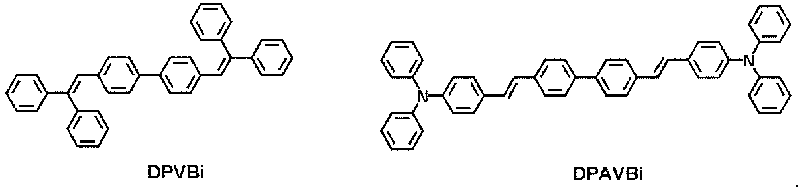

### [Delayed fluorescence material]

The emission layer may include a delayed fluorescence material.

In the present specification, the delayed fluorescence material may be selected from compounds capable of emitting delayed fluorescence by a delayed fluorescence emission mechanism.

The delayed fluorescence material included in the emission layer may act as a host or a dopant, depending on the type of other materials included in the emission layer.

In an embodiment, a difference between a triplet energy level (eV) of the delayed fluorescence material and a singlet energy level (eV) of the delayed fluorescence material may be about 0 eV or more and about 0.5 eV or less. When the difference between the triplet energy level (eV) of the delayed fluorescence material and the singlet energy level (eV) of the delayed fluorescence material is satisfied within the ranges above, up-conversion from the triplet state to the singlet state of the delayed fluorescence materials may effectively occur, thereby improving luminescence efficiency or the like of the light-emitting device 10.

For example, the delayed fluorescence material may include: i) a material including at least one electron donor (for example, a π electron-rich C₃-C₆₀ cyclic group and the like, such as a carbazole group) and at least one electron acceptor (for example, a sulfoxide group, a cyano group, a π electron-deficient nitrogen-containing C₁-C₆₀ cyclic group, and the like); and ii) a material including a C₈-C₆₀ polycyclic group in which two or more cyclic groups are condensed while sharing boron (B).

Examples of the delayed fluorescence material are at least one of Compounds DF1 to DF9:

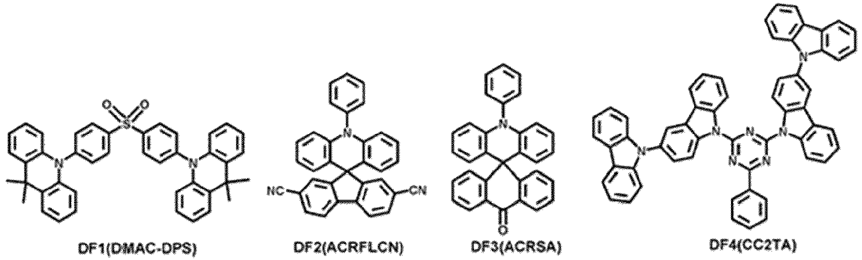

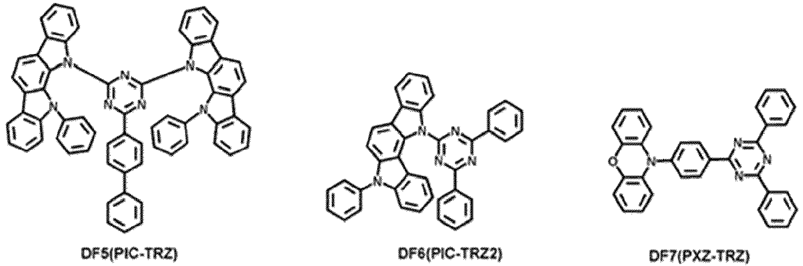

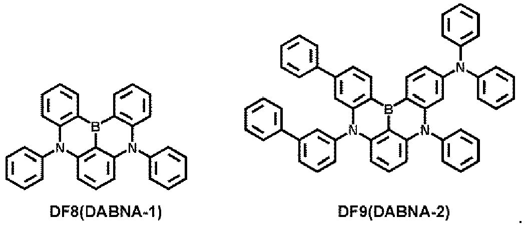

### [Quantum dot]

The emission layer may include a quantum dot.

The term "quantum dot" as used herein refers to a crystal of a semiconductor compound, and may include any material capable of emitting light of various emission wavelengths according to the size of the crystal.

A diameter of the quantum dot may be, for example, in a range of about 1 nm to about 10 nm.

The quantum dot may be synthesized by a wet chemical process, a metal organic chemical vapor deposition process, a molecular beam epitaxy process, or any process similar thereto.

The wet chemical process is a method including mixing a precursor material with an organic solvent and then growing a quantum dot particle crystal. When the crystal grows, the organic solvent naturally acts as a dispersant coordinated on the surface of the quantum dot crystal and controls the growth of the crystal so that the growth of quantum dot particles can be controlled through a process which costs lower, and is easier than vapor deposition methods, such as metal organic chemical vapor deposition (MOCVD) or molecular beam epitaxy (MBE),
The quantum dot may include: a Group II-VI semiconductor compound; a Group III-V semiconductor compound; a Group III-VI semiconductor compound; a Group I-III-VI semiconductor compound; a Group IV-VI semiconductor compound; a Group IV element or compound; or any combination thereof.

Examples of the Group II-VI semiconductor compound are: a binary compound, such as CdSe, CdTe, ZnS, ZnSe, ZnTe, ZnO, HgS, HgSe, HgTe, MgSe, MgS, and the like; a ternary compound, such as CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, and the like; a quaternary compound, such as CdZnSeS, CdZnSeTe, CdZnSTe, CdHgSeS, CdHgSeTe, CdHgSTe, HgZnSeS, HgZnSeTe, HgZnSTe, and the like; or any combination thereof.

Examples of the Group III-V semiconductor compound are: a binary compound, such as GaN, GaP, GaAs, GaSb, AIN, AIP, AlAs, AlSb, InN, InP, InAs, InSb, and the like; a ternary compound, such as GaNP, GaNAs, GaNSb, GaPAs, GaPSb, AINP, AINAs, AINSb, AIPAs, AlPSb, InGaP, InNP, InAlP, InNAs, InNSb, InPAs, InPSb, and the like; a quaternary compound, such as GaAINP, GaAINAs, GaAINSb, GaAIPAs, GaAIPSb, GalnNP, GalnNAs, GaInNSb, GalnPAs, GalnPSb, InAINP, InAINAs, InAINSb, InAIPAs, InAIPSb, and the like; or any combination thereof. The Group III-V semiconductor compound may further include a Group II element. Examples of the Groups III-V semiconductor compound further including a Group II element are InZnP, InGaZnP, InAIZnP, and the like

Examples of the Group III-VI semiconductor compound are: a binary compound, such as GaS, GaSe, Ga₂Se₃, GaTe, InS, InSe, In₂S₃, In₂Se₃, InTe, and the like; a ternary compound, such as InGaS₃, InGaSe₃, and the like; or any combination thereof.

Examples of the Group I-III-VI semiconductor compound are: a ternary compound, such as AgInS, AgInS₂, CulnS, CuInS₂, CuGaO₂, AgGaO₂, AgAlO₂, and the like; or any combination thereof.

Examples of the Group IV-VI semiconductor compound are: a binary compound, such as SnS, SnSe, SnTe, PbS, PbSe, PbTe, and the like; a ternary compound, such as SnSeS, SnSeTe, SnSTe, PbSeS, PbSeTe, PbSTe, SnPbS, SnPbSe, SnPbTe, and the like; a quaternary compound, such as SnPbSSe, SnPbSeTe, SnPbSTe, and the like; or any combination thereof.

The Group IV element or compound may include: a single element compound, such as Si, Ge, and the like; a binary compound, such as SiC, SiGe, and the like; or any combination thereof.

Each element included in a multi-element compound, such as the binary compound, the ternary compound, and the quaternary compound, may exist in a particle thereof at a uniform concentration or non-uniform concentration.

The quantum dot may have a single structure or a dual core-shell structure. In the case of the quantum dot having a single structure, a concentration of each element included in the corresponding quantum dot may be uniform. For example, a material included in the core and a material included in the shell may be different from each other.

The shell of the quantum dot may act as a protective layer that prevents chemical degeneration of the core to maintain semiconductor characteristics, and/or as a charging layer that imparts electrophoretic characteristics to the quantum dot. The shell may be a single layer or a multi-layer. The interface between the core and the shell may have a concentration gradient in which the concentration of an element existing in the shell decreases toward the center of the core.

Examples of the shell of the quantum dot are oxide of metal, metalloid, or non-metal, a semiconductor compound, or any combination thereof. Examples of the oxide of metal, metalloid, or non-metal are: a binary compound, such as SiO₂, Al₂O₃, TiO₂, ZnO, MnO, Mn₂O₃, Mn₃O₄, CuO, FeO, Fe₂O₃, Fe₃O₄, CoO, Co₃O₄, NiO, and the like; a ternary compound, such as MgAl₂O₄, CoFe₂O₄, NiFe₂O₄, CoMn₂O₄, and the like; or any combination thereof. Examples of the semiconductor compound are: as described herein, a Group II-VI semiconductor compound; a Group III-V semiconductor compound; a Group III-VI semiconductor compound; a Group I-III-VI semiconductor compound; a Group IV-VI semiconductor compound; or any combination thereof. Examples of the semiconductor compound are CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AIP, AlSb, or any combination thereof.

A full width of half maximum (FWHM) of an emission wavelength spectrum of the quantum dot may be about 45 nm or less, for example, about 40 nm or less, for example, about 30 nm or less, and within these ranges, color purity or color reproducibility may be increased. In addition, since the light emitted through the quantum dot is emitted in all directions, the wide viewing angle may be improved.

In addition, the quantum dot may be specifically, spherical, pyramidal, multi-arm, or cubic nanoparticles, nanotubes, nanowires, nanofibers, or nanoplate particles.

Since the energy band gap may be adjusted by controlling the size of the quantum dot, light having various wavelength bands may be obtained from the emission layer including the quantum dot. Accordingly, by using quantum dot of different sizes, a light-emitting device that emits light of various wavelengths may be implemented. In detail, the size of the quantum dot may be selected in consideration of emitting red light, green light, and/or blue light. In addition, the size of the quantum dot may be configured to emit white light by combination of light of various colors.

### [Electron transport region in interlayer 130]

The electron transport region may have: i) a single-layered structure consisting of a single layer consisting of a single material, ii) a single-layered structure consisting of a single layer consisting of a plurality of different materials, or iii) a multi-layered structure including a plurality of layers including different materials.

The electron-transporting region may include a buffer layer, a hole blocking layer, an electron control layer, an electron transport layer, an electron injection layer, or any combination thereof.

For example, the electron transport region may have an electron transport layer/electron injection layer structure, a hole blocking layer/electron transport layer/electron injection layer structure, an electron control layer/electron transport layer/electron injection layer structure, or a buffer layer/electron transport layer/electron injection layer structure, wherein constituent layers of each structure are sequentially stacked from the emission layer.

The electron transport region (for example, the buffer layer, the hole blocking layer, the electron control layer, or the electron transport layer in the electron transport region) may include a metal-free compound including at least one π electron-deficient nitrogen-containing C₁-C₆₀ cyclic group.

For example, the electron transport region may include a compound represented by Formula 601:

Formula 601 [Ar₆₀₁]ₓₑ₁₁-[(L₆₀₁)ₓₑ₁-R₆₀₁]ₓₑ₂₁

wherein, in Formula 601,
Ar₆₀₁ and L₆₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xe11 may be 1, 2, or 3,
xe1 may be 0, 1, 2, 3, 4, or 5,
R₆₀₁ may be a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₆₀₁)(Q₆₀₂)(Q₆₀₃), -C(=O)(Q₆₀₁), -S(=O)₂(Q₆₀₁), or-P(=O)(Q₆₀₁)(Q₆₀₂),
Q₆₀₁ to Q₆₀₃ may each be the same as described in connection with Q₁,
xe21 may be 1, 2, 3, 4, or 5, and
at least one of Ar₆₀₁, L₆₀₁, and R₆₀₁ may each independently be a π electron-deficient nitrogen-containing C₁-C₆₀ cyclic group unsubstituted or substituted with at least one R₁₀ₐ.

In an embodiment, when xe11 in Formula 601 is 2 or more, two or more of Ar₆₀₁ may be linked together via a single bond.

In one or more embodiments, Ar₆₀₁ in Formula 601 may be a substituted or unsubstituted anthracene group.

In one or more embodiments, the electron transport region may include a compound represented by Formula 601-1: 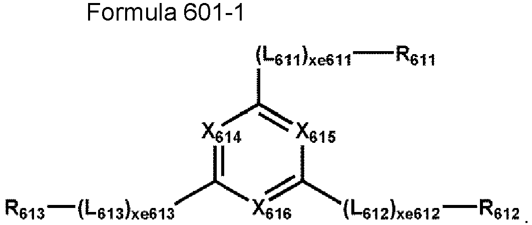 In Formula 601-1,
X₆₁₄ may be N or C(R₆₁₄), X₆₁₅ may be N or C(R₆₁₅), X₆₁₆ may be N or C(R₆₁₆), and at least one of X₆₁₄ to X₆₁₆ may be N,
L₆₁₁ to L₆₁₃ may each be the same as described in connection with L₆₀₁,
xe611 to xe613 may each be the same as described in connection with xe1,
R₆₁₁ to R₆₁₃ may each be the same as described in connection with R₆₀₁, and
R₆₁₄ to R₆₁₆ may each independently be hydrogen, deuterium, -F, -Cl, -Br, -I, a hydroxyl group, a cyano group, a nitro group, a C₁-C₂₀ alkyl group, a C₁-C₂₀ alkoxy group, a C₃-C₆₀ carbocyclic group unsubstituted or substituted with at least one R₁₀ₐ, or a C₁-C₆₀ heterocyclic group unsubstituted or substituted with at least one R₁₀ₐ.

For example, xe1 and xe611 to xe613 in Formulae 601 and 601-1 may each independently be 0, 1, or 2.

In one or more embodiments, the electron transport region may include one of Compounds ET1 to ET45, 2,9-dimethyl-4,7-diphenyl-1,10-phenanthroline (BCP), 4,7-diphenyl-1,10-phenanthroline (Bphen), Alq₃, BAIq, TAZ, NTAZ, or any combination thereof:

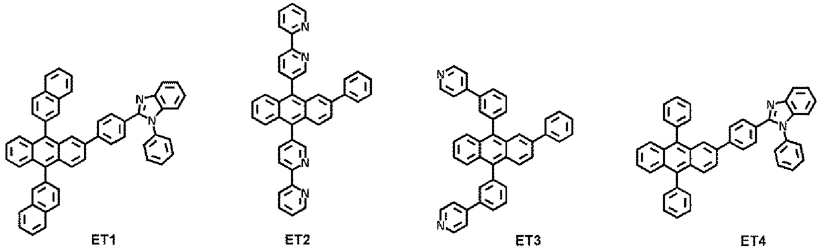

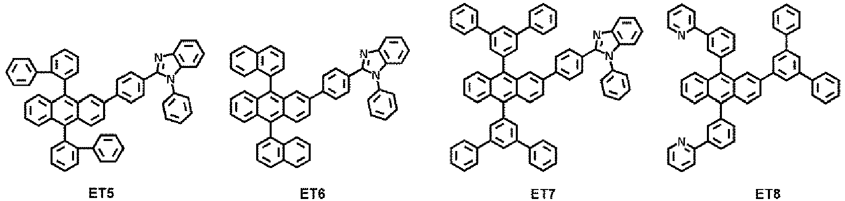

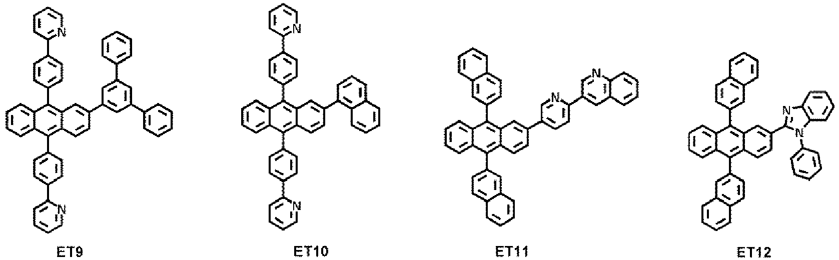

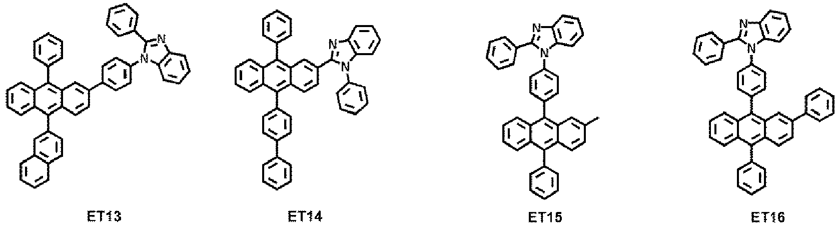

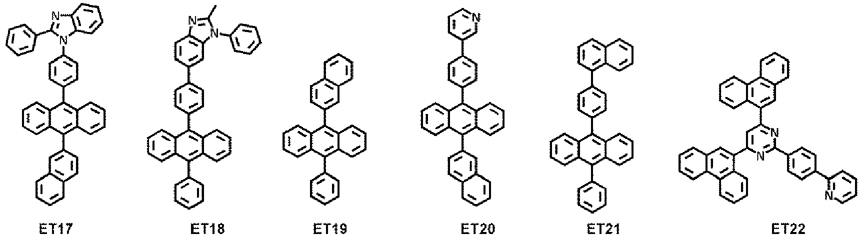

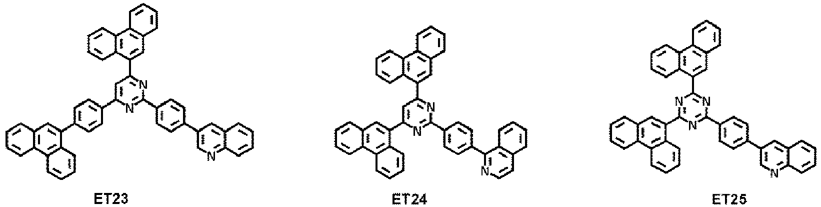

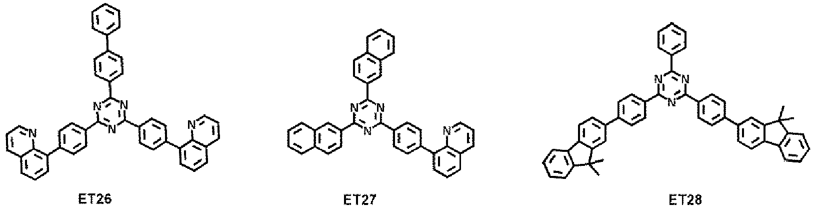

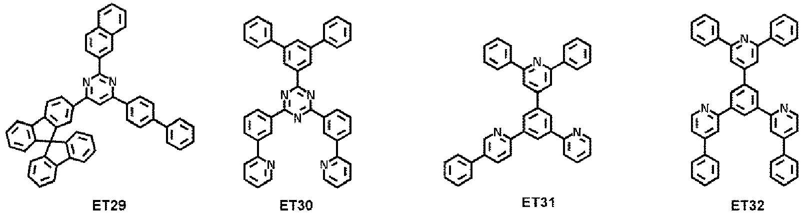

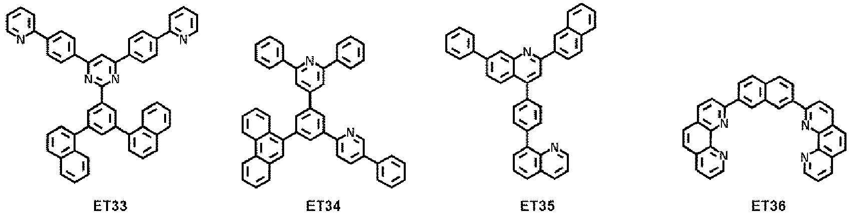

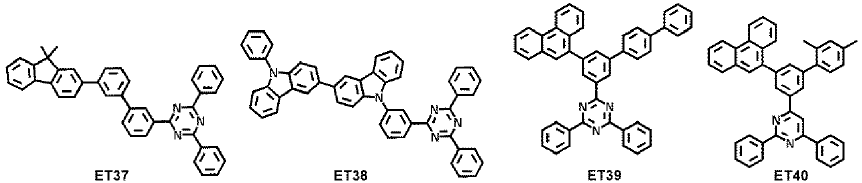

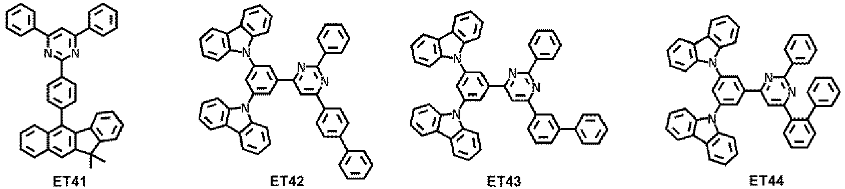

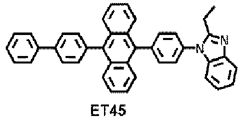

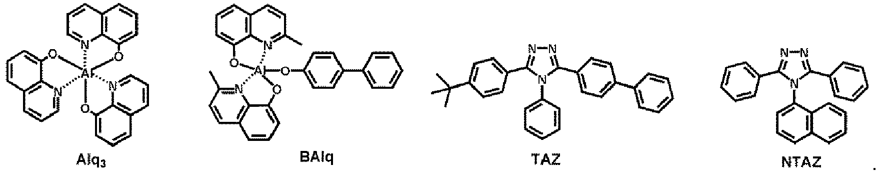

A thickness of the electron transport region may be in a range of about 100 Å to about 5,000 Å, for example, about 160 Å to about 4,000 Å. When the electron transport region includes a buffer layer, a hole blocking layer, an electron control layer, an electron transport layer, or any combination thereof, a thickness of the buffer layer, the hole blocking layer, or the electron control layer may be in a range of about 20 Å to about 1,000 Å, for example, about 30 Å to about 300 Å, and a thickness of the electron transport layer may be un a range of about 100 Å to about 1,000 Å, for example, about 150 Å to about 500 Å. When the thicknesses of the buffer layer, the hole blocking layer, the electron control layer, the electron transport layer, and/or the electron transport layer are within these ranges, satisfactory electron transporting characteristics may be obtained without a substantial increase in driving voltage.

The electron transport region (for example, the electron transport layer in the electron transport region) may further include, in addition to the materials described above, a metal-containing material.

The metal-containing material may include an alkali metal complex, an alkaline earth metal complex, or any combination thereof. The metal ion of an alkali metal complex may be a Li ion, a Na ion, a K ion, a Rb ion, or a Cs ion, and the metal ion of an alkaline earth metal complex may be a Be ion, a Mg ion, a Ca ion, a Sr ion, or a Ba ion. A ligand coordinated with the metal ion of the alkali metal complex or the alkaline earth-metal complex may include a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxyphenyloxadiazole, a hydroxyphenylthiadiazole, a hydroxyphenylpyridine, a hydroxyphenylbenzimidazole, a hydroxyphenylbenzothiazole, a bipyridine, a phenanthroline, a cyclopentadiene, or any combination thereof.

For example, the metal-containing material may include a Li complex. The Li complex may include, for example, Compound ET-D1 (LiQ) or ET-D2:

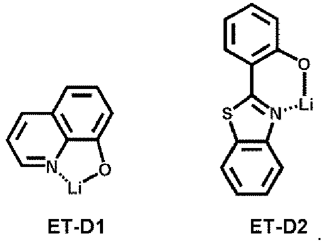

The electron transport region may include an electron injection layer that facilitates the injection of electrons from the second electrode 150. The electron injection layer may directly contact the second electrode 150.

The electron injection layer may have: i) a single-layered structure consisting of a single layer consisting of a single material, ii) a single-layered structure consisting of a single layer consisting of a plurality of different materials, or iii) a multi-layered structure including a plurality of layers including different materials.

The electron injection layer may include an alkali metal, alkaline earth metal, a rare earth metal, an alkali metal-containing compound, alkaline earth metal-containing compound, a rare earth metal-containing compound, an alkali metal complex, an alkaline earth metal complex, a rare earth metal complex, or any combination thereof.

The alkali metal may include Li, Na, K, Rb, Cs, or any combination thereof. The alkaline earth metal may include Mg, Ca, Sr, Ba, or any combination thereof. The rare earth metal may include Sc, Y, Ce, Tb, Yb, Gd, or any combination thereof.

The alkali metal-containing compound, the alkaline earth metal-containing compound, and the rare earth metal-containing compound may be oxides, halides (for example, fluorides, chlorides, bromides, iodides, and the like), or tellurides of the alkali metal, the alkaline earth metal, and the rare earth metal, or any combination thereof.

The alkali metal-containing compound may include alkali metal oxides, such as Li₂O, Cs₂O, K₂O, and the like, alkali metal halides, such as LiF, NaF, CsF, KF, Lil, Nal, Csl, KI, and the like, or any combination thereof. The alkaline earth metal-containing compound may include an alkaline earth metal compound, such as BaO, SrO, CaO, BaₓSr₁₋ₓO (wherein x is a real number satisfying the condition of 0<x<1), BaₓCa₁₋ₓO (wherein x is a real number satisfying the condition of 0<x<1), or the like. The rare earth metal-containing compound may include YbF₃, ScF₃, Sc₂O₃, Y₂O₃, Ce₂O₃, GdF₃, TbF₃, YbI₃, ScI₃, TbI₃, or any combination thereof. For example, the rare earth metal-containing compound may include lanthanide metal telluride. Examples of the lanthanide metal telluride are LaTe, CeTe, PrTe, NdTe, PmTe, SmTe, EuTe, GdTe, TbTe, DyTe, HoTe, ErTe, TmTe, YbTe, LuTe, La₂Te₃, Ce₂Te₃, Pr₂Te₃, Nd₂Te₃, Pm₂Te₃, Sm₂Te₃, Eu₂Te₃, Gd₂Te₃, Tb₂Te₃, Dy₂Te₃, Ho₂Te₃, Er₂Te₃, Tm₂Te₃, Yb₂Te₃, Lu₂Te₃, and the like.

The alkali metal complex, the alkaline earth-metal complex, and the rare earth metal complex may include: i) one of ions of the alkali metal, the alkaline earth metal, and the rare earth metal; and ii), as a ligand linked to the metal ion, for example, hydroxyquinoline, hydroxyisoquinoline, hydroxybenzoquinoline, hydroxyacridine, hydroxyphenanthridine, hydroxyphenyloxazole, hydroxyphenylthiazole, hydroxyphenyloxadiazole, hydroxyphenylthiadiazole, hydroxyphenylpyridine, hydroxyphenyl benzimidazole, hydroxyphenylbenzothiazole, bipyridine, phenanthroline, cyclopentadiene, or any combination thereof.

In an embodiment, the electron injection layer may consist of an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof, as described above. In one or more embodiments, the electron injection layer may further include an organic material (for example, the compound represented by Formula 601).

In one or more embodiments, the electron injection layer may consist of: i) an alkali metal-containing compound (for example, an alkali metal halide); or ii) a) an alkali metal-containing compound (for example, an alkali metal halide), and b) an alkali metal, an alkaline earth metal, a rare earth metal, or any combination thereof. In one or more embodiments, the electron injection layer may be a KI:Yb co-deposited layer, an Rbl:Yb co-deposited layer, and the like.

When the electron injection layer further includes an organic material, alkali metal, alkaline earth metal, rare earth metal, an alkali metal-containing compound, an alkaline earth metal-containing compound, a rare earth metal-containing compound, alkali metal complex, alkaline earth-metal complex, rare earth metal complex, or any combination thereof may be uniformly or non-uniformly dispersed in a matrix including the organic material.

A thickness of the electron injection layer may be in a range of about 1 Å to about 100 Å, for example, about 3 Å to about 90 Å. When the thickness of the electron injection layer is within these ranges, satisfactory electron injection characteristics may be obtained without a substantial increase in driving voltage.

### [Second electrode 150]

The second electrode 150 may be located on the interlayer 130 having a structure as described above. The second electrode 150 may be a cathode, which is an electron injection electrode, and as the material for the second electrode 150, a metal, an alloy, an electrically conductive compound, or any combination thereof, each having a low-work function, may be used.

The second electrode 150 may include lithium (Li), silver (Ag), magnesium (Mg), aluminum (Al), aluminum-lithium (Al-Li), calcium (Ca), magnesium-indium (Mg-In), magnesium-silver (Mg-Ag), ytterbium (Yb), silver-ytterbium (Ag-Yb), ITO, IZO, or any combination thereof. The second electrode 150 may be a transmissive electrode, a semi-transmissive electrode, or a reflective electrode.

The second electrode 150 may have a single-layered structure or a multi-layered structure including a plurality of layers.

### [Capping layer]

A first capping layer may be located outside the first electrode 110, and/or a second capping layer may be located outside the second electrode 150. In particular, the light-emitting device 10 may have a structure in which the first capping layer, the first electrode 110, the interlayer 130, and the second electrode 150 are sequentially stacked in the stated order, a structure in which the first electrode 110, the interlayer 130, the second electrode 150, and the second capping layer are sequentially stacked in the stated order, or a structure in which the first capping layer, the first electrode 110, the interlayer 130, the second electrode 150, and the second capping layer are sequentially stacked in the stated order.

Light generated in the emission layer of the interlayer 130 of the light-emitting device 10 may be extracted toward the outside through the first electrode 110 which is a semi-transmissive electrode or a transmissive electrode, and the first capping layer. Light generated in the emission layer of the interlayer 130 of the light-emitting device 10 may be extracted toward the outside through the second electrode 150 which is a semi-transmissive electrode or a transmissive electrode, and the second capping layer.

The first capping layer and the second capping layer may increase external emission efficiency according to the principle of constructive interference. Accordingly, the light extraction efficiency of the light-emitting device 10 is increased, so that the luminescence efficiency of the light-emitting device 10 may be improved.

Each of the first capping layer and the second capping layer may include a material having a refractive index of 1.6 or more (at 589 nm).

The first capping layer and the second capping layer may each independently be an organic capping layer including an organic material, an inorganic capping layer including an inorganic material, or an organic-inorganic composite capping layer including an organic material and an inorganic material.

In an embodiment, at least one of the first capping layer and the second capping layer may each independently include a carbocyclic compound, a heterocyclic compound, an amine group-containing compound, a porphine derivative, a phthalocyanine derivative, a naphthalocyanine derivative, an alkali metal complex, an alkaline earth metal complex, or any combination thereof. The carbocyclic compound, the heterocyclic compound, and the amine group-containing compound may each optionally be substituted with a substituent including O, N, S, Se, Si, F, Cl, Br, I, or any combination thereof. In one or more embodiments, at least one of the first capping layer and the second capping layer may each independently include an amine group-containing compound.

In one or more embodiments, at least one of the first capping layer and the second capping layer may each independently include a compound represented by Formula 201, a compound represented by Formula 202, or any combination thereof.

In one or more embodiments, at least one of the first capping layer and the second capping layer may each independently include one of Compounds HT28 to HT33, one of Compounds CP1 to CP6, β-NPB, or any combination thereof:

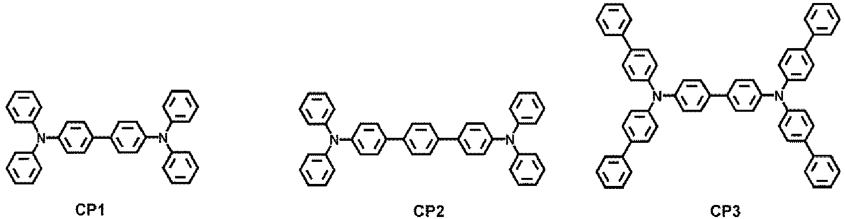

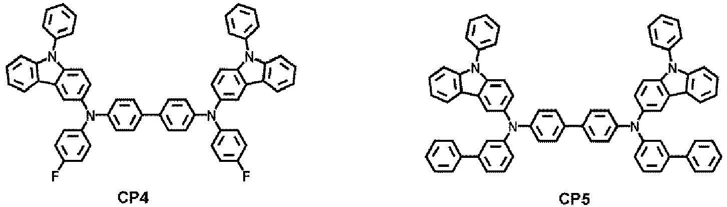

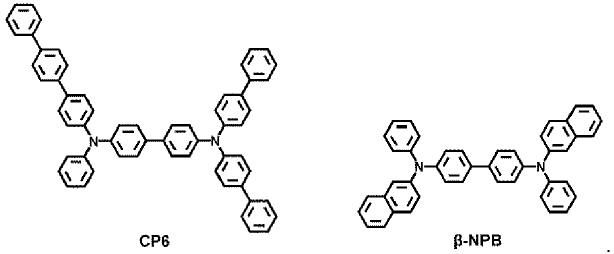

### [Film]

The heterocyclic compound represented by Formula 1 may be included in various films. Accordingly, another aspect of the present disclosure provides a film including the heterocyclic compound represented by Formula 1. The film may be, for example, an optical member (or a light control means) (for example, a color filter, a color conversion member, a capping layer, a light extraction efficiency enhancement layer, a selective light absorbing layer, a polarizing layer, a quantum dot-containing layer, or like), a light-blocking member (for example, a light reflective layer, a light absorbing layer, or the like), a protective member (for example, an insulating layer, a dielectric layer, or the like).

### [Electronic apparatus]

The light-emitting device may be included in various electronic apparatuses. For example, an electronic apparatus including the light-emitting device may be a light-emitting apparatus, an authentication apparatus, or the like.

The electronic apparatus (for example, a light-emitting apparatus) may further include, in addition to the light-emitting device, i) a color filter, ii) a color conversion layer, or iii) a color filter and a color conversion layer. The color filter and/or the color conversion layer may be located in at least one traveling direction of light emitted from the light-emitting device. For example, light emitted from the light-emitting device may be blue light or white light. Details for the light-emitting device may be the same as described herein. In an embodiment, the color conversion layer may include a quantum dot. The quantum dots may be, for example, the same as described herein.

The electronic apparatus may include a first substrate. The first substrate may include a plurality of subpixel areas, the color filter may include a plurality of color filter areas respectively corresponding to the subpixel areas, and the color conversion layer may include a plurality of color conversion areas respectively corresponding to the subpixel areas.

A pixel-defining film may be located among the subpixel areas to define each of the subpixel areas.

The color filter may further include a plurality of color filter areas and light-shielding patterns located among the color filter areas, and the color conversion layer may further include a plurality of color conversion areas and light-shielding patterns located among the color conversion areas.

The plurality of color filter areas (or the plurality of color conversion areas) may include a first area emitting first color light, a second area emitting second color light, and/or a third area emitting third color light, wherein the first color light, the second color light, and/or the third color light may have different maximum emission wavelengths from one another. For example, the first color light may be red light, the second color light may be green light, and the third color light may be blue light. For example, the plurality of color filter areas (or the plurality of color conversion areas) may include quantum dots. In particular, the first region may include red quantum dots, the second region may include green quantum dots, and the third region may not include quantum dots. Details for the quantum dots are the same as described herein. The first region, the second region, and/or the third region may each include a scatter.

For example, the light-emitting device may emit first light, the first region may absorb the first light to emit first-first color light, the second region may absorb the first light to emit second-first color light, and the third region may absorb the first light to emit third-first color light. In this regard, the first-first color light, the second-first color light, and the third-first color light may have different maximum emission wavelengths from each other. In particular, the first light may be blue light, the first-first color light may be red light, the second-first color light may be green light, and the third-first color light may be blue light.

The electronic apparatus may further include a thin-film transistor, in addition to the light-emitting device as described above. The thin-film transistor may include a source electrode, a drain electrode, and an activation layer, wherein any one of the source electrode and the drain electrode may be electrically connected to any one of the first electrode and the second electrode of the light-emitting device.

The thin-film transistor may further include a gate electrode, a gate insulating film, or the like.

The activation layer may include crystalline silicon, amorphous silicon, an organic semiconductor, an oxide semiconductor, or the like.

The electronic apparatus may further include a sealing portion for sealing the light-emitting device. The sealing portion may be located between the color conversion layer and/or color filter and the light-emitting device. The sealing portion allows light from the light-emitting device to be extracted to the outside, and simultaneously prevents ambient air and moisture from penetrating into the light-emitting device. The sealing portion may be a sealing substrate including a transparent glass substrate or a plastic substrate. The sealing portion may be a thin-film encapsulation layer including at least one layer of an organic layer and/or an inorganic layer. When the sealing portion is a thin film encapsulation layer, the electronic apparatus may be flexible.

Various functional layers may be additionally located on the sealing portion, in addition to the color filter and/or the color conversion layer, according to the use of the electronic apparatus. Examples of the functional layers may include a touch screen layer, a polarizing layer, and the like. The touch screen layer may be a pressure-sensitive touch screen layer, a capacitive touch screen layer, or an infrared touch screen layer. The authentication apparatus may be, for example, a biometric authentication apparatus that authenticates an individual by using biometric information of a living body (for example, fingertips, pupils, and the like).

The authentication apparatus may further include, in addition to the light-emitting device as described above, a biometric information collector.

The electronic apparatus may be applied to various displays, light sources, lighting, personal computers (for example, a mobile personal computer), mobile phones, digital cameras, electronic organizers, electronic dictionaries, electronic game machines, medical instruments (for example, electronic thermometers, sphygmomanometers, blood glucose meters, pulse measurement devices, pulse wave measurement devices, electrocardiogram displays, ultrasonic diagnostic devices, or endoscope displays), fish finders, various measuring instruments, meters (for example, meters for a vehicle, an aircraft, and a vessel), projectors, and the like.

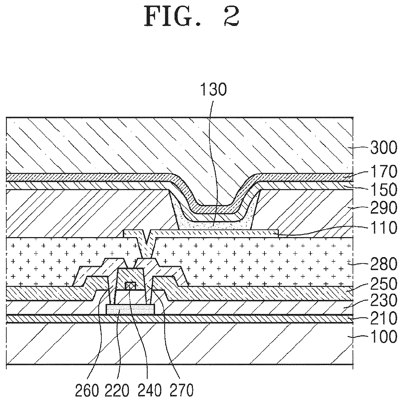

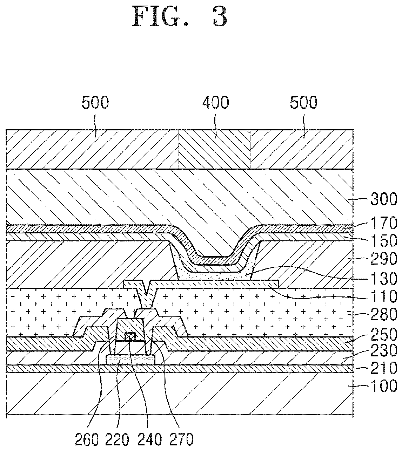

### [Description of FIGS. 2 and 3]

FIG. 2 is a cross-sectional view showing a light-emitting apparatus according to an embodiment of the present disclosure.

The light-emitting apparatus of FIG. 2 includes a substrate 100, a thin-film transistor (TFT), a light-emitting device, and an encapsulation portion 300 that seals the light-emitting device.

The substrate 100 may be a flexible substrate, a glass substrate, or a metal substrate. A buffer layer 210 may be located on the substrate 100. The buffer layer 210 may prevent penetration of impurities through the substrate 100 and may provide a flat surface on the substrate 100.

A TFT may be located on the buffer layer 210. The TFT may include an activation layer 220, a gate electrode 240, a source electrode 260, and a drain electrode 270.

The activation layer 220 may include an inorganic semiconductor such as silicon or polysilicon, an organic semiconductor, or an oxide semiconductor, and may include a source region, a drain region, and a channel region.

A gate insulating film 230 for insulating the activation layer 220 from the gate electrode 240 may be located on the activation layer 220, and the gate electrode 240 may be located on the gate insulating film 230.

An interlayer insulating film 250 may be located on the gate electrode 240. The interlayer insulating film 250 may be located between the gate electrode 240 and the source electrode 260 and between the gate electrode 240 and the drain electrode 270, to insulate from one another.

The source electrode 260 and the drain electrode 270 may be located on the interlayer insulating film 250. The interlayer insulating film 250 and the gate insulating film 230 may be formed to expose the source region and the drain region of the activation layer 220, and the source electrode 260 and the drain electrode 270 may be located in contact with the exposed portions of the source region and the drain region of the activation layer 220.

The TFT is electrically connected to a light-emitting device to drive the light-emitting device, and is covered and protected by a passivation layer 280. The passivation layer 280 may include an inorganic insulating film, an organic insulating film, or any combination thereof. A light-emitting device is provided on the passivation layer 280. The light-emitting device may include a first electrode 110, an interlayer 130, and a second electrode 150.

The first electrode 110 may be located on the passivation layer 280. The passivation layer 280 may be located to expose a portion of the drain electrode 270, not fully covering the drain electrode 270, and the first electrode 110 may be located to be connected to the exposed portion of the drain electrode 270.

A pixel defining layer 290 including an insulating material may be located on the first electrode 110. The pixel defining layer 290 may expose a certain region of the first electrode 110, and an interlayer 130 may be formed in the exposed region of the first electrode 110. The pixel defining layer 290 may be a polyimide or polyacrylic organic film. Although not shown in FIG. 2, at least some layers of the interlayer 130 may extend beyond the upper portion of the pixel defining layer 290 to be located in the form of a common layer.

The second electrode 150 may be located on the interlayer 130, and a capping layer 170 may be additionally formed on the second electrode 150. The capping layer 170 may be formed to cover the second electrode 150.

The encapsulation portion 300 may be located on the capping layer 170. The encapsulation portion 300 may be located on a light-emitting device to protect the light-emitting device from moisture or oxygen. The encapsulation portion 300 may include: an inorganic film including silicon nitride (SiNx), silicon oxide (SiOx), indium tin oxide, indium zinc oxide, or any combination thereof; an organic film including polyethylene terephthalate, polyethylene naphthalate, polycarbonate, polyimide, polyethylene sulfonate, polyoxymethylene, polyarylate, hexamethyldisiloxane, an acrylic resin (for example, polymethyl methacrylate, polyacrylic acid, and the like), an epoxy-based resin (for example, aliphatic glycidyl ether (AGE), and the like), or any combination thereof; or any combination of the inorganic films and the organic films.

FIG. 3 shows a cross-sectional view showing a light-emitting apparatus according to an embodiment of the present disclosure.

The light-emitting apparatus of FIG. 3 is the same as the light-emitting apparatus of FIG. 2, except that a light-shielding pattern 500 and a functional region 400 are additionally located on the encapsulation portion 300. The functional region 400 may be i) a color filter area, ii) a color conversion area, or iii) a combination of the color filter area and the color conversion area. In an embodiment, the light-emitting device included in the light-emitting apparatus of FIG. 3 may be a tandem light-emitting device.

### [Manufacturing method]

The layers included in the hole transport region, the emission layer, and the layers included in the electron transport region may be formed in a certain region by using various methods such as vacuum deposition, spin coating, casting, Langmuir-Blodgett (LB) deposition, ink-jet printing, laser-printing, laser-induced thermal imaging, and the like.

When layers constituting the hole transport region, an emission layer, and layers constituting the electron transport region are formed by vacuum deposition, the deposition may be performed at a deposition temperature of about 100 °C to about 500 °C, a vacuum degree of about 10⁻⁸ torr to about 10⁻³ torr, and a deposition speed of about 0.01 Å/sec to about 100 Å/sec, depending on a material to be included in a layer to be formed and the structure of a layer to be formed.

### [Definition of terms]

The term "C₃-C₆₀ carbocyclic group" as used herein refers to a cyclic group consisting of carbon only as a ring-forming atom and having three to sixty carbon atoms, and the term "C₁-C₆₀ heterocyclic group" as used herein refers to a cyclic group that has one to sixty carbon atoms and further has, in addition to carbon, a heteroatom as a ring-forming atom. The C₃-C₆₀ carbocyclic group and the C₁-C₆₀ heterocyclic group may each be a monocyclic group consisting of one ring or a polycyclic group in which two or more rings are condensed with each other. For example, the C₁-C₆₀ heterocyclic group has 3 to 61 ring-forming atoms.

The "cyclic group" as used herein may include the C₃-C₆₀ carbocyclic group, and the C₁-C₆₀ heterocyclic group.

The term "π electron-rich C₃-C₆₀ cyclic group" as used herein refers to a cyclic group that has three to sixty carbon atoms and does not include *-N=*' as a ring-forming moiety, and the term "π electron-deficient nitrogen-containing C₁-C₆₀ cyclic group" as used herein refers to a heterocyclic group that has one to sixty carbon atoms and includes *-N=*' as a ring-forming moiety.

For example,
the C₃-C₆₀ carbocyclic group may be i) a T1 group or ii) a condensed cyclic group in which two or more T1 groups are condensed with each other (for example, a cyclopentadiene group, an adamantane group, a norbornane group, a benzene group, a pentalene group, a naphthalene group, an azulene group, an indacene group, an acenaphthylene group, a phenalene group, a phenanthrene group, an anthracene group, a fluoranthene group, a triphenylene group, a pyrene group, a chrysene group, a perylene group, a pentaphene group, a heptalene group, a naphthacene group, a picene group, a hexacene group, a pentacene group, a rubicene group, a coronene group, an ovalene group, an indene group, a fluorene group, a spiro-bifluorene group, a benzofluorene group, an indenophenanthrene group, or an indenoanthracene group),
the C₁-C₆₀ heterocyclic group may be i) a T2 group, ii) a condensed cyclic group in which two or more T2 groups are condensed with each other, or iii) a condensed cyclic group in which at least one T2 group and at least one T1 group are condensed with each other (for example, a pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, and the like),
the π electron-rich C₃-C₆₀ cyclic group may be i) a T1 group, ii) a condensed cyclic group in which two or more T1 groups are condensed with each other, iii) a T3 group, iv) a condensed cyclic group in which two or more T3 groups are condensed with each other, or v) a condensed cyclic group in which at least one T3 group and at least one T1 group are condensed with each other (for example, the C₃-C₆₀ carbocyclic group, a 1H-pyrrole group, a silole group, a borole group, a 2H-pyrrole group, a 3H-pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, and the like),
the π electron-deficient nitrogen-containing C₁-C₆₀ cyclic group may be i) a T4 group, ii) a condensed cyclic group in which two or more T4 groups are condensed with each other, iii) a condensed cyclic group in which at least one T4 group and at least one T1 group are condensed with each other, iv) a condensed cyclic group in which at least one T4 group and at least one T3 group are condensed with each other, or v) a condensed cyclic group in which at least one T4 group, at least one T1 group, and at least one T3 group are condensed with one another (for example, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, and the like),
the T1 group may be a cyclopropane group, a cyclobutane group, a cyclopentane group, a cyclohexane group, a cycloheptane group, a cyclooctane group, a cyclobutene group, a cyclopentene group, a cyclopentadiene group, a cyclohexene group, a cyclohexadiene group, a cycloheptene group, an adamantane group, a norbornane (or a bicyclo[2.2.1]heptane) group, a norbornene group, a bicyclo[1.1.1]pentane group, a bicyclo[2.1.1]hexane group, a bicyclo[2.2.2]octane group, or a benzene group,
the T2 group may be a furan group, a thiophene group, a 1H-pyrrole group, a silole group, a borole group, a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, a tetrazine group, a pyrrolidine group, an imidazolidine group, a dihydropyrrole group, a piperidine group, a tetrahydropyridine group, a dihydropyridine group, a hexahydropyrimidine group, a tetrahydropyrimidine group, a dihydropyrimidine group, a piperazine group, a tetrahydropyrazine group, a dihydropyrazine group, a tetrahydropyridazine group, or a dihydropyridazine group,
the T3 group may be a furan group, a thiophene group, a 1H-pyrrole group, a silole group, or a borole group, and
the T4 group may be a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, or a tetrazine group.

The terms "the cyclic group, the C₃-C₆₀ carbocyclic group, the C₁-C₆₀ heterocyclic group, the π electron-rich C₃-C₆₀ cyclic group, or the π electron-deficient nitrogen-containing C₁-C₆₀ cyclic group" as used herein refer to a group condensed to any cyclic group, a monovalent group, or a polyvalent group (for example, a divalent group, a trivalent group, a tetravalent group, and the like) according to the structure of a formula for which the corresponding term is used. For example, the "benzene group" may be a benzo group, a phenyl group, a phenylene group, or the like, which may be easily understood by one of ordinary skill in the art according to the structure of a formula including the "benzene group."

Examples of the monovalent C₃-C₆₀ carbocyclic group and the monovalent C₁-C₆₀ heterocyclic group are a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group. Examples of the divalent C₃-C₆₀ carbocyclic group and the monovalent C₁-C₆₀ heterocyclic group are a C₃-C₁₀ cycloalkylene group, a C₁-C₁₀ heterocycloalkylene group, a C₃-C₁₀ cycloalkenylene group, a C₁-C₁₀ heterocycloalkenylene group, a C₆-C₆₀ arylene group, a C₁-C₆₀ heteroarylene group, a divalent non-aromatic condensed polycyclic group, and a substituted or unsubstituted divalent non-aromatic condensed heteropolycyclic group.

The term "C₁-C₆₀ alkyl group" as used herein refers to a linear or branched aliphatic hydrocarbon monovalent group that has one to sixty carbon atoms, and specific examples thereof are a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, a tert-decyl group, and the like. The term "C₁-C₆₀ alkylene group" as used herein refers to a divalent group having the same structure as the C₁-C₆₀ alkyl group.

The term "C₂-C₆₀ alkenyl group" as used herein refers to a monovalent hydrocarbon group having at least one carbon-carbon double bond in the middle or at the terminus of the C₂-C₆₀ alkyl group, and examples thereof are an ethenyl group, a propenyl group, a butenyl group, and the like. The term "C₂-C₆₀ alkenylene group" as used herein refers to a divalent group having the same structure as the C₂-C₆₀ alkenyl group.

The term "C₂-C₆₀ alkynyl group" as used herein refers to a monovalent hydrocarbon group having at least one carbon-carbon triple bond in the middle or at the terminus of the C₂-C₆₀ alkyl group, and examples thereof are an ethynyl group, a propynyl group, and the like. The term "C₂-C₆₀ alkynylene group" as used herein refers to a divalent group having the same structure as the C₂-C₆₀ alkynyl group.

The term "C₁-C₆₀ alkoxy group" as used herein refers to a monovalent group represented by -OA₁₀₁ (wherein A₁₀₁ is the C₁-C₆₀ alkyl group), and examples thereof are a methoxy group, an ethoxy group, an isopropyloxy group, and the like.

The term "C₃-C₁₀ cycloalkyl group" as used herein refers to a monovalent saturated hydrocarbon cyclic group having 3 to 10 carbon atoms, and examples thereof are a cyclopropyl group, a cyclobutyl group, a cyclopentyl group, a cyclohexyl group, a cycloheptyl group, a cyclooctyl group, an adamantanyl group, a norbornanyl group (or bicyclo[2.2.1]heptyl group), a bicyclo[1.1.1]pentyl group, a bicyclo[2.1.1]hexyl group, a bicyclo[2.2.2]octyl group, and the like. The term "C₃-C₁₀ cycloalkylene group" as used herein refers to a divalent group having the same structure as the C₃-C₁₀ cycloalkyl group.

The term "C₁-C₁₀ heterocycloalkyl group" as used herein refers to a monovalent cyclic group of 1 to 10 carbon atoms, further including, in addition to carbon atoms, at least one heteroatom, as ring-forming atoms, and specific examples are a 1,2,3,4-oxatriazolidinyl group, a tetrahydrofuranyl group, a tetrahydrothiophenyl group, and the like. The term "C₁-C₁₀ heterocycloalkylene group" as used herein refers to a divalent group having the same structure as the C₁-C₁₀ heterocycloalkyl group.

The term C₃-C₁₀ cycloalkenyl group used herein refers to a monovalent cyclic group that has three to ten carbon atoms and at least one carbon-carbon double bond in the ring thereof and no aromaticity, and specific examples thereof are a cyclopentenyl group, a cyclohexenyl group, a cycloheptenyl group, and the like. The term "C₃-C₁₀ cycloalkenylene group" as used herein refers to a divalent group having the same structure as the C₃-C₁₀ cycloalkenyl group.

The term "C₁-C₁₀ heterocycloalkenyl group" as used herein refers to a monovalent cyclic group of 1 to 10 carbon atoms, further including, in addition to carbon atoms, at least one heteroatom, as ring-forming atoms, and having at least one carbon-carbon double bond in the cyclic structure thereof. Examples of the C₁-C₁₀ heterocycloalkenyl group are a 4,5-dihydro-1,2,3,4-oxatriazolyl group, a 2,3-dihydrofuranyl group, a 2,3-dihydrothiophenyl group, and the like. The term "C₁-C₁₀ heterocycloalkenylene group" as used herein refers to a divalent group having the same structure as the C₁-C₁₀ heterocycloalkenyl group.

The term "C₆-C₆₀ aryl group" as used herein refers to a monovalent group having a carbocyclic aromatic system of 6 to 60 carbon atoms, and the term "C₆-C₆₀ arylene group" as used herein refers to a divalent group having a carbocyclic aromatic system of 6 to 60 carbon atoms. Examples of the C₆-C₆₀ aryl group are a phenyl group, a pentalenyl group, a naphthyl group, an azulenyl group, an indacenyl group, an acenaphthyl group, a phenalenyl group, a phenanthrenyl group, an anthracenyl group, a fluoranthenyl group, a triphenylenyl group, a pyrenyl group, a chrysenyl group, a perylenyl group, a pentaphenyl group, a heptalenyl group, a naphthacenyl group, a picenyl group, a hexacenyl group, a pentacenyl group, a rubicenyl group, a coronenyl group, an ovalenyl group, and the like. When the C₆-C₆₀ aryl group and the C₆-C₆₀ arylene group each include two or more rings, the two or more rings may be condensed with each other.

The term "C₁-C₆₀ heteroaryl group" as used herein refers to a monovalent group having a heterocyclic aromatic system of 1 to 60 carbon atoms, further including, in addition to carbon atoms, at least one heteroatom, as ring-forming atoms. The term "C₁-C₆₀ heteroarylene group" as used herein refers to a divalent group having a heterocyclic aromatic system of 1 to 60 carbon atoms, further including, in addition to carbon atoms, at least one heteroatom, as ring-forming atoms. Examples of the C₁-C₆₀ heteroaryl group are a pyridinyl group, a pyrimidinyl group, a pyrazinyl group, a pyridazinyl group, a triazinyl group, a quinolinyl group, a benzoquinolinyl group, an isoquinolinyl group, a benzoisoquinolinyl group, a quinoxalinyl group, a benzoquinoxalinyl group, a quinazolinyl group, a benzoquinazolinyl group, a cinnolinyl group, a phenanthrolinyl group, a phthalazinyl group, a naphthyridinyl group, and the like. When the C₁-C₆₀ heteroaryl group and the C₁-C₆₀ heteroarylene group each include two or more rings, the rings may be condensed with each other.

The term "monovalent non-aromatic condensed polycyclic group" as used herein refers to a monovalent group (for example, having 8 to 60 carbon atoms) having two or more rings condensed to each other, only carbon atoms as ring-forming atoms, and no aromaticity in its entire molecular structure. Examples of the monovalent non-aromatic condensed polycyclic group are an indenyl group, a fluorenyl group, a spiro-bifluorenyl group, a benzofluorenyl group, an indenophenanthrenyl group, and an indeno anthracenyl group. The term "divalent non-aromatic condensed polycyclic group" as used herein refers to a divalent group having the same structure as the monovalent non-aromatic condensed polycyclic group described above.

The term "monovalent non-aromatic condensed heteropolycyclic group" as used herein refers to a monovalent group (for example, having 1 to 60 carbon atoms) having two or more rings condensed to each other, further including, in addition to carbon atoms, at least one heteroatom, as ring-forming atoms, and having non-aromaticity in its entire molecular structure. Examples of the monovalent non-aromatic condensed heteropolycyclic group are a pyrrolyl group, a thiophenyl group, a furanyl group, an indolyl group, a benzoindolyl group, a naphthoindolyl group, an isoindolyl group, a benzoisoindolyl group, a naphthoisoindolyl group, a benzosilolyl group, a benzothiophenyl group, a benzofuranyl group, a carbazolyl group, a dibenzosilolyl group, a dibenzothiophenyl group, a dibenzofuranyl group, an azacarbazolyl group, an azafluorenyl group, an azadibenzosilolyl group, an azadibenzothiophenyl group, an azadibenzofuranyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a benzopyrazolyl group, a benzimidazolyl group, a benzoxazolyl group, a benzothiazolyl group, a benzoxadiazolyl group, a benzothiadiazolyl group, an imidazopyridinyl group, an imidazopyrimidinyl group, an imidazotriazinyl group, an imidazopyrazinyl group, an imidazopyridazinyl group, an indenocarbazolyl group, an indolocarbazolyl group, a benzofurocarbazolyl group, a benzothienocarbazolyl group, a benzosilolocarbazolyl group, a benzoindolocarbazolyl group, a benzocarbazolyl group, a benzonaphthofuranyl group, a benzonaphthothiophenyl group, a benzonaphthosilolyl group, a benzofurodibenzofuranyl group, a benzofurodibenzothiophenyl group, a benzothienodibenzothiophenyl group, and the like. The term "divalent non-aromatic condensed heteropolycyclic group" as used herein refers to a divalent group having the same structure as the monovalent non-aromatic condensed heteropolycyclic group described above.

The term "C₆-C₆₀ aryloxy group" as used herein indicates -OA₁₀₂ (wherein A₁₀₂ is the C₆-C₆₀ aryl group), and the term "C₆-C₆₀ arylthio group" as used herein indicates - SA₁₀₃ (wherein A₁₀₃ is the C₆-C₆₀ aryl group).

The term "C₇-C₆₀ aryl alkyl group" used herein refers to -A₁₀₄A₁₀₅ (where A₁₀₄ may be a C₁-C₅₄ alkylene group, and A₁₀₅ may be a C₆-C₅₉ aryl group), and the term C₂-C₆₀ heteroaryl alkyl group" used herein refers to -A₁₀₆A₁₀₇ (where A₁₀₆ may be a C₁-C₅₉ alkylene group, and A₁₀₇ may be a C₁-C₅₉ heteroaryl group).

The term "R₁₀ₐ" as used herein may be:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₁₁)(Q₁₂)(Q₁₃), -N(Q₁₁)(Q₁₂), -B(Q₁₁)(Q₁₂), -C(=O)(Q₁₁), - S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, or a C₂-C₆₀ heteroaryl alkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), - P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q31)(Q32).

In the present specification, Q₁ to Q₃, Q₁₁ to Q₁₃, Q₂₁ to Q₂₃, and Q₃₁ to Q₃₃ may each independently be: hydrogen; deuterium; -F; -Cl; -Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof; a C₇-C₆₀ aryl alkyl group; or a C₂-C₆₀ heteroaryl alkyl group.

The term "heteroatom" as used herein refers to any atom other than a carbon atom. Examples of the heteroatom are O, S, N, P, Si, B, Ge, Se, or any combinations thereof.

The term "third-row transition metal" as used herein includes hafnium (Hf), tantalum (Ta), tungsten (W), rhenium (Re), osmium (Os), iridium (Ir), platinum (Pt), gold (Au), and the like.

"Ph" as used herein refers to a phenyl group, "Me" as used herein refers to a methyl group, "Et" as used herein refers to an ethyl group, "ter-Bu" or "Bu^{t}" as used herein refers to a tert-butyl group, and "OMe" as used herein refers to a methoxy group.

The term "biphenyl group" as used herein refers to "a phenyl group substituted with a phenyl group". In other words, the "biphenyl group" is a substituted phenyl group having a C₆-C₆₀ aryl group as a substituent.

The term "terphenyl group" as used herein refers to "a phenyl group substituted with a biphenyl group". In other words, the "terphenyl group" is a substituted phenyl group having, as a substituent, a C₆-C₆₀ aryl group substituted with a C₆-C₆₀ aryl group.

* and *' as used herein, unless defined otherwise, each refer to a binding site to a neighboring atom in a corresponding formula or moiety.

Hereinafter, compounds according to embodiments and light-emitting devices according to embodiments will be described in detail with reference to the following synthesis examples and examples. The wording "B was used instead of A" used in describing Synthesis Examples means that an identical molar equivalent of B was used in place of A.

### [Examples]

### Synthesis Example 1: Synthesis of Compound 2

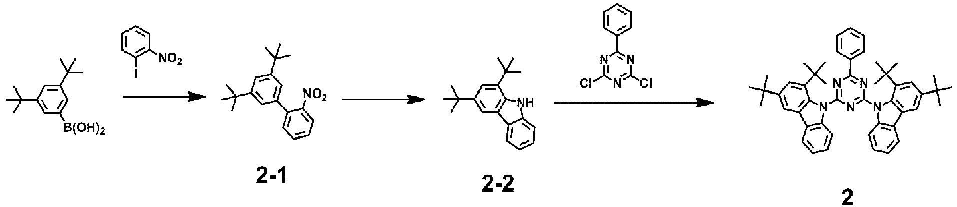

### Synthesis of Intermediate 2-1

(3,5-di-tert-butylphenyl)boronic acid (CAS#= 197223-39-5, 1.2 eq) and 1-iodo-2-nitrobenzene were reacted in the presence of Pd(PPh₃)₄ catalyst, so as to obtain Intermediate 2-1. Intermediate 2-1 was identified by LC/MS. C₂₀H₂₅NO₂ M +1 : 312.21

### Synthesis of Intermediate 2-2

Intermediate 2-1 was dissolved in a solution (0.2 M) in which o-dichlorobenzene and P(OEt)₃ were mixed at a volume ratio of 1:1 for a reaction at 180 °C, so as to obtain Intermediate 2-2. Intermediate 2-2 was identified by LC/MS. C₂₀H₂₅N M+1 : 280.21

### Synthesis of Compound 2

2.9 g of Intermediate 2-2 was added to RBF, and then, 30 mL of THF was added dropwise thereto under nitrogen conditions. nBuLi (2.0 M, 5.2 mL) was added to the reaction solution at 0 °C , and stirred for 30 minutes. To the resultant reaction solution, a mixed solution in which 2.3 g of 2,4-dichloro-6-phenyl-1,3,5-triazine was dissolved in 30 mL of THF was slowly added dropwise. The reaction solution was stirred overnight at 70 °C. After the reaction was completed, an extraction process was performed on the reaction solution by using ethylacetate. An organic layer collected therefrom was dried with magnesium sulfate, and a solvent was evaporated therefrom. A residue thus obtained was then separated and purified by silica gel column chromatography, so as to obtain 4.9 g (yield: 67 %) of Compound 2. Compound 2 was identified by LC-MS and ¹H-NMR.

### Synthesis Example 2: Synthesis of Compound 4

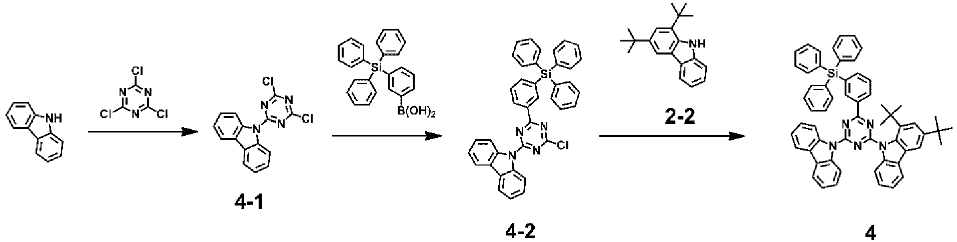

### Synthesis of Intermediate 4-1

Carbazole and nBuLi were reacted, and cyanuric chloride was added thereto and stirred overnight at 70 °C, so as to obtain Intermediate 4-1. Intermediate 4-1 was identified by LC/MS. C₁₅H₈Cl₂N₄ M+1: 315.07

### Synthesis of Intermediate 4-2

Intermediate 4-1 and (3-(triphenylsilyl)phenyl)boronic acid (CAS#= 1253912-58-1) were reacted in the presence of Pd(PPh₃)₂Cl₂ (0.01 eq) catalyst, so as to obtain Intermediate 4-2. Intermediate 4-2 was identified by LC/MS.

C₃₉H₂₇ClN₄Si M+1: 615.11

### Synthesis of Compound 4

3.4 g of Intermediate 4-2, 1.54 g of Intermediate 2-2, 0.2 g of Pd₂(dba)₃, 0.2 mL of P(tBu)₃ (50 wt% in xylene), 1.6 g of NaOtBu, and 50 mL of o-xylene were added to RBF, and stirred overnight at 160 °C. After the reaction was completed, an extraction process was performed on the reaction solution by using ethylacetate. An organic layer collected therefrom was dried with magnesium sulfate, and a solvent was evaporated therefrom. A residue thus obtained was then separated and purified by silica gel column chromatography, so as to obtain 3.4 g (yield: 72 %) of Compound 4. Compound 4 was identified by LC-MS and ¹H-NMR.

### Synthesis Example 3: Synthesis of Compound 5

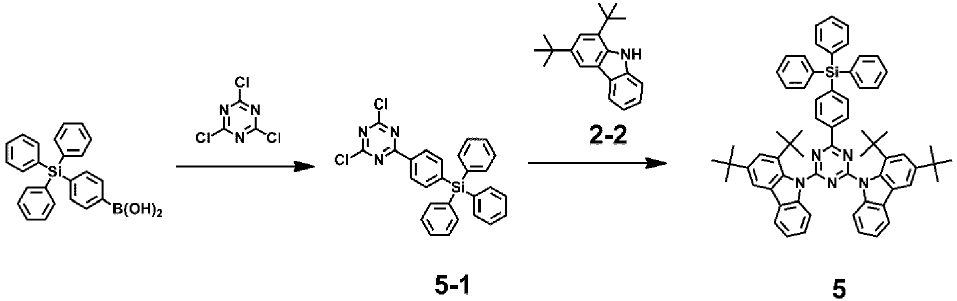

### Synthesis of Intermediate 5-1

Cyanuric chloride (1 eq) and (4-(triphenylsilyl)phenyl)boronic acid (CAS#=852475-03-7, 1 eq) were reacted in the presence of Pd(PPh₃)₂Cl₂ (0.01 eq), so as to obtain Intermediate 5-1. Intermediate 5-1 was identified by LC/MS. C₂₇H₁₉Cl₂N₃Si M+1: 484.01

### Synthesis of Compound 5

5.2 g of Intermediate 5-1, 3.0 g of Intermediate 2-2, 0.39 g of Pd₂(dba)₃, 0.2 mL of P(tBu)₃, 3 g of NaOtBu, and 50 mL of o-xylene were added to RBF, and stirred overnight at 160 °C. After the reaction was completed, an extraction process was performed on the reaction solution by using ethylacetate. An organic layer collected therefrom was dried with magnesium sulfate, and a solvent was evaporated therefrom. A residue thus obtained was then separated and purified by silica gel column chromatography, so as to obtain 5.7 g (yield: 55 %) of Compound 5. Compound 5 was identified by LC-MS and ¹H-NMR.

### Synthesis Example 4: Synthesis of Compound 23

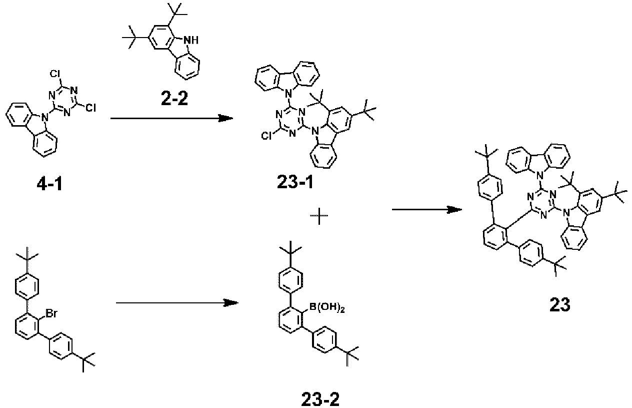

### Synthesis of Intermediate 23-1

Intermediate 4-1 and Intermediate 2-2 were reacted, so as to synthesize Intermediate 23-1. Intermediate 23-1 was identified by LC/MS. C₃₅H₃₂ClN₅ M + 1 : 558.21

### Synthesis of Intermediate 23-2

2'-bromo-4,4"-di-tert-butyl-1,1':3',1"-terphenyl (CAS#= 332104-80-0) and nBuLi were reacted, and B(OMe)₃ was added dropwise thereto. Then, the mixed solution was stirred overnight, so as to obtain Intermediate 23-2. Intermediate 23-2 was identified by LC/MS. C₂₆H₃₁BO₂ M + 1 : 387.19

### Synthesis of Compound 23

2.6 g of Intermediate 23-1, 2.2 g of Intermediate 23-2, 0.21 g of tetrakis(triphenylphosphine)palladium, and 1.6 g of potassium carbonate were added to a reaction vessel, and dissolved in 40 mL of toluene, 10 mL of ethanol, and 10 mL of distilled water. The mixed solution was then refluxed for 24 hours. After the reaction was completed, an extraction process was performed on the reaction solution by using ethylacetate. An organic layer collected therefrom was dried with magnesium sulfate, and a solvent was evaporated therefrom. A residue thus obtained was then separated and purified by silica gel column chromatography, so as to obtain 2.5 g (yield: 61 %) of Compound 23. Compound 23 was identified by LC-MS and ¹H-NMR.

### Synthesis Example 5: Synthesis of Compound 28

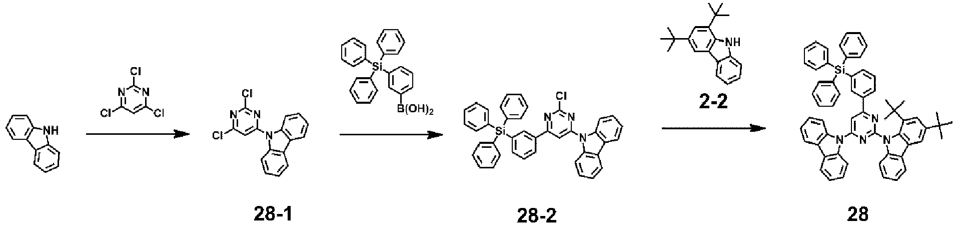

### Synthesis of Intermediate 28-1

Carbazole (1 eq) and n-BuLi (1 eq) were reacted, and then, trichloripyrimidine (1 eq) was also reacted therewith, so as to obtain Intermediate 28-1. Intermediate 28-1 was identified by LC/MS. C₁₆H₉Cl₂N₃ M + 1 : 314.07

### Synthesis of Intermediate 28-2

Intermediate 2-2 and Intermediate 28-1 were reacted in the presence of Pd(PPh₃)₂Cl₂ catalyst, so as to obtain Intermediate 28-2. Intermediate 28-2 was identified by LC/MS.

C40H28CIN3Si M + 1 : 614.11

### Synthesis of Compound 28

1.9 g of Intermediate 28-2, 0.9 g of Intermediate 2-2, 0.11 g of Pd₂(dba)₃, 0.05 mL of P(tBu)₃, 0.9 g of NaOtBu, and 30 mL of o-xylene were added to RBF, and stirred overnight at 160 °C. After the reaction was completed, an extraction process was performed on the reaction solution by using ethylacetate. An organic layer collected therefrom was dried with magnesium sulfate, and a solvent was evaporated therefrom. A residue thus obtained was then separated and purified by silica gel column chromatography, so as to obtain 1.2 g (yield: 44 %) of Compound 28. Compound 28 was identified by LC-MS and ¹H-NMR.

### Synthesis Example 6: Synthesis of Compound 30

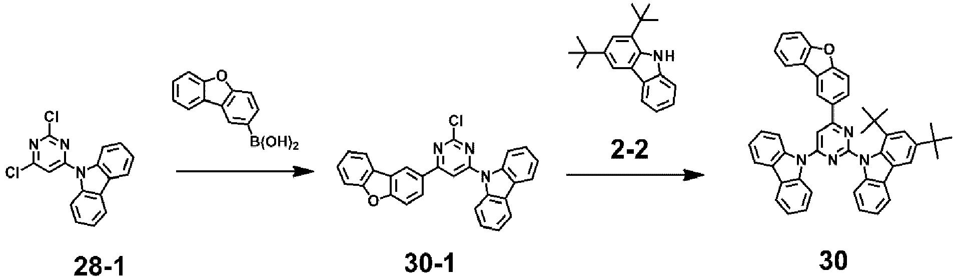

### Synthesis of Intermediate 30-1

Intermediate 28-1 and dibenzo[b,d]furan-2-ylboronic acid (CAS# = 402936-15-6) were reacted in the presence of Pd(PPh₃)₂Cl₂ catalyst, so as to obtain Intermediate 30-1. Intermediate 30-1 was identified by LC/MS. C₂₈H₁₆ClN₃O M + 1 : 446.13

### Synthesis of Compound 30

4.5 g of Intermediate 30-1, 2.8 g of Intermediate 2-2, 0.37 g of Pd₂(dba)₃, 0.2 mL of P(tBu)₃, 2.9 g of NaOtBu, and 50 mL of o-xylene were added to RBF, and stirred overnight at 160 °C. After the reaction was completed, an extraction process was performed on the reaction solution by using ethylacetate. An organic layer collected therefrom was dried with magnesium sulfate, and a solvent was evaporated therefrom. A residue thus obtained was then separated and purified by silica gel column chromatography, so as to obtain 1.8 g (yield: 86 %) of Compound 30. Compound 30 was identified by LC-MS and ¹H-NMR.

### Synthesis Example 7: Synthesis of Compound 41

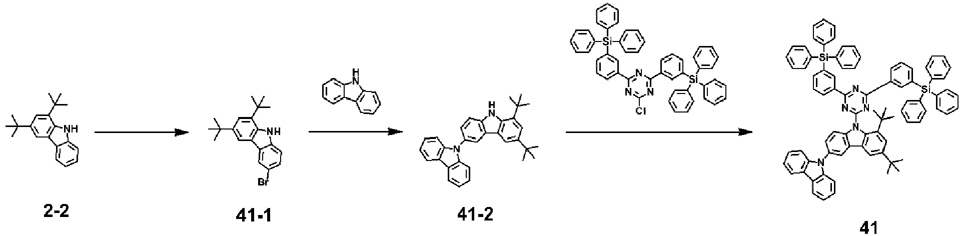

### Synthesis of Intermediate 41-1

Intermediate 2-2 and N-bromosuccinimide were reacted in the presence of DMF solvent, so as to obtain Intermediate 41-1. Intermediate 41-1 was identified by LC/MS. C₂₀H₂₄BrN M+1 : 358.12

### Synthesis of Intermediate 41-2

Intermediate 41-1 and carbazole were reacted in the presence of Pd₂dba₃ catalyst, so as to obtain Intermediate 41-2. Intermediate 41-2 was identified by LC/MS. C₃₂H₃₂N₂ M+1 : 334.23

### Synthesis of Compound 41

1.8 g of Intermediate 41-2, 3.9 g of 2-chloro-4,6-bis(3-(triphenylsilyl)phenyl)-1,3,5-triazine (CAS#= 2422046-28-2), 0.15 g of Pd₂(dba)₃, 0.1 mL of P(tBu)₃, 0.1 g of NaOtBu, and 30 mL of o-xylene were added to RBF, and stirred overnight at 160 °C. After the reaction was completed, an extraction process was performed on the reaction solution by using ethylacetate. An organic layer collected therefrom was dried with magnesium sulfate, and a solvent was evaporated therefrom. A residue thus obtained was then separated and purified by silica gel column chromatography, so as to obtain 1.33 g (yield: 49 %) of Compound 41. Compound 41 was identified by LC-MS and ¹H-NMR.

Table 1 shows ¹H NMR and LC-MS of the synthesized compounds. Synthesis methods for other compounds than the compounds shown in Table 1 may be easily recognized by those skilled in the technical field by referring to the synthesis paths and source materials described above.

**Table 1**

| Compo und | ¹H-NMR (CDCl₃ , 500 MHz) | LC-MS | |
|---|---|---|---|
| | | calc. | Found[M+1] |
| 2 | 8.56 (d) 2H, 8.37 (d) 2H, 8.15 (d) 2H, 7.93 (d) 2H, 7.51 (t) 3H, 7.36 (t) 2H, 7.18-11 (m) 4H, 1.36 (s) 18H, 1.31 (s) 18H | 711.43 | 712.41 |
| 4 | 8.57-8.53 (m) 3H, 8.37 (d) 1H, 8.17 (d) 1H, 7.96-7.92 (m) 3H, 7.88 (d) 1H, 7.64 (t) 1H, 7.56 (d) 1H, 7.48-7.32 (m) 18H, 7.18-7.13 (m) 4H, 1.34 (s) 18H, 1.32 (s) 18H | 857.39 | 858.37 |
| 5 | 8.56 (d) 2H, 8.15 (d) 2H, 7.93 (d) 2H, 7.88-7.4 (m) 2H, 7.67-7.63 (m) 2H, 7.48-7.32 (m) 17H, 7.17-7.13 (m) 4H, 1.36 (s) 18H, 1.31 (s) 18H | 969.62 | 970.61 |
| 23 | 8.57-8.54 (m) 3H, 8.17 (d) 1H, 8.05 (d) 2H, 7.96-7.93 (m) 3H, 7.82 (t) 1H, 7.38-7.30 (m) 11H, 7.17-7.14 (m) 4H, 1.36 (s) 9H, 1.32 (s) 18H, 1.30 (s) 9H | 863.49 | 864.43 |
| 28 | 8.57-8.53 (m) 3H, 8.16 (d) 1H, 7.95-7.92 (m) 4H, 7.87 (s) 1H, 7.63 (t) 1H, 7.62 (s) 1H, 7.54 (d) 1H, 7.48-7.35 (m) 15H, 7.37-7.32 (m) 2H, 7.17-7.12 (m) 4H, 1.35 (s) 9H, 1.32 (s) 9H | 856.40 | 857.41 |
| 30 | 8.54 (d) 1H, 8.19-8.15 (m) 3H, 7.99-7.95 (m) 2H, 7.90 (d) 1H, 7.83 (s) 1H, 7.77 (d) 1H, 7.58-7.50 (m) 5H, 7.40 (t) 1H, 7.35 (t) 1H, 7.29 (t) 1H, 7.20 (t) 2H, 7.17-7.12 (m) 2H, 1.36 (s) 9H, 1.32 (s) 9H | 688.32 | 689.31 |
| 41 | 8.58 (d) 2H, 8.32 (d) 1H, 8.16 (s) 1H, 8.09 (d) 1H, 7.93 (d) 2H, 7.87 (s) 1H, 7.75 (d) 1H, 7.67-7.62 (m) 2H, 7.48-7.35 (m) 30H, 7.34 (t) 2H, | 1191.51 | 1192.49 |
| | 7.15 (t) 2H, 7.11 (s) 1H, 1.36 (s) 9H, 1.31 (s) 9H | | |

### Example 1

As an anode, a Corning 15 Ω/cm² (1,200 Å) ITO glass substrate was cut to a size of 50 mm x 50 mm x 0.7 mm, sonicated with isopropyl alcohol and pure water each for 15 minutes, and then cleaned by exposure to ultraviolet rays and ozone for 30 minutes. The ITO glass substrate was loaded onto a vacuum deposition apparatus.

NPB was vacuum-deposited on the ITO anode formed on the ITO glass substrate to form a hole injection layer having a thickness of 300 Å, and then, mCP was vacuum-deposited on the hole injection layer to form a hole transport layer having a thickness of 250 Å.

Compound 2 (host) and Ir(pmp)₃ (dopant) were co-deposited at a weight ratio of 92:8 on the hole transport layer to form an emission layer having a thickness of 250 Å.

Subsequently, TAZ was deposited on the emission layer to form an electron transport layer having a thickness of 200 Å, LiF was deposited on the electron transport layer to form an electron injection layer having a thickness of 10 Å, and Al was vacuum-deposited on the electron injection layer to form a LiF/AI electrode having a thickness of 100 Å, thereby completing the manufacture of a light-emitting device.

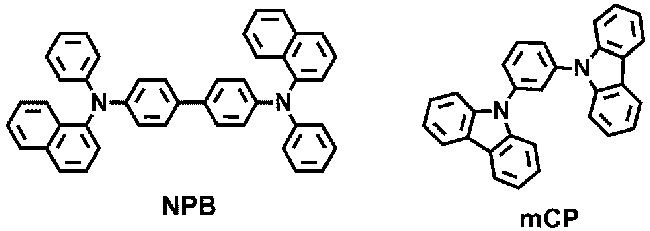

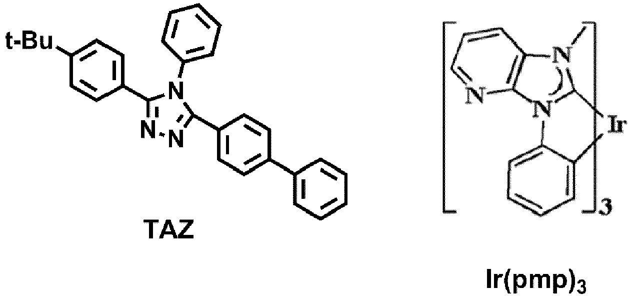

### Examples 2 to 7 and Comparative Example 1

Light-emitting devices were manufactured in the same manner as in Example 1, except that compounds shown in Table 2 were respectively used instead of Compound 2 in forming an emission layer.

### Evaluation Example 1

To evaluate characteristics of the light-emitting devices manufactured according to Examples 1 to 7 and Comparative Example 1, the driving voltage at the current density of 10 mA/cm², luminescence efficiency, and maximum external quantum efficiency (EQE) thereof were measured. The driving voltage of each of the light-emitting devices was measured using a source meter (Keithley Instrument Inc., 2400 series), and the EQE of each of the light-emitting devices was measured using an EQE measurement apparatus C9920-2-12 of Hamamatsu Photonics Inc. In evaluating the EQE, the luminance/current density was measured using a luminance meter that was calibrated for wavelength sensitivity, and the measured luminance/current density was converted into the EQE by assuming an angular luminance distribution (Lambertian) which introduced a perfect reflecting diffuser. The evaluation results of the characteristics of the light-emitting devices are shown in Table 2.

**Table 2**

| | Host in emission layer | Driving voltage (V) | Current density (mA/cm²) | EQE (%) | Emission color |
|---|---|---|---|---|---|
| Example 1 | 2 | 4.1 | 10 | 23.1 | Blue |
| Example 2 | 4 | 4.1 | 10 | 21.1 | Blue |
| Example 3 | 5 | 4.2 | 10 | 24.2 | Blue |
| Example 4 | 23 | 4.2 | 10 | 22.3 | Blue |
| Example 5 | 28 | 4.5 | 10 | 24.9 | Blue |
| Example 6 | 30 | 4.3 | 10 | 25.8 | Blue |
| Example 7 | 41 | 3.8 | 10 | 22.1 | Blue |
| Comparati ve Example 1 | Compou nd A | 4.8 | 10 | 17.2 | Blue |

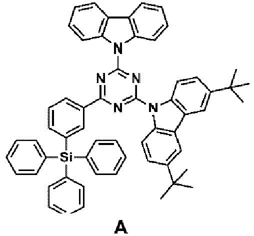

Referring to Table 2, it was confirmed that the light-emitting devices of Examples 1 to 7 had low driving voltage and excellent EQE, as compared with the light-emitting device of Comparative Example 1.

According to the one or more embodiments, the present disclosure relates to a novel heterocyclic compound, a light-emitting device including the heterocyclic compound, and an electronic apparatus including the light-emitting device. The heterocyclic compound disclosed herein may have excellent characteristics in terms of electrical stability, molecular polarity, and luminescence efficiency, and accordingly, the light-emitting device and the electronic apparatus that include the heterocyclic compound may have high luminescence efficiency and a long lifespan.

It should be understood that embodiments described herein should be considered in a descriptive sense only and not for purposes of limitation. Descriptions of features or aspects within each embodiment should typically be considered as available for other similar features or aspects in one or more embodiments. While one or more embodiments have been described with reference to the figures, it will be understood by those of ordinary skill in the art that various changes in form and details may be made therein without departing from the scope as defined by the following claims

## Claims

1. A light-emitting device comprising:
a first electrode;
a second electrode facing the first electrode;
an interlayer between the first electrode and the second electrode and comprising an emission layer; and
at least one heterocyclic compound represented by Formula 1: 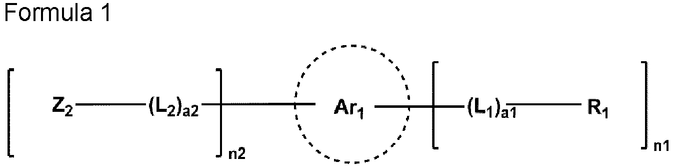 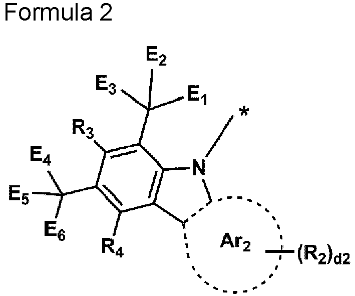 wherein, in Formulae 1 and 2,
Z₂ is a group represented by Formula 2,
Ar1 and Ar₂ are each independently a C₅-C₆₀ carbocyclic group or a C₁-C₆₀ heterocyclic group,
L₁ and L₂ are each independently a single bond, a C₅-C₆₀ carbocyclic group unsubstituted or substituted with at least one R₁₀ₐ, or a C₁-C₆₀ heterocyclic group unsubstituted or substituted with at least one R₁₀ₐ,
a1 and a2 are each independently an integer from 1 to 3,
R₁ to R₄ are each independently:
hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, - Si(Q₁)(Q₂)(Q₃), -B(Q₁)(Q₂), -C(=O)(Q₁), -S(=O)₂(Q₁), or -P(=O)(Q₁)(Q₂); or
a group represented by Formula 2,
E₁ to E₆ are each independently a C₁-C₆₀ alkyl group unsubstituted or substituted with at least one R₁₀ₐ, a C₂₋C₆₀ alkenyl group unsubstituted or substituted with at least one R₁₀ₐ, or a C₂-C₆₀ alkynyl group unsubstituted or substituted with at least one R₁₀ₐ,
d2 is an integer from 0 to 10,
n1 is an integer from 1 to 10,
n2 is an integer from 1 to 5,
* indicates a binding site to a neighboring atom,
R₁₀ₐ is:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₁₁)(Q₁₂)(Q₁₃), -N(Q₁₁)(Q₁₂), -B(Q₁₁)(Q₁₂), -C(=O)(Q₁₁), - S(=O)₂(Q₁₁), -P(=O)(Q₁₁)(Q₁₂), or any combination thereof;'
a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, or a C₂-C₆₀ heteroaryl alkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), - P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q1 to Q₃, Q₁₁ to Q₁₃, Q₂₁ to Q₂₃, and Q₃₁ to Q₃₃ are each independently: hydrogen; deuterium; -F; -Cl; -Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; or a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a C₃-C₆₀ carbocyclic group, a C₁-C₆₀ heterocyclic group, or any combination thereof.

2. The light-emitting device of claim 1, wherein the first electrode is an anode,
the second electrode is a cathode,
the interlayer further comprises a hole transport region between the emission layer and the second electrode,
the interlayer further comprises an electron transport region between the emission layer and the second electrode,
the hole transport region comprises a hole injection layer, a hole transport layer, an emission auxiliary layer, an electron blocking layer, or any combination thereof, and
the electron transport region comprises a buffer layer, a hole blocking layer, an electron control layer, an electron transport layer, an electron injection layer, or any combination thereof.

3. The light-emitting device of claim 1 or claim 2, wherein the emission layer comprises the at least one heterocyclic compound represented by Formula 1.

4. The light-emitting device of claim 3, wherein the emission layer further comprises an organometallic compound represented by Formula 401:
Formula 401 M(L₄₀₁)_{xc1}(L₄₀₂)_{xc2}
wherein, in Formula 401,
M is a transition metal,
L₄₀₁ is a ligand represented by Formula 402, and xc1 is 1, 2, or 3, wherein, when xc1 is 2 or more, two or more of L₄₀₁ are identical to or different from each other, and
L₄₀₂ is an organic ligand, and xc2 is 0, 1, 2, 3, or 4, wherein, when xc2 is 2 or more, two or more of L₄₀₂ are identical to or different from each other, 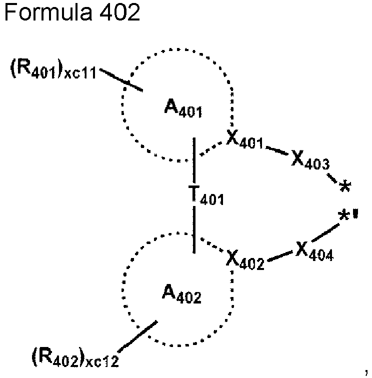 wherein, in Formula 402,
X₄₀₁ and X₄₀₂ are each independently nitrogen or carbon,
ring A₄₀₁ and ring A₄₀₂ are each independently a C₃-C₆₀ carbocyclic group or a C₁-C₆₀ heterocyclic group,
T401 is a single bond, *-O-*', *-S-*', *-C(=O)-*', *-N(Q₄₁₁)-*', *-C(Q₄₁₁)(Q₄₁₂)-*', *-C(Q₄₁₁)=C(Q₄₁₂)-*', *-C(Q₄₁₁)=*', or *=C(Q₄₁₁)=*',
X₄₀₃ and X₄₀₄ are each independently a chemical bond, O, S, N(Q₄₁₃), B(Q₄₁₃), P(Q₄₁₃), C(Q₄₁₃)(Q₄₁₄), or Si(Q₄₁₃)(Q₄₁₄),
R₄₀₁ and R₄₀₂ are each independently hydrogen, deuterium, -F, -Cl, -Br, -I, a hydroxyl group, a cyano group, a nitro group, a C₁-C₂₀ alkyl group unsubstituted or substituted with at least one R₁₀ₐ, a C₁-C₂₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, - Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), -S(=O)₂(Q₄₀₁), or - P(=O)(Q₄₀₁)(Q₄₀₂),
Q₄₀₁ to Q₄₀₃ and Q₄₁₁ to Q₄₁₄ are the same as described in connection with Q₁ in claim 1,
R₁₀ₐ is the same as described in claim 1,
xc11 and xc12 are each independently an integer from 0 to 10, and * and *' in Formula 402 each indicate a binding site to M in Formula 401.

5. An electronic apparatus comprising the light-emitting device of any one of claims 1 to 4.

6. The electronic apparatus of claim 5, further comprising:
a thin-film transistor, wherein
the thin-film transistor comprises a source electrode and a drain electrode, and
the first electrode of the light-emitting device is electrically connected to the source electrode or the drain electrode; optionally further comprising:
a color filter, a color conversion layer, a touch screen layer, a polarizing layer, or any combination thereof.

7. A heterocyclic compound represented by Formula 1: 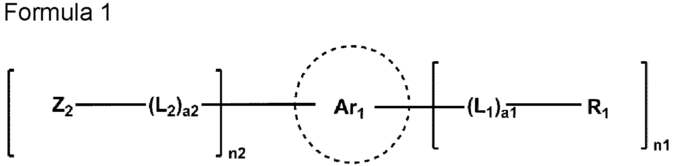 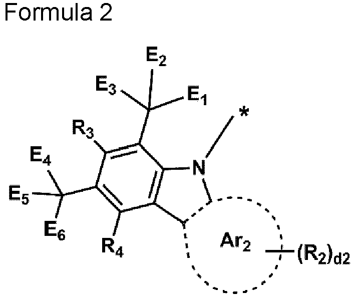 wherein, in Formulae 1 and 2,
Z₂ is a group represented by Formula 2,
Ar₁ and Ar₂ are each independently a C₅-C₆₀ carbocyclic group or a C₁-C₆₀ heterocyclic group,
L₁ and L₂ are each independently a single bond, a C₅-C₆₀ carbocyclic group unsubstituted or substituted with at least one R₁₀ₐ, or a C₁-C₆₀ heterocyclic group unsubstituted or substituted with at least one R₁₀ₐ,
a1 and a2 are each independently an integer from 1 to 3,
R₁ to R₄ are each independently:
hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, - Si(Q₁)(Q₂)(Q₃), -B(Q₁)(Q₂), -C(=O)(Q₁), -S(=O)₂(Q₁), or -P(=O)(Q₁)(Q₂); or
a group represented by Formula 2,
E₁ to E₆ are each independently a C₁-C₆₀ alkyl group unsubstituted or substituted with at least one R₁₀ₐ, a C₂₋C₆₀ alkenyl group unsubstituted or substituted with at least one R₁₀ₐ, or a C₂₋C₆₀ alkynyl group unsubstituted or substituted with at least one R₁₀ₐ,
d2 is an integer from 0 to 10,
n1 is an integer from 1 to 10,
n2 is an integer from 1 to 5,
* indicates a binding site to a neighboring atom,
R₁₀ₐ is:
deuterium, -F, -Cl, -Br, -I, a hydroxyl group, a cyano group, or a nitro group; a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₁₁)(Q₁₂)(Q₁₃), -N(Q₁₁)(Q₁₂), -B(Q₁₁)(Q₁₂), -C(=O)(Q₁₁), - S(=O)₂(Q₁₁), -P(=O)(Q₁₁)(Q₁₂), or any combination thereof; a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, or a C₂-C₆₀ heteroaryl alkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), - P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q1 to Q₃, Q₁₁ to Q₁₃, Q₂₁ to Q₂₃, and Q₃₁ to Q₃₃ are each independently: hydrogen; deuterium; -F; -Cl; -Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; or a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a C₃-C₆₀ carbocyclic group, a C₁-C₆₀ heterocyclic group, or any combination thereof;
wherein the heterocyclic compound represented by Formula 1 satisfies at least one of Conditions 1 and 2:
Condition 1
Ar1 is a π electron-deficient nitrogen-containing C₁-C₆₀ cyclic group; and
Condition 2
wherein at least one of R₁(s) in the number of n1 is -Si(Q₁)(Q₂)(Q₃).

8. The heterocyclic compound of claim 7, wherein in
Condition 2
n2 is an integer from 1 to 3

9. The heterocyclic compound of claim 7 or claim 8, wherein the heterocyclic compound represented by Formula 1 is represented by Formula 1-A: 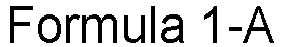 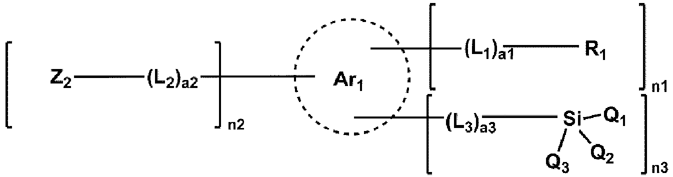 wherein, in Formula 1-A,
Z₂, Ar₁, L₁, L₂, n1, n2, a1, a2, R₁, and Q₁ to Q₃ are each as described in claim 7, L₃ is as described in connection with L₁ in claim 7,
a3 is as described in connection with a1 in claim 7, and
n3 is an integer from 0 to 10.

10. The heterocyclic compound of any one of claims 7 to 9, wherein Ar₁ is a cyclopentadiene group, an adamantane group, a norbornane group, a benzene group, a pentalene group, a naphthalene group, an azulene group, an indacene group, an acenaphthylene group, a phenalene group, a phenanthrene group, an anthracene group, a fluoranthene group, a triphenylene group, a pyrene group, a chrysene group, a perylene group, a pentaphene group, a heptalene group, a naphthacene group, a picene group, a hexacene group, a pentacene group, a rubicene group, a coronene group, an ovalene group, an indene group, a fluorene group, a spiro-bifluorene group, a benzofluorene group, an indenophenanthrene group, an indenoanthracene group, a 1H-pyrrole group, a silole group, a borole group, a 2H-pyrrole group, a 3H-pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, or an azadibenzofuran group, and/or wherein L₁ and L₂ are each independently:
a single bond; or
a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-a fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluoren-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group, each unsubstituted or substituted with at least one R₁₀ₐ, and
R₁₀ₐ is as described in claim 8.

11. The heterocyclic compound of any one of claims 7 to 10, wherein L₁ and L₂ are each independently:
a single bond; or
one of groups represented by Formulae 3-1 to 3-40: 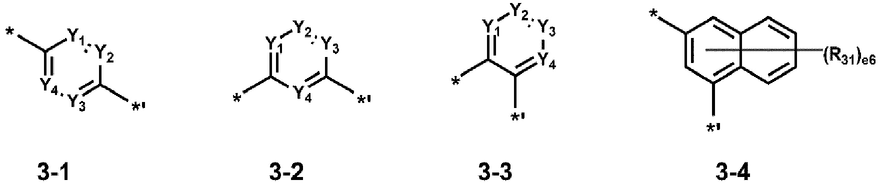 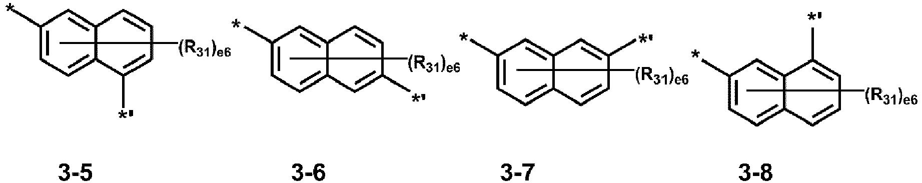 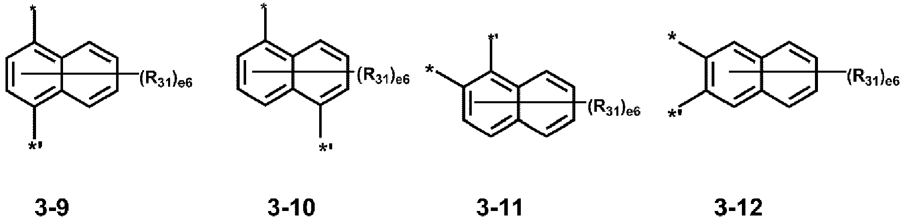 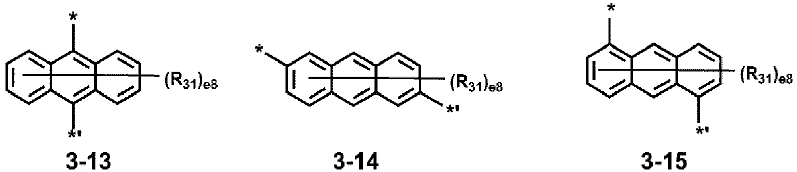 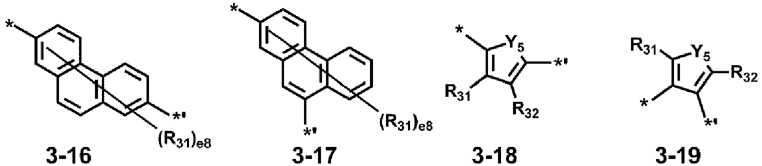 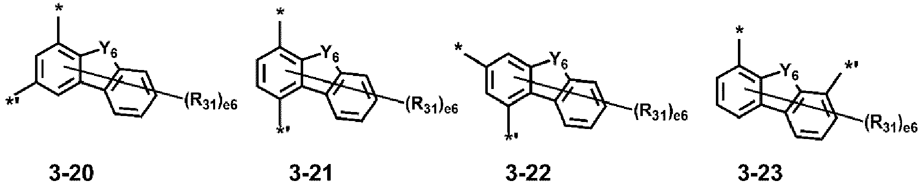 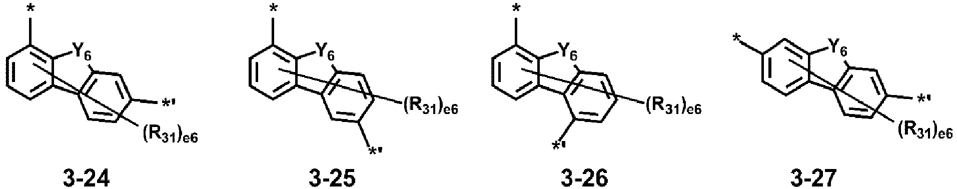 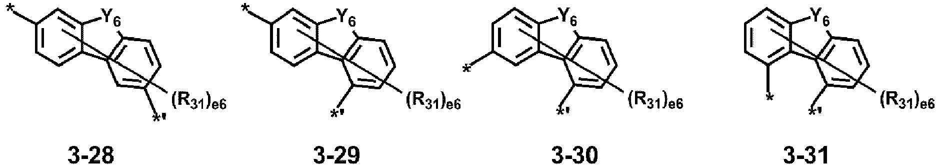 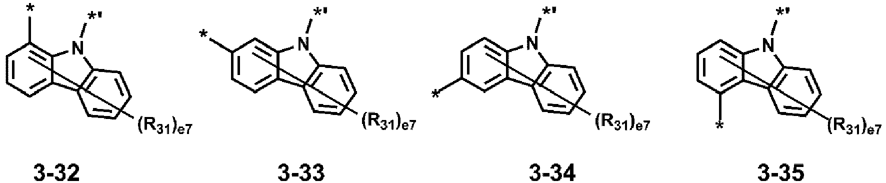 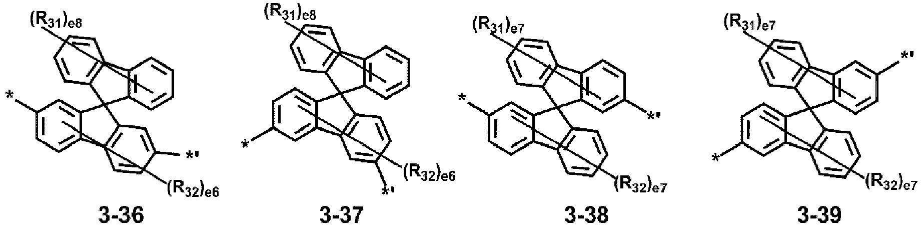 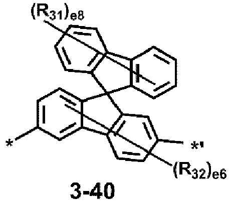 wherein, in Formulae 3-1 to 3-40,
Y₁ is N or C(R₃₃),
Y₂ is N or C(R₃₄),
Y₃ is N or C(R₃₅),
Y₄ is N or C(R₃₆),
Y₅ is O, S, or Se,
Y₆ is O, S, Se, N(R₃₇), or C(R₃₇)(R₃₈),
R₃₁ to R₃₈ are each independently:
hydrogen, deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, -Si(Q₁₁)(Q₁₂)(Q₁₃), -B(Q₁₁)(Q₁₂), - C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group; or a C₆-C₆₀ arylthio group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, -Si(Q₂₁)(Q₂₂)(Q₂₃), -B(Q₂₁)(Q₂₂), -C(=O)(Q₂₁), -S(=O)₂(Q₂₁), -P(=O)(Q₂₁)(Q₂₂), or any combination thereof; or
-Si(Q₃₁)(Q₃₂)(Q₃₃), -B(Q₃₁)(Q₃₂), -C(=O)(Q₃₁), -S(=O)₂(Q₃₁), or -P(=O)(Q₃₁)(Q₃₂), Q11 to Q₁₃, Q₂₁ to Q₂₃, and Q₃₁ to Q₃₃ are each as described in claim 7,
e6 is an integer from 0 to 6,
e7 is an integer from 0 to 7,
e8 is an integer from 0 to 8, and
* and *' each indicate a binding site to a neighboring atom.

12. The heterocyclic compound of any one of claims 7 to 11, wherein the heterocyclic compound represented by Formula 1 is represented by one of Formulae 1-1 to 1-5: 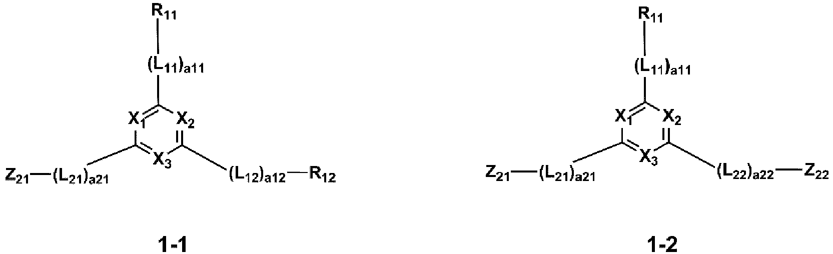 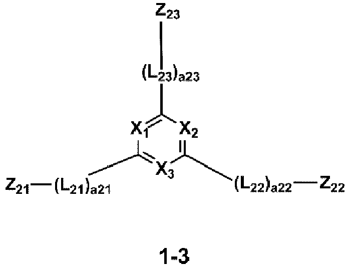 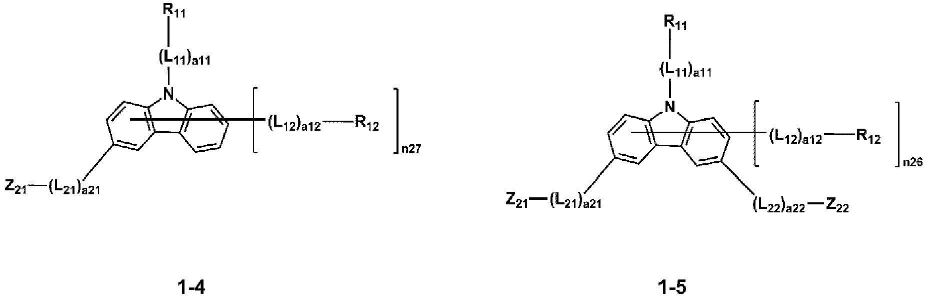 wherein, in Formulae 1-1 to 1-5,
X₁ is C(R₁₃) or N,
X₂ is C(R₁₄) or N,
X₃ is C(R₁₅) or N,
R₁₁ to R₁₅ are each as described in connection with R₁ in claim 7,
L₁₁ and L₁₂ are each as described in connection with L₁ in claim 7,
a11 and a12 are each as described in connection with a1 in claim 7,
n26 is an integer from 1 to 6,
n27 is an integer from 1 to 7,
L₂₁ to L₂₃ are each as described in connection with L₂ in claim 7,
a21 to a23 are each as described in connection with a2 in claim 7, and
Z₂₁ to Z₂₃ are each as described in connection with Z₂ in claim 7.

13. The heterocyclic compound of claim 12, wherein at least one of X₁ to X₃ is N, and/or, wherein R₁₁ is -Si(Q₁)(Q₂)(Q₃), and
Q₁ to Q₃ are each as described in claim 7.

14. The heterocyclic compound of claim 7, wherein E₁ to E₆ are each independently:
a C₁-C₂₀ alkyl group, a C₂-C₆₀ alkenyl group, or a C₂-C₆₀ alkynyl group; or
a C₁-C₂₀ alkyl group, a C₂-C₆₀ alkenyl group, or a C₂-C₂₀ alkynyl group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazine group, a hydrazone group, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a biphenyl group, a naphthyl group, a pyridinyl group, a pyrimidinyl group, or any combination thereof, and/or, wherein the group represented by Formula 2 is represented by Formula 2-1: 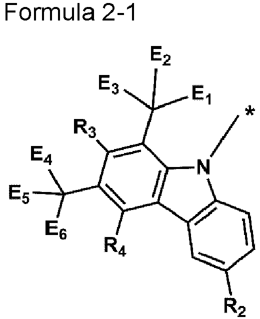 wherein, in Formula 2-1,
E₁ to E₆ and R₂ to R₄ are each as described in claim 7, and
* indicates a binding site to a neighboring atom.

15. The heterocyclic compound of claim 7, wherein the heterocyclic compound is one of Compounds 1 to 50:

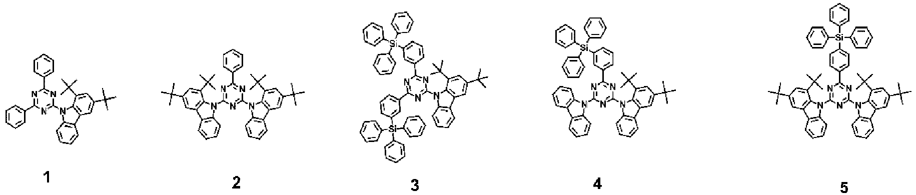

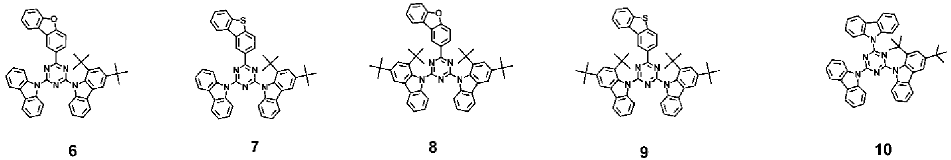

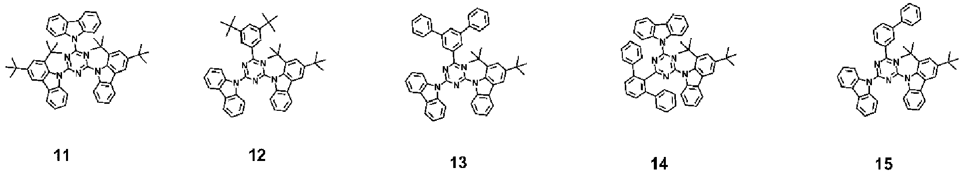

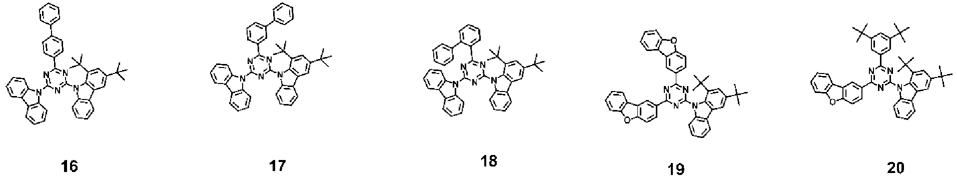

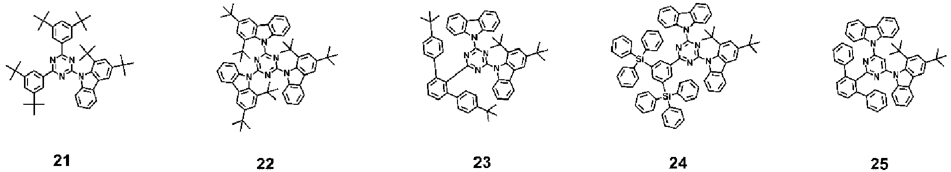

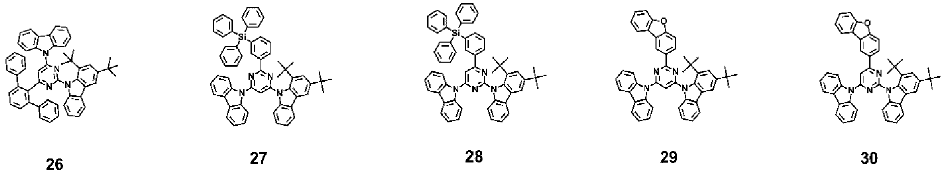

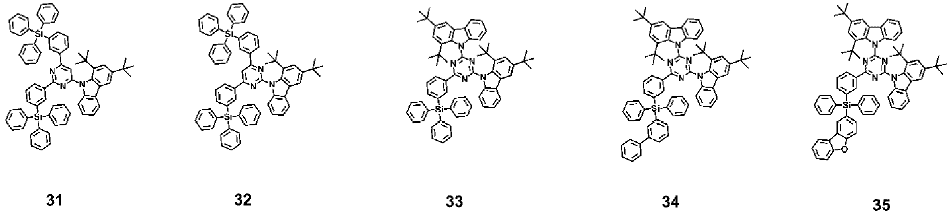

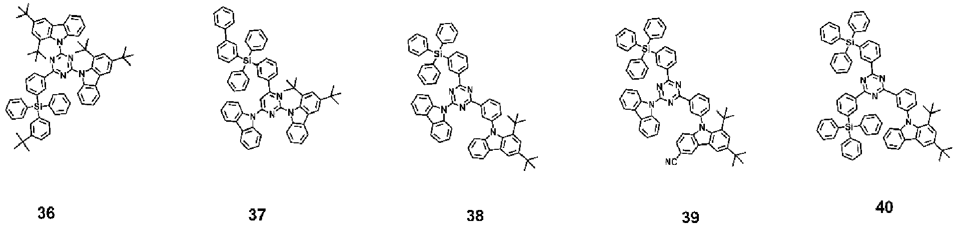

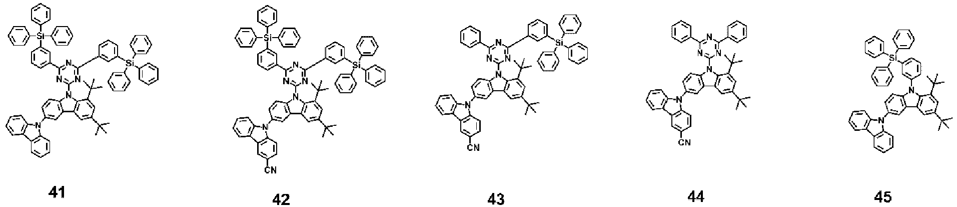

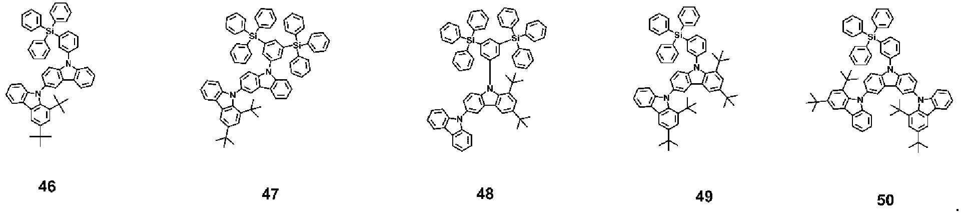

## Patentansprüche

1. Lichtemittierende Vorrichtung, die Folgendes umfasst:
eine erste Elektrode;
eine zweite Elektrode, die der ersten Elektrode gegenüberliegt;
eine Zwischenschicht zwischen der ersten Elektrode und der zweiten Elektrode und eine Emissionsschicht umfassend; und
mindestens eine heterocyclische Verbindung, dargestellt durch Formel 1: 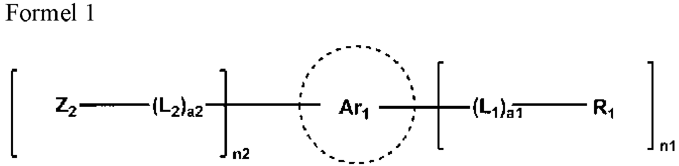 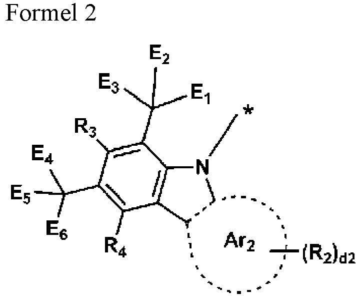 wobei in Formeln 1 und 2
Z₂ eine durch Formel 2 dargestellte Gruppe ist,
Ar₁ und Ar₂ jeweils unabhängig eine carbocyclische C₅-C₆₀-Gruppe oder eine heterocyclische C₁-C₆₀-Gruppe sind,
L₁ und L₂ jeweils unabhängig eine Einfachbindung, eine carbocyclische C₅-C₆₀-Gruppe, die unsubstituiert oder mit mindestens einem R₁₀ₐ substituiert ist, oder eine heterocyclische C₁-C₆₀-Gruppe sind, die unsubstituiert oder mit mindestens einem R₁₀ₐ substituiert ist,
a1 und a2 jeweils unabhängig eine ganze Zahl von 1 bis 3 sind,
R₁ bis R₄ jeweils unabhängig Folgendes sind:
Wasserstoff, Deuterium, -F, -Cl, -Br, -I, eine Hydroxygruppe, eine Cyanogruppe, eine Nitrogruppe, eine C₁-C₆₀-Alkylgruppe, die unsubstituiert oder mit mindestens einem R₁₀ₐ substituiert ist, eine C₂-C₆₀-Alkenylgruppe, die unsubstituiert oder mit mindestens einem R₁₀ₐ substituiert ist, eine C₂-C₆₀-Alkinylgruppe, die unsubstituiert oder mit mindestens einem R₁₀ₐ substituiert ist, eine C₁-C₆₀-Alkoxygruppe, die unsubstituiert oder mit mindestens einem R₁₀ₐ substituiert ist, eine carbocyclische C₃-C₆₀-Gruppe, die unsubstituiert oder mit mindestens einem R₁₀ₐ substituiert ist, eine heterocyclische C₁-C₆₀-Gruppe, die unsubstituiert oder mit mindestens einem R₁₀ₐ substituiert ist, eine C₆-C₆₀-Aryloxygruppe, die unsubstituiert oder mit mindestens einem R₁₀ₐ substituiert ist, eine C₆-C₆₀-Arylthiogruppe, die unsubstituiert oder mit mindestens einem R₁₀ₐ substituiert ist, -Si(Q₁)(Q₂)(Q₃), -B(Q₁)(Q₂), -C(=O)(Q₁), -S(=O)₂(Q₁) oder - P(=O)(Q₁)(Q₂); oder
eine durch Formel 2 dargestellte Gruppe,
E₁ bis E₆ jeweils unabhängig eine C₁-C₆₀-Alkylgruppe ist, die unsubstituiert oder mit mindestens einem R₁₀ₐ substituiert ist, eine C₂-C₆₀-Alkenylgruppe, die unsubstituiert oder mit mindestens einem R₁₀ₐ substituiert ist, oder eine C₂-C₆₀-Alkinylgruppe, die unsubstituiert oder mit mindestens einem R₁₀ₐ substituiert ist,
d2 eine ganze Zahl von 0 bis 10 ist,
n1 eine ganze Zahl von 1 bis 10 ist,
n2 eine ganze Zahl von 1 bis 5 ist,
* eine Bindungsstelle zu einem benachbarten Atom anzeigt,
R₁₀ₐ Folgendes ist:
Deuterium, -F, -Cl, -Br, -I, eine Hydroxygruppe, eine Cyanogruppe, oder eine Nitrogruppe;
eine C₁-C₆₀-Alkylgruppe, eine C₂-C₆₀-Alkenylgruppe, eine C₂-C₆₀-Alkinylgruppe, oder eine C₁-C₆₀-Alkoxygruppe, jeweils unsubstituiert oder substituiert mit Deuterium, -F, -Cl, -Br, -I, eine Hydroxygruppe, eine Cyanogruppe, eine Nitrogruppe, eine carbocyclische C₃-C₆₀-Gruppe, eine heterocyclische C₁-C₆₀-Gruppe, eine C₆-C₆₀-Aryloxygruppe, eine C₆-C₆₀-Arylthiogruppe, eine C₇-C₆₀-Arylalkylgruppe, eine C₂-C₆₀-Heteroarylalkylgruppe, -Si(Q₁₁)(Q₁₂)(Q₁₃), - N(Q₁₁)(Q₁₂), -B(Q₁₁)(Q₁₂), -C(=O)(Q₁₁), -S(=O)₂(Q₁₁), -P(=O)(Q₁₁)(Q₁₂) oder eine Kombination davon;
eine carbocyclische C₃-C₆₀-Gruppe, eine heterocyclische C₁-C₆₀-Gruppe, eine C₆-C₆₀-Aryloxygruppe, eine C₆-C₆₀-Arylthiogruppe, eine C₇-C₆₀-Arylalkylgruppe, oder eine C₂-C₆₀-Heteroarylalkylgruppe, jeweils unsubstituiert oder substituiert mit Deuterium, -F, -Cl, -Br, -I, eine Hydroxygruppe, eine Cyanogruppe, eine Nitrogruppe, eine C₁-C₆₀-Alkylgruppe, eine C₂-C₆₀-Alkenylgruppe, eine C₂-C₆₀-Alkinylgruppe, eine C₁-C₆₀-Alkoxygruppe, eine carbocyclische C₃-C₆₀-Gruppe, eine heterocyclische C₁-C₆₀-Gruppe, eine C₆-C₆₀-Aryloxygruppe, eine C₆-C₆₀-Arylthiogruppe, eine C₇-C₆₀-Arylalkylgruppe, eine C₂-C₆₀-Heteroarylalkylgruppe, - Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), -P(=O)(Q₂₁)(Q₂₂) oder eine Kombination davon; oder
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), oder - P(=O)(Q₃₁)(Q₃₂), und
Q₁ bis Q₃, Q₁₁ bis Q₁₃, Q₂₁ bis Q₂₃ und Q₃₁ bis Q₃₃ jeweils unabhängig: Wasserstoff; Deuterium; -F; -Cl; -Br; -I; eine Hydroxygruppe; eine Cyanogruppe; eine Nitrogruppe; eine C₁-C₆₀-Alkylgruppe; eine C₂-C₆₀-Alkenylgruppe, eine C₂-C₆₀-Alkinylgruppe, eine C₁-C₆₀-Alkoxygruppe; oder eine carbocyclische C₃-C₆₀-Gruppe oder eine heterocyclische C₁-C₆₀-Gruppe sind, jeweils unsubstituiert oder substituiert mit Deuterium, -F, eine Cyanogruppe, eine C₁-C₆₀-Alkylgruppe, eine C₁-C₆₀-Alkoxygruppe, eine carbocyclische C₃-C₆₀-Gruppe, eine heterocyclische C₁-C₆₀-Gruppe oder eine Kombination davon.

2. Lichtemittierende Vorrichtung nach Anspruch 1, wobei die erste Elektrode eine Anode ist,
die zweite Elektrode eine Kathode ist,
die Zwischenschicht ferner einen Lochtransportbereich zwischen der Emissionsschicht und der zweiten Elektrode umfasst,
die Zwischenschicht ferner einen Elektronentransportbereich zwischen der Emissionsschicht und der zweiten Elektrode umfasst,
der Lochtransportbereich eine Lochinjektionsschicht, eine Lochtransportschicht, eine Emissionshilfsschicht, eine Elektronenblockierschicht oder eine Kombination davon umfasst, und
der Elektronentransportbereich eine Pufferschicht, eine Lochblockierschicht, eine Elektronenkontrollschicht, eine Elektronentransportschicht, eine Elektroneninj ektionsschicht oder eine Kombination davon umfasst.

3. Lichtemittierende Vorrichtung nach Anspruch 1 oder Anspruch 2, wobei die Emissionsschicht die mindestens eine durch Formel 1 dargestellte heterocyclische Verbindung umfasst.

4. Lichtemittierende Vorrichtung nach Anspruch 3, wobei die Emissionsschicht ferner eine Organometallverbindung dargestellt durch Formel 401 umfasst:
Formel 401 M(L₄₀₁)_{xc1}(L₄₀₂)_{xc2}
wobei in Formel 401
M ein Übergangsmetall ist,
L₄₀₁ ein Ligand ist, der durch Formel 402 dargestellt wird, und xc1 1, 2 oder 3 ist, wobei, wenn xc1 2 oder mehr ist, zwei oder mehr von L₄₀₁ miteinander identisch oder voneinander verschieden sind, und
L₄₀₂ ein organischer Ligand ist und xc2 0, 1, 2, 3 oder 4 ist, wobei, wenn xc2 2 oder mehr ist, zwei oder mehr von L₄₀₂ miteinander identisch oder voneinander verschieden sind, 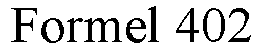 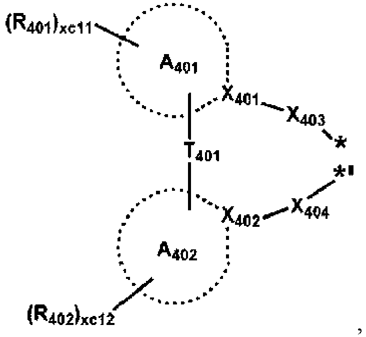 wobei in Formel 402
X₄₀₁ und X₄₀₂ jeweils unabhängig Stickstoff oder Kohlenstoff sind,
Ring A₄₀₁ und Ring A₄₀₂ jeweils unabhängig eine carbocyclische C₃-C₆₀-Gruppe oder eine heterocyclische C₁-C₆₀-Gruppe sind,
T₄₀₁ eine Einfachbindung ist, *-O-*', *-S-*', *-C(=O)-*', *-N(Q₄₁₁)-*', *-C(Q₄₁₁)(Q₄₁₂)-*', *-C(Q₄₁₁)=C(Q₄₁₂)-*', *-C(Q₄₁₁)=*', oder *=C(Q₄₁₁)=*',
X₄₀₃ und X₄₀₄ jeweils unabhängig eine chemische Bindung, O, S, N(Q₄₁₃), B(Q₄₁₃), P(Q₄₁₃), C(Q₄₁₃)(Q₄₁₄), oder Si(Q₄₁₃)(Q₄₁₄) sind,
R₄₀₁ und R₄₀₂ jeweils unabhängig Wasserstoff, Deuterium, -F, -Cl, -Br, -I, eine Hydroxygruppe, eine Cyanogruppe, eine Nitrogruppe, eine C₁-C₂₀-Alkylgruppe, die unsubstituiert oder mit mindestens einem R₁₀ₐ substituiert ist, eine C₁-C₂₀-Alkoxygruppe, die unsubstituiert oder mit mindestens einem R₁₀ₐ substituiert ist, eine carbocyclische C₃-C₆₀-Gruppe, die unsubstituiert oder mit mindestens einem R₁₀ₐ substituiert ist, eine heterocyclische C₁-C₆₀-Gruppe, die unsubstituiert oder mit mindestens einem R₁₀ₐ substituiert ist, -Si(Q₄₀₁)(Q₄₀₂)(Q₄₀₃), - N(Q₄₀₁)(Q₄₀₂), -B(Q₄₀₁)(Q₄₀₂), -C(=O)(Q₄₀₁), -S(=O)₂(Q₄₀₁) oder -P(=O)(Q₄₀₁)(Q₄₀₂) sind,
Q₄₀₁ bis Q₄₀₃ und Q₄₁₁ bis Q₄₁₄ dieselben wie die in Zusammenhang mit Q₁ in Anspruch 1 beschriebenen sind,
R₁₀ₐ dasselbe wie das in Anspruch 1 beschriebene ist,
xc111 und xc12 jeweils unabhängig eine ganze Zahl von 0 bis 10 sind, und
* und *' in Formel 402 jeweils eine Bindungsstelle an M in Formel 401 anzeigen.

5. Elektronisches Gerät, umfassend die lichtemittierende Vorrichtung nach einem der Ansprüche 1 bis 4.

6. Elektronisches Gerät nach Anspruch 5, ferner umfassend:
einen Dünnschichttransistor, wobei
der Dünnschichttransistor eine Source-Elektrode und eine Drain-Elektrode umfasst, und
die erste Elektrode der lichtemittierenden Vorrichtung elektrisch mit der Source-Elektrode oder der Drain-Elektrode verbunden ist; gegebenenfalls ferner umfassend:
einen Farbfilter, eine Farbumwandlungsschicht, eine Touchscreen-Schicht, eine Polarisationsschicht oder eine Kombination davon.

7. Heterocyclische Verbindung, dargestellt durch Formel 1: 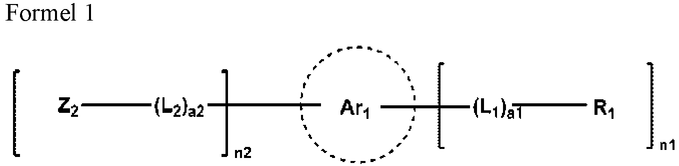 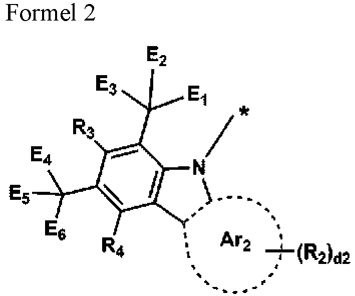 wobei in Formeln 1 und 2
Z₂ eine durch Formel 2 dargestellte Gruppe ist,
Ar₁ und AR₂ jeweils unabhängig eine carbocyclische C₅-C₆₀-Gruppe oder eine heterocyclische C₁-C₆₀-Gruppe sind,
L₁ und L₂ jeweils unabhängig eine Einfachbindung, eine carbocyclische C₅-C₆₀-Gruppe, die unsubstituiert oder mit mindestens einem R₁₀ₐ substituiert ist, oder eine heterocyclische C₁-C₆₀-Gruppe sind, die unsubstituiert oder mit mindestens einem R₁₀ₐ substituiert ist,
a1 und a2 jeweils unabhängig eine ganze Zahl von 1 bis 3 sind,
R₁ bis R₄ jeweils unabhängig Folgendes sind:
Wasserstoff, Deuterium, -F, -Cl, -Br, -I, eine Hydroxygruppe, eine Cyanogruppe, eine Nitrogruppe, eine C₁-C₆₀-Alkylgruppe, die unsubstituiert oder mit mindestens einem R₁₀ₐ substituiert ist, eine C₂-C₆₀-Alkenylgruppe, die unsubstituiert oder mit mindestens einem R₁₀ₐ substituiert ist, eine C₂-C₆₀-Alkinylgruppe, die unsubstituiert oder mit mindestens einem R₁₀ₐ substituiert ist, eine C₁-C₆₀-Alkoxygruppe, die unsubstituiert oder mit mindestens einem R₁₀ₐ substituiert ist, eine carbocyclische C₃-C₆₀-Gruppe, die unsubstituiert oder mit mindestens einem R₁₀ₐ substituiert ist, eine heterocyclische C₁-C₆₀-Gruppe, die unsubstituiert oder mit mindestens einem R₁₀ₐ substituiert ist, eine C₆-C₆₀-Aryloxygruppe, die unsubstituiert oder mit mindestens einem R₁₀ₐ substituiert ist, eine C₆-C₆₀-Arylthiogruppe, die unsubstituiert oder mit mindestens einem R₁₀ₐ substituiert ist, -Si(Q₁)(Q₂)(Q₃), -B(Q₁)(Q₂), -C(=O)(Q₁), -S(=O)₂(Q₁), oder - P(=O)(Q₁)(Q₂); oder
eine durch Formel 2 dargestellte Gruppe,
E₁ bis E₆ jeweils unabhängig eine C₁-C₆₀-Alkylgruppe sind, die unsubstituiert oder mit mindestens einem R₁₀ₐ substituiert ist, eine C₂-C₆₀-Alkenylgruppe, die unsubstituiert oder mit mindestens einem R₁₀ₐ substituiert ist, oder eine C₂-C₆₀-Alkinylgruppe, die unsubstituiert oder mit mindestens einem R₁₀ₐ substituiert ist,
d2 eine ganze Zahl von 0 bis 10 ist,
n1 eine ganze Zahl von 1 bis 10 ist,
n2 eine ganze Zahl von 1 bis 5 ist,
* eine Bindungsstelle zu einem benachbarten Atom anzeigt,
R₁₀ₐ Folgendes ist:
Deuterium, -F, -Cl, -Br, -I, eine Hydroxygruppe, eine Cyanogruppe, oder eine Nitrogruppe;
eine C₁-C₆₀-Alkylgruppe, eine C₂-C₆₀-Alkenylgruppe, eine C₂-C₆₀-Alkinylgruppe, oder eine C₁-C₆₀-Alkoxygruppe, jeweils unsubstituiert oder substituiert mit Deuterium, -F, -Cl, -Br, -I, eine Hydroxygruppe, eine Cyanogruppe, eine Nitrogruppe, eine carbocyclische C₃-C₆₀-Gruppe, eine heterocyclische C₁-C₆₀-Gruppe, eine C₆-C₆₀-Aryloxygruppe, eine C₆-C₆₀-Arylthiogruppe, eine C₇-C₆₀-Arylalkylgruppe, eine C₂-C₆₀-Heteroarylalkylgruppe, -Si(Q₁₁)(Q₁₂)(Q₁₃), - N(Q₁₁)(Q₁₂), -B(Q₁₁)(Q₁₂), -C(=O)(Q₁₁), -S(=O)₂(Q₁₁), -P(=O)(Q₁₁)(Q₁₂) oder eine Kombination davon;
eine carbocyclische C₃-C₆₀-Gruppe, eine heterocyclische C₁-C₆₀-Gruppe, eine C₆-C₆₀-Aryloxygruppe, eine C₆-C₆₀-Arylthiogruppe, eine C₇-C₆₀-Arylalkylgruppe, oder eine C₂-C₆₀-Heteroarylalkylgruppe, jeweils unsubstituiert oder substituiert mit Deuterium, -F, -Cl, -Br, -I, eine Hydroxygruppe, eine Cyanogruppe, eine Nitrogruppe, eine C₁-C₆₀-Alkylgruppe, eine C₂-C₆₀-Alkenylgruppe, eine C₂-C₆₀-Alkinylgruppe, eine C₁-C₆₀-Alkoxygruppe, eine carbocyclische C₃-C₆₀-Gruppe, eine heterocyclische C₁-C₆₀-Gruppe, eine C₆-C₆₀-Aryloxygruppe, eine C₆-C₆₀-Arylthiogruppe, eine C₇-C₆₀-Arylalkylgruppe, eine C₂-C₆₀-Heteroarylalkylgruppe, - Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), -P(=O)(Q₂₁)(Q₂₂) oder eine beliebige Kombination davon; oder
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), oder - P(=O)(Q₃₁)(Q₃₂), und
Q₁ bis Q₃, Q₁₁ bis Q₁₃, Q₂₁ bis Q₂₃ und Q₃₁ bis Q₃₃ jeweils unabhängig: Wasserstoff; Deuterium; -F; -Cl; -Br; -I; sind, eine Hydroxygruppe; eine Cyanogruppe; eine Nitrogruppe; eine C₁-C₆₀-Alkylgruppe, eine C₂-C₆₀-Alkenylgruppe, eine C₂-C₆₀-Alkinylgruppe, eine C₁-C₆₀-Alkoxygruppe; oder eine carbocyclische C₃-C₆₀-Gruppe oder eine heterocyclische C₁-C₆₀-Gruppe sind, jeweils unsubstituiert oder substituiert mit Deuterium, -F, eine Cyanogruppe, eine C₁-C₆₀-Alkylgruppe, eine C₁-C₆₀-Alkoxygruppe, eine carbocyclische C₃-C₆₀-Gruppe, eine heterocyclische C₁-C₆₀-Gruppe oder eine Kombination davon;
wobei die durch Formel 1 dargestellte heterocyclische Verbindung mindestens eine der Bedingungen 1 und 2 erfüllt:
Bedingung 1
An ist eine π-elektronenarme, stickstoffhaltige cyclische C₁-C₆₀-Gruppe; und
Bedingung 2
wobei mindestens eines von R₁(s) in der Anzahl von n1 -Si(Q₁)(Q₂)(Q₃) ist.

8. Heterocyclische Verbindung nach Anspruch 7, wobei in Bedingung 2 n2 eine ganze Zahl von 1 bis 3 ist.

9. Heterocyclische Verbindung nach Anspruch 7 oder Anspruch 8, wobei die durch Formel 1 dargestellte heterocyclische Verbindung durch Formel 1-A dargestellt wird: 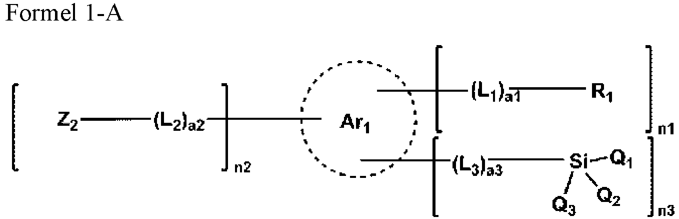 wobei in Formel 1-A
Z₂, Ar₁, L₁, L₂, n1, n2, a1, a2, R₁ und Q₁ bis Q₃ jeweils wie in Anspruch 7 beschrieben sind,
L₃ dasselbe wie in Zusammenhang mit L₁ in Anspruch 7 beschriebene ist,
a3 dasselbe wie in Zusammenhang mit a1 in Anspruch 7 beschriebene ist, und
n3 eine ganze Zahl von 0 bis 10 ist.

10. Heterocyclische Verbindung nach einem der Ansprüche 7 bis 9, wobei Ar₁ eine Cyclopentadiengruppe, eine Adamantangruppe, eine Norbornangruppe, eine Benzolgruppe, eine Pentalengruppe, eine Naphthalingruppe, eine Azulengruppe, eine Indacengruppe, eine Acenaphthylengruppe, eine Phenalengruppe, eine Phenanthrengruppe, eine Anthracengruppe, eine Fluoranthengruppe, eine Triphenylengruppe, eine Pyrengruppe, eine Chrysengruppe, eine Perylengruppe, eine Pentaphengruppe, eine Heptalengruppe, eine Naphthacengruppe, eine Picengruppe, eine Hexacengruppe, eine Pentacengruppe, eine Rubicengruppe, eine Coronengruppe, eine Ovalengruppe, eine Indengruppe, eine Fluorengruppe, eine Spiro-Bifluorengruppe, eine Benzofluorengruppe, eine Indenophenanthrengruppe, eine Indenoanthracengruppe, eine 1H-Pyrrolgruppe, eine Silanolgruppe, eine Borgruppe, eine 2H-Pyrrolgruppe, eine 3H-Pyrrolgruppe, eine Thiophengruppe, eine Furangruppe, eine Indolgruppe, eine Benzoindolgruppe, eine Naphthoindolgruppe, eine Isoindolgruppe, eine Benzoisoindolgruppe, eine Naphthoisoindolgruppe, eine Benzosilolgruppe, eine Benzothiophengruppe, eine Benzofurangruppe, eine Carbazolgruppe, eine Dibenzosilolgruppe, eine Dibenzothiophengruppe, eine Dibenzofurangruppe, eine Indenocarbazolgruppe, eine Indolocarbazolgruppe, eine Benzofurocarbazolgruppe, eine Benzothienocarbazolgruppe, eine Benzosilolcarbazolgruppe, eine Benzoindolocarbazolgruppe, eine Benzocarbazolgruppe, eine Benzonaphthofurangruppe, eine Benzonaphthothiophengruppe, eine Benzonaphthosilolgruppe, eine Benzofurodibenzofurangruppe, eine Benzofurodibenzothiophengruppe, eine Benzothienodibenzothiophengruppe, eine Pyrazolgruppe, eine Imidazolgruppe, eine Triazolgruppe, eine Oxazolgruppe, eine Isoxazolgruppe, eine Oxadiazolgruppe, eine Thiazolgruppe, eine Isothiazolgruppe, eine Thiadiazolgruppe, eine Benzopyrazolgruppe, eine Benzimidazolgruppe, eine Benzoxazolgruppe, eine Benzoisoxazolgruppe, eine Benzothiazolgruppe, eine Benzoisothiazolgruppe, eine Pyridingruppe, eine Pyrimidingruppe, eine Pyrazingruppe, eine Pyridazingruppe, eine Triazingruppe, eine Chinolingruppe, eine Isochinolingruppe, eine Benzochinolingruppe, eine Benzoisochinolingruppe, eine Chinoxalingruppe, eine Benzochinoxalingruppe, eine Chinazolingruppe, eine Benzochinazolingruppe, eine Phenanthrolingruppe, eine Cinnolingruppe, eine Phthalazingruppe, eine Naphthyridingruppe, eine Imidazopyridingruppe, eine Imidazopyrimidingruppe, eine Imidazotriazingruppe, eine Imidazopyrazingruppe, eine Imidazopyridazingruppe, eine Azacarbazolgruppe, eine Azafluorengruppe, eine Azadibenzosilolgruppe, eine Azadibenzothiophengruppe oder eine Azadibenzofurangruppe ist,
und/oder wobei L₁ und L₂ jeweils unabhängig sind:
eine Einfachbindung; oder
eine Benzolgruppe, eine Naphthalingruppe, eine Anthracengruppe, eine Phenanthrengruppe, eine Triphenylengruppe, eine Pyrengruppe, eine Chrysengruppe, eine Cyclopentadiengruppe, eine 1,2,3,4-Tetrahydronaphthalingruppe, eine Thiophengruppe, eine Furangruppe, eine Indolgruppe, eine Benzoborolgruppe, eine Benzophospholgruppe, eine Indengruppe, eine Benzosilolgruppe, eine Benzogermolgruppe, eine Benzothiophengruppe, eine Benzoselenophengruppe, eine Benzofurangruppe, eine Carbazolgruppe, eine Dibenzoborolgruppe, eine Dibenzophospholgruppe, eine Fluorengruppe, eine Dibenzosilolgruppe, eine Dibenzogermolgruppe, eine Dibenzothiophengruppe, eine Dibenzoselenophengruppe, eine Dibenzofurangruppe, eine Dibenzothiophen-5-oxidgruppe, eine 9H-a-Fluoren-9-ongruppe, eine Dibenzothiophen-5,5-dioxidgruppe, eine Azaindolgruppe, eine Azabenzoborolgruppe, eine Azabenzophospholgruppe, eine Azaindengruppe, eine Azabenzosilolgruppe, eine Azabenzogermolgruppe, eine Azabenzothiophengruppe, eine Azabenzoselenophengruppe, eine Azabenzofurangruppe, eine Azacarbazolgruppe, eine Azadibenzoborolgruppe, eine Azadibenzophospholgruppe, eine Azafluorengruppe, eine Azadibenzosilolgruppe, eine Azadibenzogermolgruppe, eine Azadibenzothiophengruppe, eine Azadibenzoselenophengruppe, eine Azadibenzofurangruppe, eine Azadibenzothiophen-5-oxidgruppe, eine Aza-9H-Fluoren-9-ongruppe, eine Azadibenzothiophen-5,5-dioxidgruppe, eine Pyridingruppe, eine Pyrimidingruppe, eine Pyrazingruppe, eine Pyridazingruppe, eine Triazingruppe, eine Chinolingruppe, eine Isochinolingruppe, eine Chinoxalingruppe, eine Chinazolingruppe, eine Phenanthrolingruppe, eine Pyrrolgruppe, eine Pyrazolgruppe, eine Imidazolgruppe, eine Triazolgruppe, eine Oxazolgruppe, eine Isoxazolgruppe, eine Thiazolgruppe, eine Isothiazolgruppe, eine Oxadiazolgruppe, eine Thiadiazolgruppe, eine Benzopyrazolgruppe, eine Benzimidazolgruppe, eine Benzoxazolgruppe, eine Benzothiazolgruppe, eine Benzooxadiazolgruppe, eine Benzothiadiazolgruppe, eine 5,6,7,8-Tetrahydroisochinolingruppe, oder eine 5,6,7,8-Tetrahydrochinolingruppe, jeweils unsubstituiert oder substituiert mit mindestens einem R₁₀ₐ, und
R₁₀ₐ wie in Anspruch 8 beschrieben ist.

11. Heterocyclische Verbindung nach einem der Ansprüche 7 bis 10, wobei L₁ und L₂ jeweils unabhängig Folgendes sind:
eine Einfachbindung; oder
eine der durch Formeln 3-1 bis 3-40 dargestellten Gruppen: 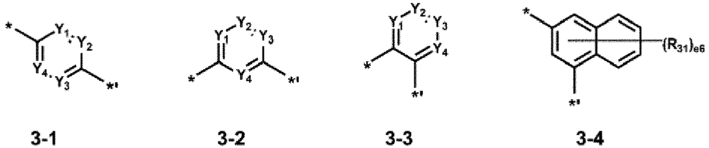 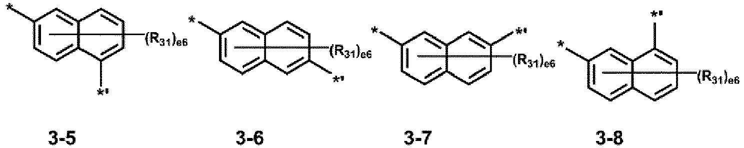 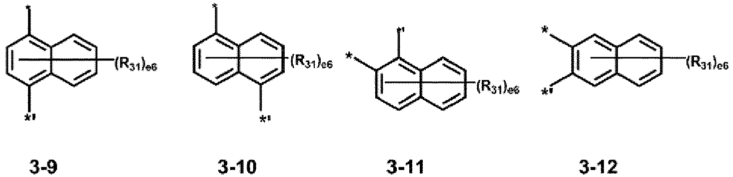 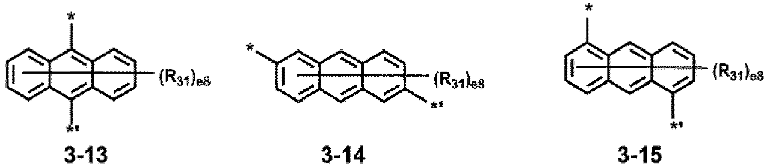 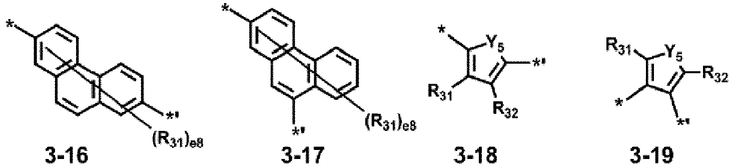 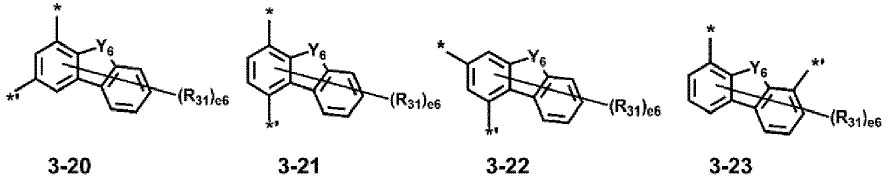 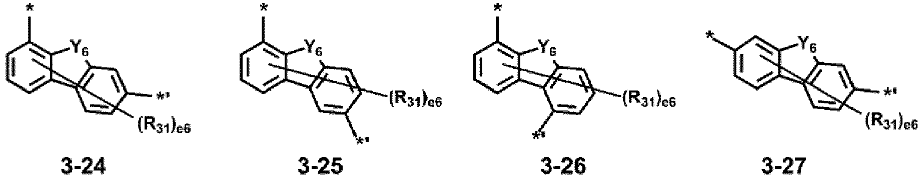 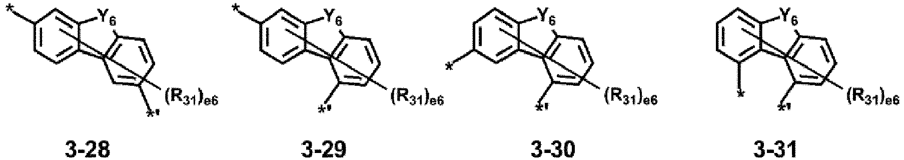 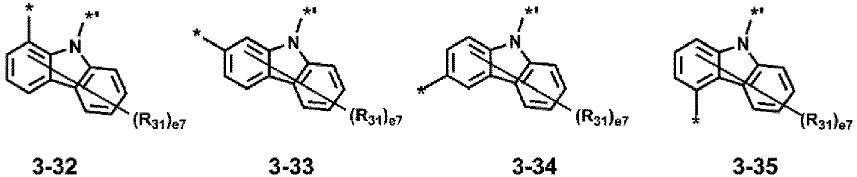 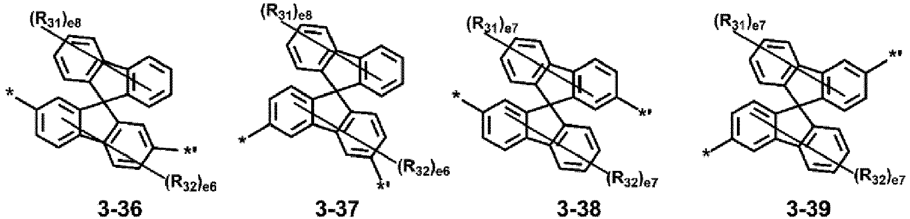 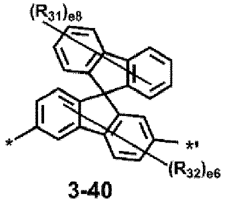 wobei in Formeln 3-1 bis 3-40
Y₁ N oder C(R₃₃) ist,
Y₂ N oder C(R₃₄) ist,
Y₃ N oder C(R₃₅) ist,
Y₄ N oder C(R₃₆) ist,
Y₅ O, S oder Se ist,
Y₆ ist O, S, Se, N(R₃₇) oder C(R₃₇)(R₃₈) ist,
R₃₁ bis R₃₈ jeweils unabhängig Folgendes sind:
Wasserstoff, Deuterium, -F, -Cl, -Br, -I, eine Hydroxygruppe, eine Cyanogruppe oder eine Nitrogruppe;
eine C₁-C₆₀-Alkylgruppe, eine C₂-C₆₀-Alkenylgruppe, eine C₂-C₆₀-Alkinylgruppe oder eine C₁-C₆₀-Alkoxygruppe, jeweils unsubstituiert oder substituiert mit Deuterium, -F, -Cl, -Br, -I, eine Hydroxygruppe, eine Cyanogruppe, eine Nitrogruppe, eine carbocyclische C₃-C₆₀-Gruppe, eine heterocyclische C₁-C₆₀-Gruppe, eine C₆-C₆₀-Aryloxygruppe, eine C₆-C₆₀-Arylthiogruppe, - Si(Q₁₁)(Q₁₂)(Q₁₃), -B(Q₁₁)(Q₁₂), -C(=O)(Q₁₁), -S(=O)₂(Q₁₁), -P(=O)(Q₁₁)(Q₁₂) oder eine Kombination davon;
eine carbocyclische C₃-C₆₀-Gruppe, eine heterocyclische C₁-C₆₀-Gruppe, eine C₆-C₆₀-Aryloxygruppe, oder eine C₆-C₆₀-Arylthiogruppe, jeweils unsubstituiert oder substituiert mit Deuterium, -F, -Cl, -Br, -I, eine Hydroxygruppe, eine Cyanogruppe, eine Nitrogruppe, eine C₁-C₆₀-Alkylgruppe, eine C₂-C₆₀-Alkenylgruppe, eine C₂-C₆₀-Alkinylgruppe eine C₁-C₆₀-Alkoxygruppe, eine carbocyclische C₃-C₆₀-Gruppe, eine heterocyclische C₁-C₆₀-Gruppe, eine C₆-C₆₀-Aryloxygruppe, eine C₆-C₆₀-Arylthiogruppe, -Si(Q₂₁)(Q₂₂)(Q₂₃), -B(Q₂₁)(Q₂₂), -C(=O)(Q₂₁), - S(=O)₂(Q₂₁), -P(=O)(Q₂₁)(Q₂₂), oder eine Kombination davon; oder
-Si(Q₃₁)(Q₃₂)(Q₃₃), -B(Q₃₁)(Q₃₂), -C(=O)(Q₃₁), -S(=O)₂(Q₃₁), oder -P(=O)(Q₃₁)(Q₃₂), Q₁₁ bis Q₁₃, Q₂₁ bis Q₂₃ und Q₃₁ bis Q₃₃ jeweils wie in Anspruch 7 beschrieben sind, e6 eine ganze Zahl von 0 bis 6 ist,
e7 eine ganze Zahl von 0 bis 7 ist,
e8 eine ganze Zahl von 0 bis 8 ist, und
* und *' jeweils eine Bindungsstelle zu einem benachbarten Atom anzeigen.

12. Heterocyclische Verbindung nach einem der Ansprüche 7 bis 11, wobei die durch Formel 1 dargestellte heterocyclische Verbindung durch eine von Formeln 1-1 bis 1-5 dargestellt wird: 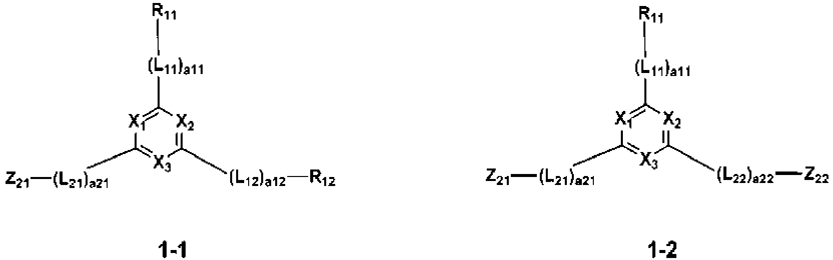 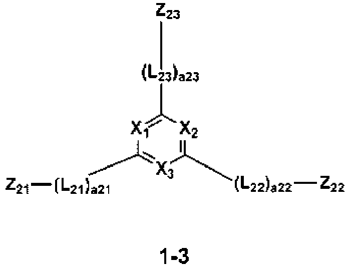 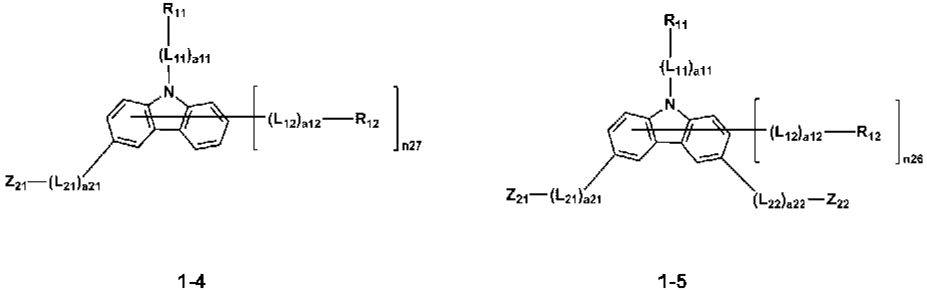 wobei in Formeln 1-1 bis 1-5
X₁ C(R₁₃) oder N ist,
X₂ C(R₁₄) oder N ist,
X₃ C(R₁₅) oder N ist,
R₁₁ bis R₁₅ jeweils dieselben wie die in Zusammenhang mit R₁ in Anspruch 7 beschriebenen sind,
L₁₁ und L₁₂ jeweils wie in Zusammenhang mit L₁ in Anspruch 7 beschrieben sind,
a11 und a12 jeweils wie in Zusammenhang mit a1 in Anspruch 7 beschrieben sind,
n26 eine ganze Zahl von 1 bis 6 ist,
n27 eine ganze Zahl von 1 bis 7 ist,
L₂₁ bis L₂₃ jeweils wie in Zusammenhang mit L₂ in Anspruch 7 beschrieben sind,
a21 bis a23 jeweils wie in Zusammenhang mit a2 in Anspruch 7 beschrieben sind, und
Z₂₁ bis Z₂₃ jeweils wie in Zusammenhang mit Z₂ in Anspruch 7 beschrieben sind.

13. Heterocyclische Verbindung nach Anspruch 12, wobei mindestens eines von X₁ bis X₃ N ist, und/oder wobei R₁₁ -Si(Q₁)(Q₂)(Q₃) ist, und
Q₁ bis Q₃ jeweils wie in Anspruch 7 beschrieben sind.

14. Heterocyclische Verbindung nach Anspruch 7, wobei E₁ bis E₆ jeweils unabhängig Folgendes sind:
eine C₁-C₂₀-Alkylgruppe, eine C₂-C₆₀-Alkenylgruppe oder eine C₂-C₆₀-Alkinylgruppe, oder eine C₁-C₂₀-Alkylgruppe, eine C₂-C₆₀-Alkenylgruppe oder eine C₂-C₂₀-Alkinylgruppe, jeweils substituiert mit Deuterium, -F, -Cl, -Br, -I, -CD₃, -CD₂H, -CDH₂, -CF₃, -CF₂H, -CFH₂, eine Hydroxygruppe, eine Cyanogruppe, eine Nitrogruppe, eine Amidinogruppe, eine Hydrazingruppe, eine Hydrazongruppe, eine C₁-C₁₀-Alkylgruppe, eine Cyclopentylgruppe, eine Cyclohexylgruppe, eine Cycloheptylgruppe, eine Cyclooctylgruppe, eine Adamantanylgruppe, eine Norbornanylgruppe, eine Norbornenylgruppe, eine Cyclopentenylgruppe, eine Cyclohexanylgruppe, eine Cycloheptenylgruppe, eine Phenylgruppe, eine Biphenylgruppe, eine Naphthylgruppe, eine Pyridinylgruppe, eine Pyrimidinylgruppe oder eine Kombination davon und/oder, wobei die durch Formel 2 dargestellte Gruppe durch Formel 2-1 dargestellt wird: 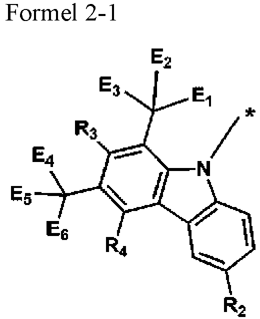 wobei in Formel 2-1,
E₁ bis E₆ und R₂ bis R₄ jeweils wie in Anspruch 7 beschrieben sind, und
* eine Bindungsstelle zu einem benachbarten Atom anzeigt.

15. Heterocyclische Verbindung nach Anspruch 7, wobei die heterocyclische Verbindung eine der Verbindungen 1 bis 50 ist:

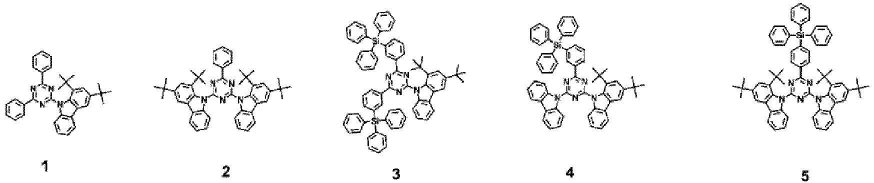

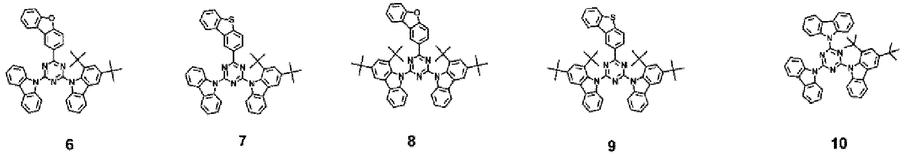

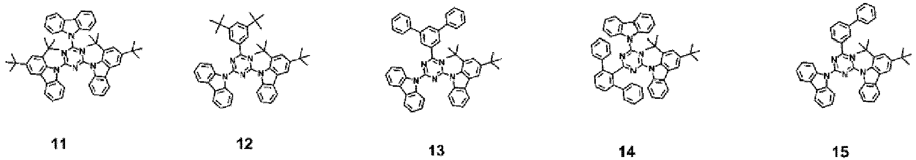

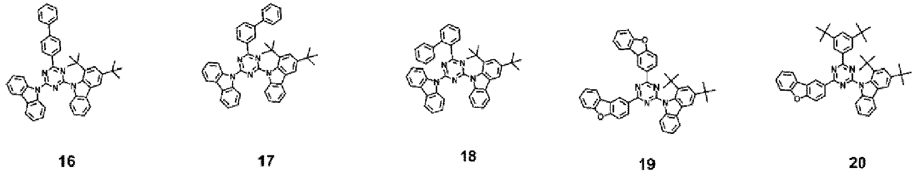

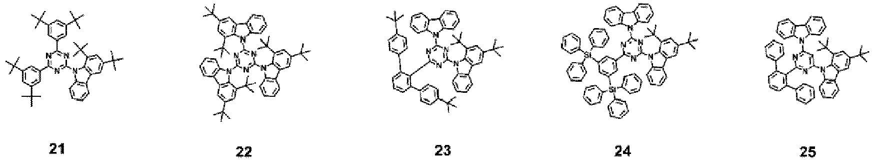

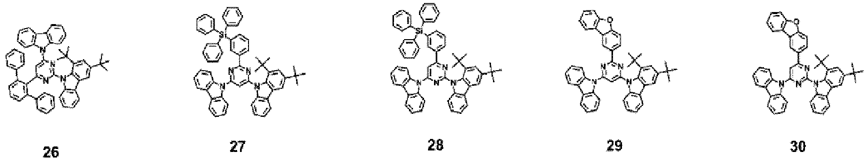

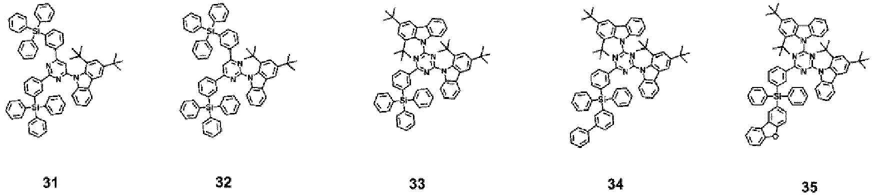

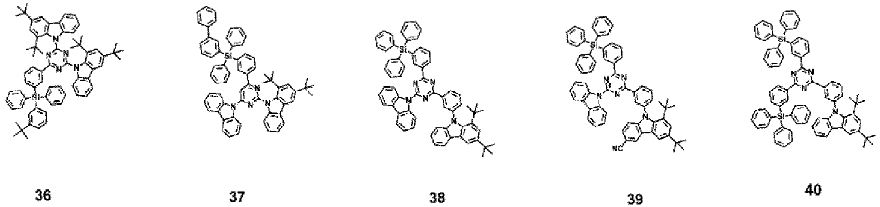

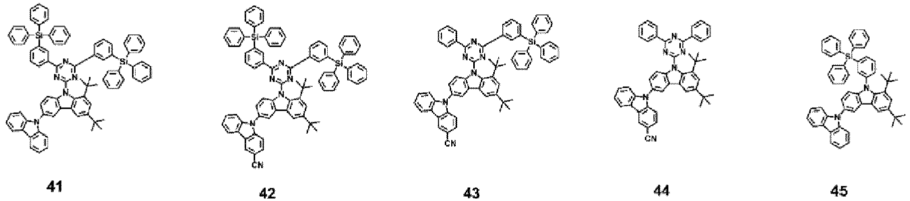

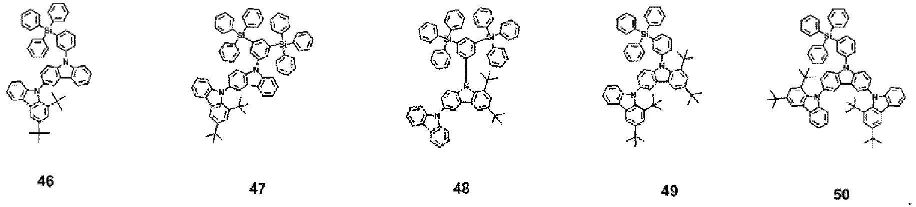

## Revendications

1. Dispositif électroluminescent comprenant :
une première électrode ;
une seconde électrode faisant face à la première électrode ;
une couche intermédiaire entre la première électrode et la seconde électrode et comprenant une couche d'émission ; et
au moins un composé hétérocyclique représenté par la formule 1 : 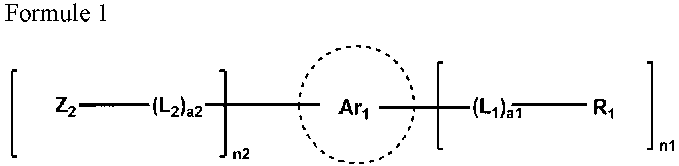 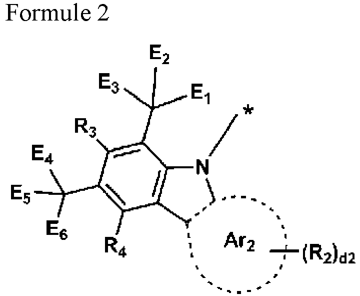 dans lesquelles, dans les formules 1 et 2,
Z₂ est un groupe représenté par la formule 2,
Ar₁ et Ar₂ représentent chacun indépendamment un groupe C₅-C₆₀ carbocyclique ou un groupe C₁-C₆₀ hétérocyclique,
L₁ et L₂ représentent chacun indépendamment une liaison simple, un groupe C₅-C₆₀ carbocyclique non substitué ou substitué par au moins un groupe R₁₀ₐ, ou un groupe C₁-C₆₀ hétérocyclique non substitué ou substitué par au moins un groupe R₁₀ₐ,
a1 et a2 représentent chacun indépendamment un entier de 1 à 3,
R₁ à R₄ représentent chacun indépendamment :
un atome d'hydrogène, un atome de deutérium, un groupe -F, -Cl, -Br, -I, un groupe hydroxy, un groupe cyano, un groupe nitro, un groupe C₁-C₆₀ alkyle non substitué ou substitué par au moins un groupe R₁₀ₐ, un groupe C₂-C₆₀ alcényle non substitué ou substitué par au moins un groupe R₁₀ₐ, un groupe C₂-C₆₀ alcynyle non substitué ou substitué par au moins un groupe R₁₀ₐ, un groupe C₁-C₆₀ alcoxy non substitué ou substitué par au moins un groupe R₁₀ₐ, un groupe C₃-C₆₀ carbocyclique non substitué ou substitué par au moins un groupe R₁₀ₐ, un groupe C₁-C₆₀ hétérocyclique non substitué ou substitué par au moins un groupe R₁₀ₐ, un groupe C₆-C₆₀ aryloxy non substitué ou substitué par au moins un groupe R₁₀ₐ, un groupe C₆-C₆₀ arylthio non substitué ou substitué par au moins un groupe R₁₀ₐ, un groupe -Si(Q₁)(Q₂)(Q₃), -B(Q₁)(Q₂), -C(=O)(Q₁), - S(=O)₂(Q₁) ou -P(=O)(Q₁)(Q₂) ; ou
un groupe représenté par la formule 2,
E₁ à E₆ représentent chacun indépendamment un groupe C₁-C₆₀ alkyle non substitué ou substitué par au moins un groupe R₁₀ₐ, un groupe C₂-C₆₀ alcényle non substitué ou substitué par au moins un groupe R₁₀ₐ, ou un groupe C₂-C₆₀ alcynyle non substitué ou substitué par au moins un groupe R₁₀ₐ,
d2 représente un entier allant de 0 à 10,
n1 représente un entier allant de 1 à 10,
n2 représente un entier allant de 1 à 5,
* indique un site de liaison à un atome voisin,
R₁₀ₐ représente :
un atome de deutérium, un groupe -F, -Cl, -Br, -I, un groupe hydroxy, un groupe cyano, ou un groupe nitro ;
un groupe C₁-C₆₀ alkyle, un groupe C₂-C₆₀ alcényle, un groupe C₂-C₆₀ alcynyle, ou un groupe C₁-C₆₀ alcoxy, chacun non substitué ou substitué par un atome de deutérium, un groupe - F, -Cl, -Br, -I, un groupe hydroxy, un groupe cyano, un groupe nitro, un groupe C₃-C₆₀ carbocyclique, un groupe C₁-C₆₀ hétérocyclique, un groupe C₆-C₆₀ aryloxy, un groupe C₆-C₆₀ arylthio, un groupe C₇-C₆₀ arylalkyle, un groupe C₂-C₆₀ hétéroarylalkyle, -Si(Q₁₁)(Q₁₂)(Q₁₃), - N(Q₁₁)(Q₁₂), -B(Q₁₁)(Q₁₂), -C(=O)(Q₁₁), -S(=O)₂(Q₁₁), -P(=O)(Q₁₁)(Q₁₂), ou toute combinaison de ceux-ci ;
un groupe C₃-C₆₀ carbocyclique, un groupe C₁-C₆₀ hétérocyclique, un groupe C₆-C₆₀ aryloxy, un groupe C₆-C₆₀ arylthio, un groupe C₇-C₆₀ arylalkyle, ou un groupe C₂-C₆₀ hétéroarylalkyle, chacun non substitué ou substitué par un atome de deutérium, un groupe -F, -Cl, -Br, -I, un groupe hydroxy, un groupe cyano, un groupe nitro, un groupe C₁-C₆₀ alkyle, un groupe C₂-C₆₀ alcényle, un groupe C₂-C₆₀ alcynyle, un groupe C₁-C₆₀ alcoxy, un groupe C₃-C₆₀ carbocyclique, un groupe C₁-C₆₀ hétérocyclique, un groupe C₆-C₆₀ aryloxy, un groupe C₆-C₆₀ arylthio, un groupe C₇-C₆₀ arylalkyle, un groupe C₂-C₆₀ hétéroarylalkyle, -Si(Q₂₁)(Q₂₂)(Q₂₃), - N(Q₂₁)(Q₂₂), -B(Q₂₁)(Q₂₂), -C(=O)(Q₂₁), -S(=O)₂(Q₂₁), -P(=O)(Q₂₁)(Q₂₂), ou toute combinaison de ceux-ci ; ou
un groupe -Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁) ou - P(=O)(Q₃₁)(Q₃₂), et
Q1 à Q₃, Q₁₁ à Q₁₃, Q₂₁ à Q₂₃, et Q₃₁ à Q₃₃ représentent chacun indépendamment : un atome d'hydrogène ; un atome de deutérium ; un groupe -F ; -Cl ; -Br ; -I ; un groupe hydroxy ; un groupe cyano ; un groupe nitro ; un groupe C₁-C₆₀ alkyle ; un groupe C₂-C₆₀ alcényle ; un groupe C₂-C₆₀ alcynyle ; un groupe C₁-C₆₀ alcoxy ; ou un groupe C₃-C₆₀ carbocyclique ou un groupe C₁-C₆₀ hétérocyclique, chacun non substitué ou substitué par un atome de deutérium, un groupe -F, un groupe cyano, un groupe C₁-C₆₀ alkyle, un groupe C₁-C₆₀ alcoxy, un groupe C₃-C₆₀ carbocyclique, un groupe C₁-C₆₀ hétérocyclique, ou toute combinaison de ceux-ci.

2. Dispositif électroluminescent selon la revendication 1, ladite première électrode étant une anode,
ladite seconde électrode étant une cathode,
ladite couche intermédiaire comprenant en outre une zone de transport de trous entre la couche d'émission et la seconde électrode,
ladite couche intermédiaire comprenant en outre une zone de transport d'électrons entre la couche d'émission et la seconde électrode,
ladite zone de transport de trous comprenant une couche d'injection de trous, une couche de transport de trous, une couche auxiliaire d'émission, une couche de blocage d'électrons, ou toute combinaison de celles-ci, et
ladite zone de transport d'électrons comprenant une couche tampon, une couche de blocage de trous, une couche de commande d'électrons, une couche de transport d'électrons, une couche d'injection d'électrons, ou toute combinaison de celles-ci.

3. Dispositif électroluminescent selon la revendication 1 ou la revendication 2, ladite couche d'émission comprenant ledit au moins un composé hétérocyclique représenté par la formule 1.

4. Dispositif électroluminescent selon la revendication 3, ladite couche d'émission comprenant en outre un composé organométallique représenté par la formule 401 :
Formule 401 M(L₄₀₁)_{xc1}(L₄₀₂)_{xc2}
dans laquelle, dans la formule 401,
M représente un métal de transition,
L₄₀₁ est un ligand représenté par la formula 402, et xc1 vaut 1, 2 ou 3, lorsque xc1 vaut 2 ou plus, deux des groupes L₄₀₁ ou plus étant identiques les uns aux autres ou différents les uns des autres, et
L₄₀₂ représente un ligand organique, et xc2 vaut 0, 1, 2, 3 ou 4, lorsque xc2 vaut 2 ou plus, deux des groupes L₄₀₂ ou plus étant identiques les uns aux autres ou différents les uns des autres,
Formule 402 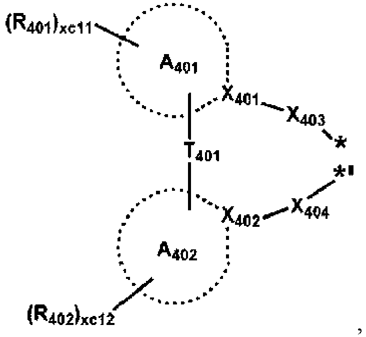 dans laquelle, dans la formule 402,
X₄₀₁ et X₄₀₂ représentent chacun indépendamment un atome d'azote ou de carbone,
le noyau A₄₀₁ et le noyau A₄₀₂ représentent chacun indépendamment un groupe C₃-C₆₀ carbocyclique ou un groupe C₁-C₆₀ hétérocyclique,
T₄₀₁ représente une liaison simple, un groupe *-O-*', *-S-*', *-C(=O)-*', *-N(Q₄₁₁)-*', *-C(Q₄₁₁)(Q₄₁₂)-*', *-C(Q₄₁₁)=C(Q₄₁₂)-*', *-C(Q₄₁₁)=*' ou *=C(Q₄₁₁)=*',
X₄₀₃ et X₄₀₄ représentent chacun indépendamment une liaison chimique, un atome O, S, un groupe N(Q₄₁₃), B(Q₄₁₃), P(Q₄₁₃), C(Q₄₁₃)(Q₄₁₄) ou Si(Q₄₁₃)(Q₄₁₄),
R₄₀₁ and R₄₀₂ représentent chacun indépendamment un atome d'hydrogène, un atome de deutérium, un groupe -F, -Cl, -Br, -I, un groupe hydroxy, un groupe cyano, un groupe nitro, un groupe C₁-C₂₀ alkyle non substitué ou substitué par au moins un groupe R₁₀ₐ, un groupe C₁-C₂₀ alcoxy non substitué ou substitué par au moins un groupe R₁₀ₐ, un groupe C₃-C₆₀ carbocyclique non substitué ou substitué par au moins un groupe R₁₀ₐ, un groupe C₁-C₆₀ hétérocyclique non substitué ou substitué par au moins un groupe R₁₀ₐ, -Si(Q₄₀₁)(Q₄₀₂)(Q₄₀₃), -N(Q₄₀₁)(Q₄₀₂), - B(Q₄₀₁)(Q₄₀₂), -C(=O)(Q₄₀₁), -S(=O)₂(Q₄₀₁) ou -P(=O)(Q₄₀₁)(Q₄₀₂),
Q₄₀₁ à Q₄₀₃ et Q₄₁₁ à Q₄₁₄ sont les mêmes que ceux décrits en lien avec Q₁ dans la revendication 1,
R₁₀ₐ est le même que celui décrit dans la revendication 1,
xc11 et xc12 représentent chacun indépendamment un entier allant de 0 à 10, et
* et *' dans la formule 402 indiquent chacun un site de liaison à M dans la formule 401.

5. Appareil électronique comprenant le dispositif électroluminescent selon l'une quelconque des revendications 1 à 4.

6. Appareil électronique selon la revendication 5, comprenant en outre :
un transistor à couches minces,
ledit transistor à couches minces comprenant une électrode de source et une électrode de drain, et
ladite première électrode du dispositif électroluminescent étant électriquement connectée à l'électrode de source ou l'électrode de drain ; éventuellement comprenant en outre :
un filtre de couleur, une couche de conversion de couleur, une couche tactile, une couche polarisante, ou toute combinaison de ceux-ci.

7. Composé hétérocyclique représenté par la formule 1 : 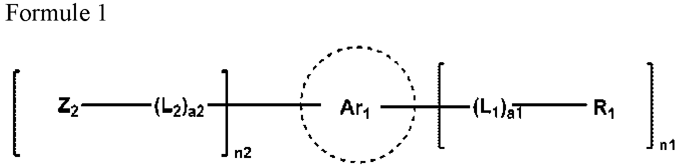 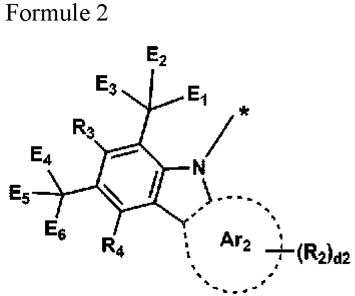 dans lesquelles, dans les formules 1 et 2,
Z₂ est un groupe représenté par la formule 2,
Ar₁ et Ar₂ représentent chacun indépendamment un groupe C₅-C₆₀ carbocyclique ou un groupe C₁-C₆₀ hétérocyclique,
L₁ et L₂ représentent chacun indépendamment une liaison simple, un groupe C₅-C₆₀ carbocyclique non substitué ou substitué par au moins un groupe R₁₀ₐ, ou un groupe C₁-C₆₀ hétérocyclique non substitué ou substitué par au moins un groupe R₁₀ₐ,
a1 et a2 représentent chacun indépendamment un entier allant de 1 à 3,
R₁ à R₄ représentent chacun indépendamment :
un atome d'hydrogène, un atome de deutérium, un groupe -F, -Cl, -Br, -I, un groupe hydroxy, un groupe cyano, un groupe nitro, un groupe C₁-C₆₀ alkyle non substitué ou substitué par au moins un groupe R₁₀ₐ, un groupe C₂-C₆₀ alcényle non substitué ou substitué par au moins un groupe R₁₀ₐ, un groupe C₂-C₆₀ alcynyle non substitué ou substitué par au moins un groupe R₁₀ₐ, un groupe C₁-C₆₀ alcoxy non substitué ou substitué par au moins un groupe R₁₀ₐ, un groupe C₃-C₆₀ carbocyclique non substitué ou substitué par au moins un groupe R₁₀ₐ, un groupe C₁-C₆₀ hétérocyclique non substitué ou substitué par au moins un groupe R₁₀ₐ, un groupe C₆-C₆₀ aryloxy non substitué ou substitué par au moins un groupe R₁₀ₐ, un groupe C₆-C₆₀ arylthio non substitué ou substitué par au moins un groupe R₁₀ₐ, un groupe -Si(Q₁)(Q₂)(Q₃), -B(Q₁)(Q₂), -C(=O)(Q₁), - S(=O)₂(Q₁) ou -P(=O)(Q₁)(Q₂) ; ou
un groupe représenté par la formule 2,
E₁ à E₆ représentent chacun indépendamment un groupe C₁-C₆₀ alkyle non substitué ou substitué par au moins un groupe R₁₀ₐ, un groupe C₂-C₆₀ alcényle non substitué ou substitué par au moins un groupe R₁₀ₐ, ou un groupe C₂-C₆₀ alcynyle non substitué ou substitué par au moins un groupe R₁₀ₐ,
d2 représente un entier allant de 0 à 10,
n1 représente un entier allant de 1 à 10,
n2 représente un entier allant de 1 à 5,
* indique un site de liaison à un atome voisin,
R₁₀ₐ représente :
un atome de deutérium, un groupe -F, -Cl, -Br, -I, un groupe hydroxy, un groupe cyano, ou un groupe nitro ;
un groupe C₁-C₆₀ alkyle, un groupe C₂-C₆₀ alcényle, un groupe C₂-C₆₀ alcynyle, ou un groupe C₁-C₆₀ alcoxy, chacun non substitué ou substitué par un atome de deutérium, un groupe - F, -Cl, -Br, -I, un groupe hydroxy, un groupe cyano, un groupe nitro, un groupe C₃-C₆₀ carbocyclique, un groupe C₁-C₆₀ hétérocyclique, un groupe C₆-C₆₀ aryloxy, un groupe C₆-C₆₀ arylthio, un groupe C₇-C₆₀ arylalkyle, un groupe C₂-C₆₀ hétéroarylalkyle, -Si(Q₁₁)(Q₁₂)(Q₁₃), - N(Q₁₁)(Q₁₂), -B(Q₁₁)(Q₁₂), -C(=O)(Q₁₁), -S(=O)₂(Q₁₁), -P(=O)(Q₁₁)(Q₁₂), ou toute combinaison de ceux-ci ;
un groupe C₃-C₆₀ carbocyclique, un groupe C₁-C₆₀ hétérocyclique, un groupe C₆-C₆₀ aryloxy, un groupe C₆-C₆₀ arylthio, un groupe C₇-C₆₀ arylalkyle, ou un groupe C₂-C₆₀ hétéroarylalkyle, chacun non substitué ou substitué par un atome de deutérium, un groupe -F, -Cl, -Br, -I, un groupe hydroxy, un groupe cyano, un groupe nitro, un groupe C₁-C₆₀ alkyle, un groupe C₂-C₆₀ alcényle, un groupe C₂-C₆₀ alcynyle, un groupe C₁-C₆₀ alcoxy, un groupe C₃-C₆₀ carbocyclique, un groupe C₁-C₆₀ hétérocyclique, un groupe C₆-C₆₀ aryloxy, un groupe C₆-C₆₀ arylthio, un groupe C₇-C₆₀ arylalkyle, un groupe C₂-C₆₀ hétéroarylalkyle, -Si(Q₂₁)(Q₂₂)(Q₂₃), - N(Q₂₁)(Q₂₂), -B(Q₂₁)(Q₂₂), -C(=O)(Q₂₁), -S(=O)₂(Q₂₁), -P(=O)(Q₂₁)(Q₂₂), ou toute combinaison de ceux-ci ; ou
un groupe -Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁) ou - P(=O)(Q₃₁)(Q₃₂), et
Q1 à Q₃, Q₁₁ à Q₁₃, Q₂₁ à Q₂₃, et Q₃₁ à Q₃₃ représentent chacun indépendamment : un atome d'hydrogène ; un atome de deutérium ; un groupe -F ; -Cl ; -Br ; -I ; un groupe hydroxy ; un groupe cyano ; un groupe nitro ; un groupe C₁-C₆₀ alkyle ; un groupe C₂-C₆₀ alcényle ; un groupe C₂-C₆₀ alcynyle ; un groupe C₁-C₆₀ alcoxy ; ou un groupe C₃-C₆₀ carbocyclique ou un groupe C₁-C₆₀ hétérocyclique, chacun non substitué ou substitué par un atome de deutérium, un groupe -F, un groupe cyano, un groupe C₁-C₆₀ alkyle, un groupe C₁-C₆₀ alcoxy, un groupe C₃-C₆₀ carbocyclique, un groupe C₁-C₆₀ hétérocyclique, ou toute combinaison de ceux-ci ;
ledit composé hétérocyclique représenté par la formule 1 satisfaisant au moins l'une des conditions 1 et 2 :
Condition 1
Ar₁ représente un groupe C₁-C₆₀ cyclique contenant de l'azote et déficient en électrons π ; et
Condition 2
dans laquelle au moins l'un des groupes R₁ au nombre de n1 représente un groupe - Si(Q₁)(Q₂)(Q₃).

8. Composé hétérocyclique selon la revendication 7, dans la condition 2 n2 représentant un entier allant de 1 à 3.

9. Composé hétérocyclique selon la revendication 7 ou la revendication 8, ledit composé hétérocyclique représenté par la formule 1 étant représenté par la formule 1-A : 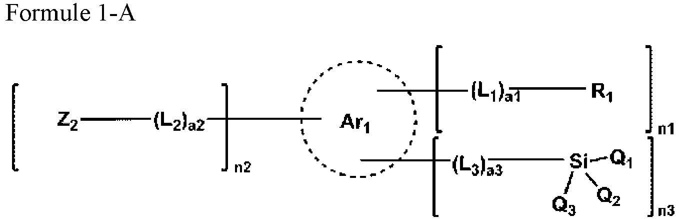 dans laquelle, dans la formule 1-A,
Z₂, Ar₁, L₁, L₂, n1, n2, a1, a2, R₁, et Q₁ à Q₃ sont tels que décrits dans la revendication 7,
L₃ est tel que décrit en lien avec L₁ dans la revendication 7,
a3 est tel que décrit en lien avec a1 dans la revendication 7, et
n3 est un entier allant de 0 à 10.

10. Composé hétérocyclique selon l'une quelconque des revendications 7 à 9, Ar₁ représentant un groupe cyclopentadiène, un groupe adamantane, un groupe norbornane, un groupe benzène, un groupe pentalène, un groupe naphtalène, un groupe azulène, un groupe indacène, un groupe acénaphtylène, un groupe phénalène, un groupe phénanthrène, un groupe anthracène, un groupe fluoranthène, un groupe triphénylène, un groupe pyrène, un groupe chrysène, un groupe pérylène, un groupe pentaphène, un groupe heptalène, un groupe naphtacène, un groupe picène, un groupe hexacène, un groupe pentacène, un groupe rubicène, un groupe coronène, un groupe ovalène, un groupe indène, un groupe fluorène, un groupe spiro-bifluorène, un groupe benzofluorène, un groupe indénophénanthrène, un groupe indénoanthracène, un groupe 1H-pyrrole, un groupe silole, un groupe borole, un groupe 2H-pyrrole, un groupe 3H-pyrrole, un groupe thiophène, un groupe furane, un groupe indole, un groupe benzoindole, un groupe naphtoindole, un groupe isoindole, un groupe benzoisoindole, un groupe naphtoisoindole, un groupe benzosilole, un groupe benzothiophène, un groupe benzofurane, un groupe carbazole, un groupe dibenzosilole, un groupe dibenzothiophène, un groupe dibenzofurane, un groupe indénocarbazole, un groupe indolocarbazole, un groupe benzofurocarbazole, un groupe benzothiénocarbazole, un groupe benzosilolocarbazole, un groupe benzoindolocarbazole, un groupe benzocarbazole, un groupe benzonaphtofurane, un groupe benzonaphtothiophène, un groupe benzonaphtosilole, un groupe benzofurodibenzofurane, un groupe benzofurodibenzothiophène, un groupe benzothiénodibenzothiophène, un groupe pyrazole, un groupe imidazole, un groupe triazole, un groupe oxazole, un groupe isoxazole, un groupe oxadiazole, un groupe thiazole, un groupe isothiazole, un groupe thiadiazole, un groupe benzopyrazole, un groupe benzimidazole, un groupe benzoxazole, un groupe benzoisoxazole, un groupe benzothiazole, un groupe benzoisothiazole, un groupe pyridine, un groupe pyrimidine, un groupe pyrazine, un groupe pyridazine, un groupe triazine, un groupe quinoline, un groupe isoquinoline, un groupe benzoquinoline, un groupe benzoisoquinoline, un groupe quinoxaline, un groupe benzoquinoxaline, un groupe quinazoline, un groupe benzoquinazoline, un groupe phénanthroline, un groupe cinnoline, un groupe phtalazine, un groupe naphtyridine, un groupe imidazopyridine, un groupe imidazopyrimidine, un groupe imidazotriazine, un groupe imidazopyrazine, un groupe imidazopyridazine, un groupe azacarbazole, un groupe azafluorène, un groupe azadibenzosilole, un groupe azadibenzothiophène, ou un groupe azadibenzofurane, et/ou
L₁ et L₂ représentant chacun indépendamment :
une liaison simple ; ou
un groupe benzène, un groupe naphtalène, un groupe anthracène, un groupe phénanthrène, un groupe triphénylène, un groupe pyrène, un groupe chrysène, un groupe cyclopentadiène, un groupe 1,2,3,4-tétrahydronaphtalène, un groupe thiophène, un groupe furane, un groupe indole, un groupe benzoborole, un groupe benzophosphole, un groupe indène, un groupe benzosilole, un groupe benzogermole, un groupe benzothiophène, un groupe benzosélénophène, un groupe benzofurane, un groupe carbazole, un groupe dibenzoborole, un groupe dibenzophosphole, un groupe fluorène, un groupe dibenzosilole, un groupe dibenzogermole, un groupe dibenzothiophène, un groupe dibenzosélénophène, un groupe dibenzofurane, un groupe 5-oxyde de dibenzothiophène, un groupe 9H-a fluorén-9-one, un groupe 5,5-dioxyde de dibenzothiophène, un groupe azaindole, un groupe azabenzoborole, un groupe azabenzophosphole, un groupe azaindène, un groupe azabenzosilole, un groupe azabenzogermole, un groupe azabenzothiophène, un groupe azabenzosélénophène, un groupe azabenzofurane, un groupe azacarbazole, un groupe azadibenzoborole, un groupe azadibenzophosphole, un groupe azafluorène, un groupe azadibenzosilole, un groupe azadibenzogermole, un groupe azadibenzothiophène, un groupe azadibenzosélénophène, un groupe azadibenzofurane, un groupe 5-oxyde d'azadibenzothiophène, un groupe aza-9H-fluorén-9-one, un groupe 5,5-dioxyde d'azadibenzothiophène, un groupe pyridine, un groupe pyrimidine, un groupe pyrazine, un groupe pyridazine, un groupe triazine, un groupe quinoline, un groupe isoquinoline, un groupe quinoxaline, un groupe quinazoline, un groupe phénanthroline, un groupe pyrrole, un groupe pyrazole, un groupe imidazole, un groupe triazole, un groupe oxazole, un groupe isoxazole, un groupe thiazole, un groupe isothiazole, un groupe oxadiazole, un groupe thiadiazole, un groupe benzopyrazole, un groupe benzimidazole, un groupe benzoxazole, un groupe benzothiazole, un groupe benzoxadiazole, un groupe benzothiadiazole, un groupe 5,6,7,8-tétrahydroisoquinoline, ou un groupe 5,6,7,8-tétrahydroquinoline, chacun substitué ou non substitué par au moins un groupe R₁₀ₐ, et
R₁₀ₐ est tel que décrit dans la revendication 8.

11. Composé hétérocyclique selon l'une quelconque des revendications 7 à 10, L₁ et L₂ représentant chacun indépendamment :
une liaison simple ; ou
l'un des groupes représentés par les formules 3-1 à 3-40 : 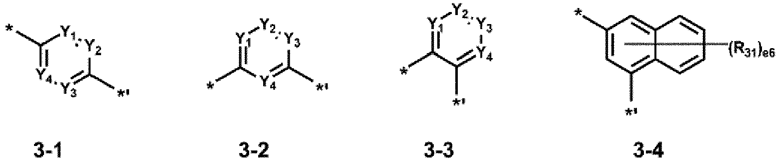 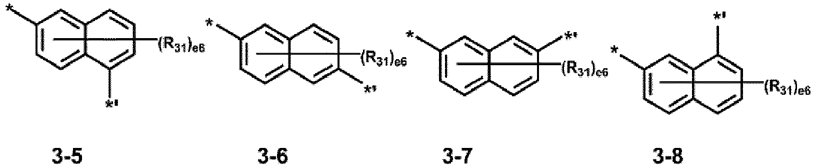 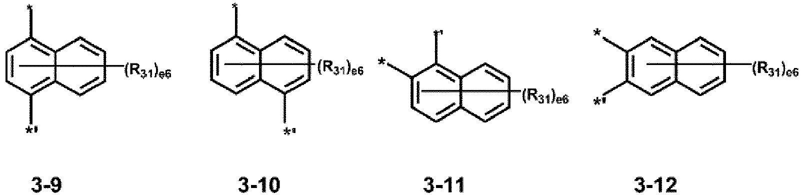 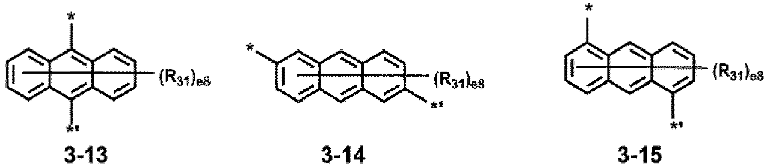 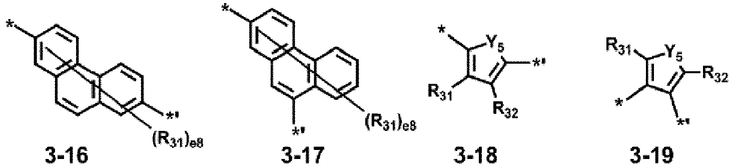 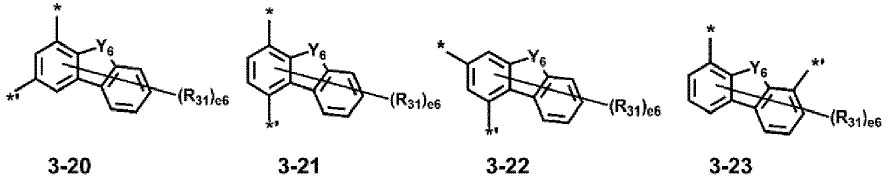 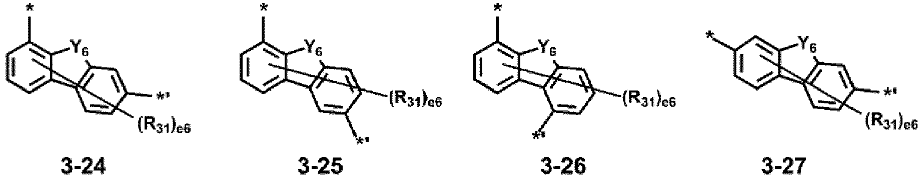 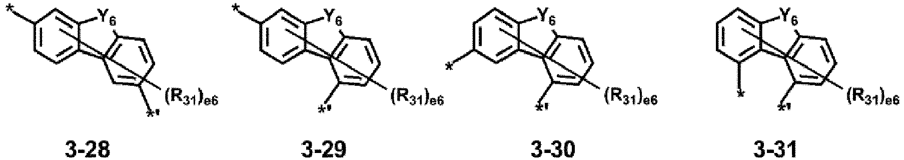 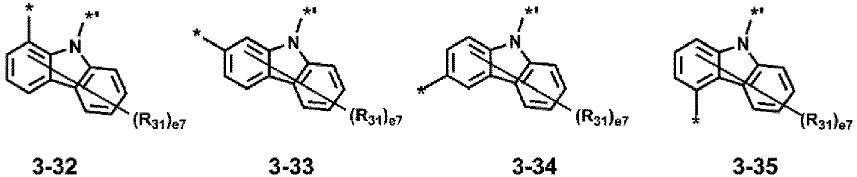 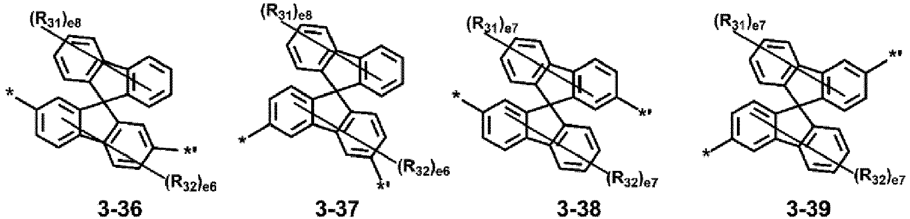 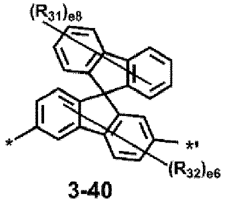 dans lesquelles, dans les formules 3-1 à 3-40,
Y₁ représente un atome N ou un groupe C(R₃₃),
Y₂ représente un atome N ou un groupe C(R₃₄),
Y₃ représente un atome N ou un groupe C(R₃₅),
Y₄ représente un atome N ou un groupe C(R₃₆),
Y₅ représente un atome O, S ou Se,
Y₆ représente un atome O, S, Se, un groupe N(R₃₇) ou C(R₃₇)(R₃₈),
R₃₁ à R₃₈ représentent chacun indépendamment :
un atome d'hydrogène, un atome de deutérium, un groupe -F, -Cl, -Br, -I, un groupe hydroxy, un groupe cyano ou un groupe nitro ;
un groupe C₁-C₆₀ alkyle, un groupe C₂-C₆₀ alcényle, un groupe C₂-C₆₀ alcynyle, ou un groupe C₁-C₆₀ alcoxy, chacun non substitué ou substitué par un atome de deutérium, un groupe - F, -Cl, -Br, -I, un groupe hydroxy, un groupe cyano, un groupe nitro, un groupe C₃-C₆₀ carbocyclique, un groupe C₁-C₆₀ hétérocyclique, un groupe C₆-C₆₀ aryloxy, un groupe C₆-C₆₀ arylthio, -Si(Q₁₁)(Q₁₂)(Q₁₃), -B(Q₁₁)(Q₁₂), -C(=O)(Q₁₁), -S(=O)₂(Q₁₁), -P(=O)(Q₁₁)(Q₁₂), ou toute combinaison de ceux-ci ;
un groupe C₃-C₆₀ carbocyclique, un groupe C₁-C₆₀ hétérocyclique, un groupe C₆-C₆₀ aryloxy ; ou un groupe C₆-C₆₀ arylthio, chacun non substitué ou substitué par un atome de deutérium, un groupe -F, -Cl, -Br, -I, un groupe hydroxy, un groupe cyano, un groupe nitro, un groupe C₁-C₆₀ alkyle, un groupe C₂-C₆₀ alcényle, un groupe C₂-C₆₀ alcynyle, un groupe C₁-C₆₀ alcoxy, un groupe C₃-C₆₀ carbocyclique, un groupe C₁-C₆₀ hétérocyclique, un groupe C₆-C₆₀ aryloxy, un groupe C₆-C₆₀ arylthio, -Si(Q₂₁)(Q₂₂)(Q₂₃), -B(Q₂₁)(Q₂₂), -C(=O)(Q₂₁), -S(=O)₂(Q₂₁), -P(=O)(Q₂₁)(Q₂₂), ou toute combinaison de ceux-ci ; ou
-Si(Q₃₁)(Q₃₂)(Q₃₃), -B(Q₃₁)(Q₃₂), -C(=O)(Q₃₁), -S(=O)₂(Q₃₁) ou -P(=O)(Q₃₁)(Q₃₂), Q₁₁ à Q₁₃, Q₂₁ à Q₂₃, et Q₃₁ à Q₃₃ sont chacun tels que décrits dans la revendication 7, e6 représente un entier allant de 0 à 6,
e7 représente un entier allant de 0 à 7,
e8 représente un entier allant de 0 à 8, et
* et *' indiquant chacun un site de liaison à un atome voisin.

12. Composé hétérocyclique selon l'une quelconque des revendications 7 à 11, ledit composé hétérocyclique représenté par la formule 1 étant représenté par l'une des formules 1-1 à 1-5 : 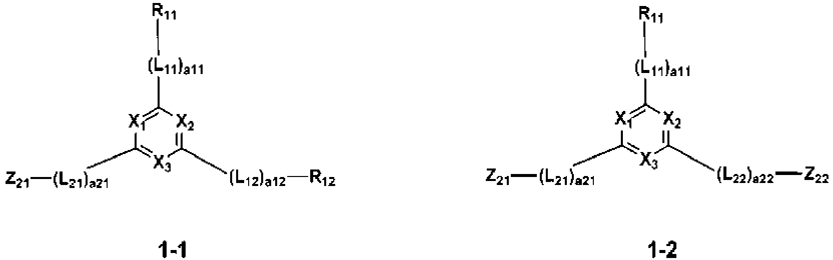 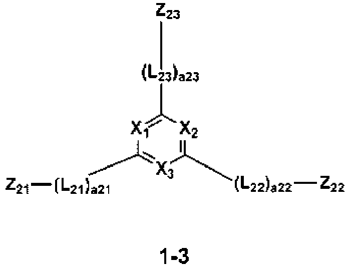 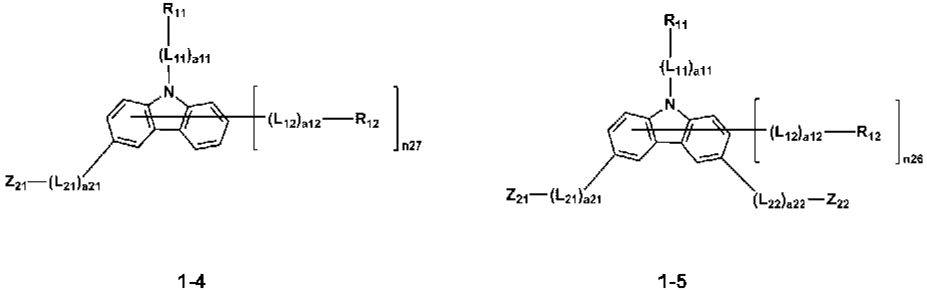 dans lesquelles, dans les formules 1-1 à 1-5,
X₁ représente un groupe C(R₁₃) ou un atome N,
X₂ représente un groupe C(R₁₄) ou un atome N,
X₃ représente un groupe C(R₁₅) ou un atome N,
R₁₁ à R₁₅ sont chacun tels que décrits en lien avec R₁ dans la revendication 7,
L₁₁ et L₁₂ sont chacun tels que décrits en lien avec L₁ dans la revendication 7,
a11 et a12 sont chacun tels que décrits en lien avec a1 dans la revendication 7,
n26 représente un entier allant de 1 à 6,
n27 représente un entier allant de 1 à 7,
L₂₁ à L₂₃ sont chacun tels que décrits en lien avec L₂ dans la revendication 7,
a21 à a23 sont chacun tels que décrits en lien avec a2 dans la revendication 7, et
Z₂₁ à Z₂₃ sont chacun tels que décrits en lien avec Z₂ dans la revendication 7.

13. Composé hétérocyclique selon la revendication 12, au moins l'un des groupes X₁ à X₃ représentant un atome N, et/ou R₁₁ représentant un groupe -Si(Q₁)(Q₂)(Q₃), et
Q₁ à Q₃ sont tels que décrits dans la revendication 7.

14. Composé hétérocyclique selon la revendication 7, E₁ à E₆ représentant chacun indépendamment :
un groupe C₁-C₂₀ alkyle, un groupe C₂-C₆₀ alcényle, ou un groupe C₂-C₆₀ alcynyle ; ou
un groupe C₁-C₂₀ alkyle, un groupe C₂-C₆₀ alcényle, ou un groupe C₂-C₂₀ alcynyle, chacun substitué par un atome de deutérium, un groupe -F, -Cl, -Br, -I, -CD₃, -CD₂H, -CDH₂, -CF₃, - CF₂H, -CFH₂, un groupe hydroxy, un groupe cyano, un groupe nitro, un groupe amidino, un groupe hydrazine, un groupe hydrazone, un groupe C₁-C₁₀ alkyle, un groupe cyclopentyle, un groupe cyclohexyle, un groupe cycloheptyle, un groupe cyclooctyle, un groupe adamantanyle, un groupe norbornanyle, un groupe norboményle, un groupe cyclopentényle, un groupe cyclohexényle, un groupe cycloheptényle, un groupe phényle, un groupe biphényle, un groupe naphtyle, un groupe pyridinyle, un groupe pyrimidinyle, ou toute combinaison de ceux-ci, et/ou,
ledit groupe représenté par la formule 2 étant représenté par la formule 2-1 : 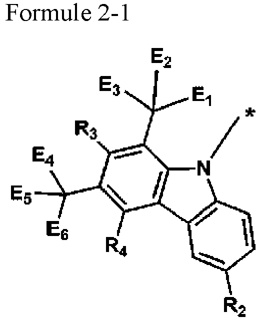 dans laquelle, dans la formule 2-1,
E₁ à E₆ et R₂ à R₄ sont chacun tels que décrits dans la revendication 7, et * indique un site de liaison à un atome voisin.

15. Composé hétérocyclique selon la revendication 7, ledit composé hétérocyclique étant l'un des composés 1 à 50 :
